(19) Europäisches Patentamt
European Patent Office
Office européen des brevets

(11) **EP 2 323 604 B1**

(12) **EUROPÄISCHE PATENTSCHRIFT**

(45) Veröffentlichungstag und Bekanntmachung des Hinweises auf die Patenterteilung:
**25.01.2012 Patentblatt 2012/04**

(21) Anmeldenummer: 09778143.9

(22) Anmeldetag: **27.08.2009**

(51) Int Cl.:
**A61F 13/15** (2006.01)

(86) Internationale Anmeldenummer:
**PCT/EP2009/006204**

(87) Internationale Veröffentlichungsnummer:
**WO 2010/028751 (18.03.2010 Gazette 2010/11)**

(54) **INKONTINENZARTIKEL IN HÖSCHENFORM**

INCONTINENCE ARTICLE IN THE FORM OF BRIEFS

ARTICLE POUR INCONTINENTS SOUS FORME DE CULOTTE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **10.09.2008 DE 102008046607**

(43) Veröffentlichungstag der Anmeldung:
**25.05.2011 Patentblatt 2011/21**

(73) Patentinhaber: **Paul Hartmann AG**
**89522 Heidenheim (DE)**

(72) Erfinder:
• **WENZEL, Benjamin**
**89231 Neu-Ulm (DE)**
• **HORNUNG, Fridmann**
**Penalolén (CL)**

(56) Entgegenhaltungen:
**WO-A1-2004/052260      US-A1- 2005 020 991**
**US-A1- 2008 114 325**

## Beschreibung

Die Erfindung betrifft einen Inkontinenzartikel in Höschenform für die Aufnahme von Körperausscheidungen mit einem vorderen Bauchabschnitt und einem hinteren Rückenabschnitt, die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind, und mit einem einen Absorptionskörper aufweisenden Schrittabschnitt, der sich in einer Längsrichtung zwischen Bauchabschnitt und Rückenabschnitt erstreckt und an den Bauchabschnitt und an den Rückenabschnitt unlösbar angefügt ist, wobei sowohl der Schrittabschnitt als auch der Bauchabschnitt und der Rückenabschnitt die Beinöffnungen des Inkontinenzartikels begrenzen. Ein derart dreikomponentiger Inkontinenzartikel ist beispielsweise aus WO 2004/052260 A1 bekannt. Bei diesem spezifischen Produktkonzept kann nach dem Anfügen des in Längsrichtung erstreckten Schrittabschnitts an den im Wesentlichen in Quer- oder Hüftumfangsrichtung erstreckten Bauchabschnitt und den entsprechend erstreckten Rückenabschnitt im eben ausgebreiteten Zustand dieser drei Komponenten eine H-förmige Grundstruktur des Inkontinenzartikels realisiert werden. Der Inkontinenzartikel ist dann modular aus den Komponenten Schrittabschnitt, Bauchabschnitt und Rückenabschnitt gebildet. Diese Komponenten werden vorteilhafterweise zunächst über den Schrittabschnitt miteinander verbunden, und vorzugsweise danach wird in beidseitigen Seitennahtbereichen der Bauchabschnitt mit dem Rückenabschnitt verbunden. Hierbei handelt es sich um eine herstellerseitige Verbindung, durch welche die Höschenform erhalten wird. Diese Verbindung ist typischerweise unlösbar. Die Höschenform kann aber auch insbesondere entlang einer Sollbruchlinie, die insbesondere im Seitennahtbereich verlaufen kann, auftrennbar sein, beispielsweise zum Abnehmen eines gebrauchten Inkontinenzartikels bei einer pflegebedürftigen Person.

Inkontinenzartikel in Höschenform unterscheiden sich prinzipiell von traditionellen öffenbaren und schließbaren Inkontinenzartikeln in üblicher Windelform dadurch, dass in der Regel der Hüftumfang vorgegeben ist und die Anpassung an unterschiedliche Körpergrößen ausgehend von einer Anzahl von Grundgrößen durch eine elastische Dehnbarkeit des Artikels erreicht wird. Hierfür werden in der Regel Elastifizierungsmittel, insbesondere in Form von Bändern oder Fäden, häufig als Lycra-Fäden bezeichnet, in vorgedehntem Zustand (Stretch-Bond-Verfahren) mit Chassismaterialien des Inkontinenzartikels verbunden, das heißt, sie werden in vorgedehntem Zustand an den Chassismaterialien z.B. mittels Kleber fixiert. Infolge ihrer Vorspannung raffen diese Elastifizierungsmittel die Chassismaterialien zusammen und bilden dabei Fältelungen. Der Inkontinenzartikel bzw. die elastifizierten Chassismaterialien des Inkontinenzartikels lassen sich dann wieder elastisch dehnen, wenn der Inkontinenzartikel wie ein Höschen an den Benutzer angelegt wird. Inkontinenzartikel in Höschenform mit derart elastifizierten Chassismaterialien sind mehrfach bekannt geworden und beispielsweise auch in der vorerwähnten WO 2004/052260 A1 behandelt.

Bei der Herstellung der H-förmigen Grundstruktur, also bei der Verbindung des Schrittabschnitts mit dem Bauchabschnitt und mit dem Rückenabschnitt, muss eine für sämtliche Anforderungen des bestimmungsgemäßen Gebrauchs hinreichende Fügeverbindung zwischen diesen Komponenten hergestellt werden, damit ausgeschlossen werden kann, dass bei Auftreten großer Zugbelastungen die Verbindung zwischen Schrittabschnitt und Bauchabschnitt oder Rückenabschnitt gelöst wird. Es wirken in der Tragesituation eines Inkontinenzartikels in Höschenform mitunter große Kräfte, und zwar zum einen infolge der Gewichtskraft eines mit beträchtlichen Flüssigkeitsmengen beaufschlagten Absorptionskörpers; zum anderen werden innerhalb der Chassismaterialien hohe Zugkräfte übertragen, und zwar zum einen durch die an die Chassismaterialien üblicherweise im Stretch-Bond-Verfahren angefügten Elastifizierungsmittel, und zum anderen durch Zugkräfte, die durch Bewegungen des Benutzers übertragen werden. Es hat sich hier gezeigt, dass solche hohen Zugspannungen überraschenderweise sowohl bei mobilen Benutzern auftreten, die den Inkontinenzartikel quasi wie ein Unterbekleidungsstück benutzen, als auch bei bettlägerigen, pflegebedürftigen Personen, deren Mobilität stark eingeschränkt ist. Aufgrund unkontrollierter und insbesondere auch langsamer Bewegungen besteht für Chassismaterialien des Inkontinenzartikels nämlich keine Möglichkeit, auftretenden Zugspannungen auszuweichen. Gerade in diesen Situationen werden Fügeverbindungen des Inkontinenzartikels, und zwar gerade zwischen Schrittabschnitt und Bauchabschnitt und zwischen Schrittabschnitt und Rückenabschnitt, in starkem Maße beansprucht.

Wenn zur Herstellung einer stabilen und dauerhaft auf Zug beanspruchbaren Verbindung zwischen Schrittabschnitt und Bauchabschnitt und zwischen Schrittabschnitt und Rückenabschnitt eine große Menge von insbesondere flächenhaft aufgetragenem Klebermaterial verwendet wird, so führt dies in nachteiliger Weise zu einer Versteifung im Überlappungsbereich von Schrittabschnitt und Bauchabschnitt bzw. Rückenabschnitt. Dies wird einerseits als unangenehm empfunden, und andererseits kann es eine erwünschte Dehnbarkeit der Chassismaterialien in diesem Bereich behindern, wobei an dieser Stelle auch erwähnt sei, dass in diesem Bereich eine flächenhafte Dehnbarkeit häufig nicht erwünscht ist. Doch auch in diesem Fall stellt sich das Problem, dass Klebermaterialien die Einstellung der erwünschten Produkteigenschaften behindern.

Wenn zur Herstellung einer stabilen und dauerhaft auf Zug beanspruchbaren Verbindung zwischen Schrittabschnitt und Rückenabschnitt und zwischen Schrittabschnitt und Bauchabschnitt für den Schrittabschnitt dickere Materialien, also Materialien mit einem hohen Flächengewicht eingesetzt werden, so führt dies ebenso in nachteiliger Weise zu einer großflächigen Versteifung im Überlappungsbereich von Schrittabschnitt und Bauchabschnitt bzw. Rückenabschnitt.

Materialien mit geringen Flächengewichten sind aufgrund der damit zumeist verbundenen Flexibilität und damit auch der besseren Anpassbarkeit und insbesondere auch der Weichheit für die Ausgestaltung des Schrittabschnittes wünschenswert. Jedoch lassen sich derartige Materialien aufgrund ihrer zu geringen Eigenstabilität oft nur bedingt plan an andere Komponenten, wie dem Rückenabschnitt bzw. dem Bauchabschnitt anordnen, so dass aufgrund von nicht planaren, das heißt nicht faltenfreien Auflageflächen nachteiligerweise weniger stabile Verbindungen resultieren.

Hiervon ausgehend liegt der vorliegenden Erfindung die Aufgabe zugrunde, den vorstehend geschilderten Problemen zu begegnen, also insbesondere eine stabile Verbindung zwischen Schrittabschnitt und Bauchabschnitt und/ oder zwischen Schrittabschnitt und Rückenabschnitt bei einem gattungsgemäßen Inkontinenzartikel in Höschenform auszubilden, ohne dass dies mit einer Beeinträchtigung des Tragekomforts oder weiteren die Funktionalität des Inkontinenzartikels beeinträchtigenden Konsequenzen verbunden ist. Diese Aufgabe wird nach der Erfindung gelöst durch einen Inkontinenzartikel mit den Merkmalen des Anspruchs 1.

Dadurch, dass die H-förmige Grundstruktur des Inkontinenzartikels modular aus den Komponenten Schrittabschnitt, Bauchabschnitt und Rückenabschnitt gebildet wird und der Schrittabschnitt beidseits der Längsränder des Absorptionskörpers einen Überhang aus hochflexiblen, chassisbildenden Hüllmaterialien mit geringen Flächengewichten hat, erweist sich eine optimale Anbringung des Schrittabschnittes als schwierig, insbesondere wenn - wie bei der Herstellung moderner Inkontinenzartikel üblich - mit hohen Maschinengeschwindigkeiten, insbesondere in der Größenordnung von mehreren 100 m/min gearbeitet wird.

Inkontinenzartikel weisen zudem die Anpassbarkeit fördernde Konturen auf. Der Schrittabschnitt bzw. die Längsränder des Schrittabschnitts, welche die Beinöffnungen begrenzen, sind vorteilhafterweise bogenförmig konturiert. Weiter vorteilhaft können der Bauchabschnitt und Rückenabschnitt eine in Richtung Quermittelachse zulaufende Randkontur zur Begrenzung der Beinöffnungen aufweisen. Auch erweist es sich herstellungstechnisch als schwierig, eine saubere Konturierung bei einem beidseits der Längsränder des Absorptionskörpers vorhandenen Überhang aus chassisbildenden Hüllmaterialien mit geringen Flächengewichten zu erhalten.

Eine weitere Aufgabe der vorliegenden Erfindung ist die Bereitstellung eines auf wirtschaftliche Weise durchführbaren Verfahrens zum Herstellen eines Inkontinenzartikels in Höschenform mit den eingangs genannten Merkmalen, welches insbesondere den vorstehend genannten Aspekten der stabilen Verbindungen von Schrittabschnitt und Bauchabschnitt bzw. Rückenabschnitt unter Beibehaltung des Tragekomforts und der Funktionalität Rechnung trägt. Diese Aufgabe wird gelöst durch ein Verfahren zur Herstellung eines derartigen Inkontinzenzartikels mit den Verfahrensschritten gemäß Anspruch 15.

Vorteilhafte Weiterbildungen von Inkontinzenzartikel und Verfahren ergeben sich aus den jeweiligen Unteransprüchen.

Mit der Erfindung wurde erkannt, dass eine derartige Konstruktion eines Inkontinenzartikels in Höschenform wesentliche Vorteile mit sich bringt:

Dadurch, dass der Schrittabschnitt mit einer sehr ausladenden Breite in Querrichtung von wenigstens 200 mm ausgebildet wird und der Überhang des Deckblatt-Materials oder des Deckblatt- und Backsheet-Materials in Querrichtung jeweils außerhalb der Längsränder des Absorptionskörpers in der Summe, also beidseits der Längsränder des Absorptionskörpers, wenigstens 25% bezogen auf die größte Breite des Schrittabschnitts beträgt, wird ein großer Überlappungsbereich zwischen Schrittabschnitt und Bauchabschnitt einerseits und zwischen Schrittabschnitt und Rückenabschnitt andererseits realisiert. Aufgrund des großen Überhangs der Chassismaterialien des Schrittabschnitts in Querrichtung über den Absorptionskörper hinaus, wird außerdem ein flächenmäßig großer Überlappungsbereich zwischen Schrittabschnitt und Bauchabschnitt bzw. Rückenabschnitt außerhalb des Absorptionskörpers erreicht, was wiederum mit funktionstechnischen Vorteilen verbunden ist. Einerseits muss nämlich die Verbindung zwischen Schrittabschnitt und Bauchabschnitt bzw. Rückenabschnitt optimiert werden, andererseits soll hierdurch die Komponente des Absorptionskörpers möglichst wenig beeinflusst werden. Je größer der Überlappungsbereich von Schrittabschnitt und Bauchabschnitt bzw. von Schrittabschnitt und Rückenabschnitt außerhalb des Absorptionskörpers ist, umso größer sind die Freiheitsgrade im Hinblick auf die Ausbildung einer funktionstüchtigen Verbindung. Ein großer Überhang der Chassismaterialien in Querrichtung beidseits der Längsränder des Absorptionskörpers schafft auch in vorteilhafter Weise die Möglichkeit, zusätzliche Beinelastifizierungsmittel in Längsrichtung beidseits des Absorptionskörpers vorzusehen, die in einem größtmöglichen Abstand zum Absorptionskörper verlaufen. Hierdurch kann vorteilhafterweise verhindert werden, dass den Absorptionskörper verwindende, also auf Zug und Druck beanspruchende Kräfte durch die Beinelastifizierungsmittel ausgeübt werden. Solche unkalkulierbaren Kräfte wirken sich nämlich negativ aus das Absorptionsverhalten des Absorptionskörpers aus. Es kann solchenfalls zu unkontrollierter Verdichtung oder Aufweitung von Absorptionskörperbereichen kommen, welche das bestimmungsgemäße Absorptionsverhalten in nachteiliger Weise beeinflussen. In diesem Zusammenhang wird der verhältnismäßig große Überlappungsbereich zwischen Schrittabschnitt und Bauchabschnitt bzw. zwischen Schrittabschnitt und Rückenabschnitt auch dadurch gekennzeichnet, dass der Schrittabschnitt im vorderen Überlappungsbereich wenigstens 12 % der Fläche des Bauchabschnitts und im hinteren Überlappungsbereich wenigstens 20 % der Fläche des Rückenabschnitts überlappt.

Dadurch, dass für die Ausbildung des Schrittabschnitts ein Backsheet-Material bzw. ein Deckblatt-Material mit geringen Flächengewichten, nämlich von 10-40 g/m$^2$ bzw. 5 - 20 g/m$^2$ eingesetzt wird, wird die für den Anwender des Inkontinenzartikels in diesen empfindlichen Körperregionen gewünschte Weichheit, Anpassbarkeit und Drapierbarkeit realisiert.

Aufgrund der geringen Flächengewichte und der damit einhergehenden geringeren Eigenstabilität wird aber zum einen hierdurch die erhöhte Gefahr des Einreißens der Chassismaterialien geschaffen. Zum anderen bringen Chassismaterialien mit geringen Flächengewichten bei an sich großem Überhang auch den Nachteil mit sich, dass der Überhang eben aufgrund der geringen Eigenstabilität zu unerwünschten Fältelungen, also zu lokalen Materialanhäufungen sowohl in Quer- und/oder auch in Längsrichtung neigt. Nachteilig dabei ist, dass Fältelungen im Überhang zu einer verminderten Stabilität der Verbindung mit Bauchabschnitt und/oder Rückenabschnitt führen können.

Auch dahingehend, dass Bauchabschnitt und Rückenabschnitt in dem schrittseitig und den Beinöffnungen zugewandten Bereich mit zweiten Elastifizierungsmitteln versehen sind, welche gewünscht die Dehnbarkeit und die Anpassung an unterschiedliche Körpergrößen und -formen ermöglichen, ist eine weitere, aber unkoordinierte Fältelung der chassisbildenden Hüllmaterialien des Schrittabschnitts für eine optimale Verbindung nachteilig.

Es wurde erkannt, dass sich die in dem vorderen und/oder hinteren Überlappungsbereich durch die geringen Flächengewichte der Chassismaterialien des Schrittabschnitts bedingten problematischen Bereiche erfindungsgemäß durch ein Verstärkungsmittel im jeweiligen Überhang beidseits der Längsränder des Absorptionskörpers stabilisieren lassen. Das Verstärkungsmittel ist dabei in jeweils einem den jeweiligen Längsrand des Absorptionskörpers überbrückenden Bereich vorgesehen. Vorteilhaft dabei ist, dass das Verstärkungsmittel dabei sowohl jeweils einen Längsrandbereich des Absorptionskörpers überfängt, und dann über den Längsrand des Absorptionskörpers in den daran angrenzenden Teilbereich des Überhangs übergeht. Der Absorptionskörper selbst hat bedingt durch sein Flächengewicht eine im Vergleich zu den chassisbildenden Hüllmaterialien des Schrittabschnitts größere Eigenstabilität. Durch die Anordnung des Verstärkungsmittels im Längsrandbereich des Absorptionskörpers wird diese Eigenstabilität des Absorptionskörpers mittels des Verstärkungsmittels sozusagen in den jeweils angrenzenden Teilbereich des Überhangs weitergeleitet und dann durch das Verstärkungsmittel als solches fortgesetzt. Vorteilhaft wird dabei ausgehend vom Längsrandbereich über den Längsrand des Absorptionskörpers bis in den angrenzenden Teilbereich des Überhangs, und letztlich bis zum Längsrand des Schrittbereichs insgesamt eine Art "Verstärkungsprofil" mit abnehmender Stärke geschaffen. Dadurch, dass das Verstärkungsmittel nur in einem Teilbereich des Überhangs vorgesehen ist, das heißt, dass dieser Teilbereich eine geringere Breite aufweist als der Überhang, also vor den Längsrändern des Schrittabschnitts endet, wird eine Verhärtung dieser Randbereiche vermieden. Denn verhärtete Randbereiche würden zu Reizungen der Haut des Anwenders führen und damit den Tragekomfort des Inkontinenzartikels mindern. Des Weiteren ist die Ausbildung des Verstärkungsmittels in einem Teilbereich des Überhangs auch dahingehend vorteilhaft, dass je nach Ausführungsform des Verstärkungsmittels, insbesondere in der Ausführung eines Klebers, ein ungewollter produktionstechnisch bedingter Austritt des Verstärkungsmittels aus den chassisbildenden Hüllmaterialien vermieden wird.

Ebenso wird durch das Verstärkungsmittel in Form einer verstärkenden Beschichtung die in diesen Bereichen des Inkontinenzartikels wünschenswerte Anpassbarkeit beibehalten. Es wurde erkannt, dass der Einsatz von Beschichtungen mit Flächengewichten von 1 - 30 g/m$^2$ vorteilhaft ist, um eine genügend große Stabilität in Kombination mit den Chassismaterialien herbeizuführen, ohne dabei eine unnötige Versteifung zu verursachen.

Die Anordnung des Verstärkungsmittels auf der Innenseite des Deckblatt-Materials, also auf der vom Anwender abgewandten Oberseite und damit auf der dem Absorptionskörper zugewandten Oberseite des Deckblatt-Materials, ist vorteilhaft, da für den Anwender weiterhin die für die Haut angenehmen Eigenschaften der Vliesstruktur des Deckblatt-Materials beibehalten werden. Da das Verstärkungsmittel im Teilbereich des Überhangs eine im Vergleich zum Überhang in Querrichtung schmälere Erstreckung aufweist, dient das Deckblatt-Material als eine Art Schutzhülle für das Verstärkungsmittel. Andernfalls könnte es in der Anordnung auf der Außenseite, also auf der dem Anwender zugewandten Oberseite des Deckblatt-Materials möglicherweise aufgrund der Beschaffenheit des Verstärkungsmittels zu Unannehmlichkeiten für den Anwender kommen bzw. es müssten noch weitere zusätzliche Maßnahmen getroffen werden, um eine hautfreundliche Oberfläche des Verstärkungsmittels zu gewährleisten. Ebenso würde die Anordnung des in dem Teilbereich des Überhangs in Querrichtung mit einer schmäleren Erstreckung als der Überhang ausgebildeten Verstärkungsmittels auf der Außenseite des Deckblatt-Materials weitere Nachteile hervorrufen, in der Form, dass es durch die Bewegung des Anwenders hervorgerufenen Reibungskräfte zu einem teilweisen Ablösen des Verstärkungsmittels kommen könnte.

Vorteilhaft kann der Überhang in dem durch das Verstärkungsmittel überfangenen Teilbereich im vorderen und/oder hinteren Überlappungsbereich weitgehend faltenfrei auf dem Bauchabschnitt und/oder Rückenabschnitt angeordnet werden. Unter "weitgehend faltenfrei" wird dabei verstanden, dass die den Überhang bildenden Materiallagen selbst und auch in Relation zu den daran angrenzenden Materiallagen der damit zu verbindenden Komponenten, im flach ausgelegten und eben ausgebreiteten Zustand betrachtet, weitgehend keine lokalen Materialverschiebungen und Materialanhäufungen aufweisen, sondern die Materiallagen weitgehend plan aneinandergefügt sind. Vorteilhaft ist diese weitgehend faltenfreie Anordnung auch dahingehend, dass die durch die ersten und/oder zweiten Elastifizierungsmittel in den entsprechenden Bereichen gewünscht eingebrachten Rückstellkräfte und Fältelungen in deren Ausgestaltung durch die anderenfalls unerwünschten lokalen Materialansammlungen des Überhangs nicht nachteilig beeinflusst werden.

Unter "Überhang" wird hierbei die Erstreckung des Deckblatt-Materials oder des Deckblatt-Materials und des Backsheet-Materials seitlich außerhalb der Längsränder des Absorptionskörpers verstanden, wobei jeweils die maximale Erstreckung, also die von den Längsrändern des Absorptionskörpers am weitesten distal gelegene äußere Erstreckung des Deckblatt-Materials und/oder des Backsheet-Materials herangezogen wird. Das Backsheet-Material und/oder das Deckblatt-Material kann vorteilhafterweise aus mehreren Komponenten bestehen, so beispielsweise kann das Deckblatt-Material vorteilhafterweise ein Verbund aus einem Topsheet-Material und in Längsrichtung beidseits angrenzenden Barrieremitteln sein. Es wird also so verstanden, dass auch bei Verbunden, also zusammengesetzten Deckblatt-Materialien und/oder Backsheet-Materialien, bei der Betrachtung des Überhangs die jeweils maximale, also am weitesten distal gelegene äußere Erstreckung des Verbundes herangezogen wird.

Vorteilhafterweise haben das Backsheet-Material und das Deckblatt-Material dieselbe Erstreckung in Querrichtung. Sie sind kongruent, also deckungsgleich zueinander.

Weiter vorteilhaft ist aber auch, wenn das Backsheet-Material und Deckblatt-Material nicht kongruent zueinander sind. Insbesondere vorteilhafterweise hat das Backsheet-Material eine im Vergleich zum Deckblatt-Material schmälere Erstreckung in Querrichtung. Damit wird das den Anwender im Tragekomfort möglicherweise störende Backsheet-Material, wie beispielsweise eine Folie, durch das hautfreundliche Vliesmaterial des Deckblatt-Materials überdeckt.

Unter "Innenseite" des Deckblatt-Materials, des Topsheet-Materials, des Backsheet-Materials oder des Barrieremittels wird hierbei jeweils die dem Absorptionskörper zugewandte Oberseite des zu betrachtenden Materials verstanden.

Mit den Merkmalen des Patentanspruchs 1 wird insgesamt ein Inkontinenzartikel in Höschenform mit dem genannten dreikomponentigen Aufbau geschaffen, bei dem sich eine sichere Verbindung der Komponenten realisieren lässt, und zwar ohne dass dies mit einer Beeinträchtigung des Tragekomforts oder der Funktionalität des Inkontinenzartikels oder seiner Komponenten verbunden wäre.

In Weiterbildung der Erfindung erweist es sich als vorteilhaft, wenn nur der Überhang im hinteren Überlappungsbereich Verstärkungsmittel aufweist. Besonders bei Personen mit geringer Mobilität, so also auch bei bettlägrigen Personen, ist aufgrund des vorwiegenden Liegens auf der Rückenseite der hintere Überlappungsbereich besonders beansprucht. Durch das Einbringen von Verstärkungsmitteln werden vorteilhafterweise unerwünschte Fältelungen, welche nachteiligerweise auch zu Hautreizungen führen können, insbesondere damit im hinteren Überlappungsbereich, vermieden.

In einer weiteren vorteilhaften Weiterbildung ist das Verstärkungsmittel sowohl im vorderen als auch im hinteren Überlappungsbereich in jeweils einem den jeweiligen Längsrand des Absorptionskörpers überbrückenden Bereich angebracht. Damit kann ein vom späteren Einsatzbereich unabhängiger und damit universal einsetzbarer Inkontinenzartikel, der unterschiedlichen Anforderungsprofilen gerecht wird, bereitgestellt werden.

Es wird das Verständnis zugrunde gelegt, dass die im vorderen und/oder im hinteren Überlappungsbereich beidseits des Absorptionskörpers in Längsrichtung vorgesehenen Verstärkungsmittel vorzugsweise jeweils symmetrisch zueinander angeordnet sind und vorzugsweise auch jeweils die gleiche Ausgestaltung aufweisen, so beispielsweise also hinsichtlich Längenerstreckung, Breite, Überlappungsgrad, Materialzusammensetzung und/oder Flächengewicht übereinstimmen.

In Abhängigkeit des Anwendungsbereiches des Inkontinenzartikels und auch der Mobilität des Anwenders kann es vorteilhaft sein, das Verstärkungsmittel im vorderen und im hinteren Überlappungsbereich unterschiedlich zu gestalten. Vorzugsweise unterscheiden sich die Verstärkungsmittel im vorderen und hinteren Überlappungsbereich zumindest in einem der Parameter Längenerstreckung, Breite, Überlappungsgrad, Materialzusammensetzung und/oder Flächengewicht und/oder Kombinationen davon.

In Weiterbildung der Erfindung erweist es sich als vorteilhaft, wenn das Verstärkungsmittel sich in Längsrichtung zumindest bis zum Querrand des Absorptionskörpers und bis zum schrittzugewandten Querrand des Bauchabschnitts und/oder des Rückenabschnitts erstreckt. Durch diese Erstreckung des Verstärkungsmittels in Längsrichtung zwischen dem jeweiligen Querrand des Absorptionskörpers und dem jeweiligen schrittzugewandten Querrand des Bauchabschnitts oder des Rückenabschnitts werden zumindest die Bereiche entlang des Absorptionskörpers verstärkt und stabilisiert, welche andernfalls aufgrund unkontrollierter Fältungen des Überhangs einer damit bedingt weniger stabilen Verbindung mit den Komponenten des Bauchabschnitts und/oder Rückenabschnitts ausgesetzt wären.

In Abhängigkeit des Einsatzbereiches des Inkontinenzartikels und auch der Mobilität des Anwenders kann es vorteilhaft sein, das Verstärkungsmittel im vorderen und hinteren Überlappungsbereich unterschiedlich in seiner Längenerstreckung zu gestalten. Weiter vorzugsweise weist das Verstärkungsmittel im hinteren Überlappungsbereich eine größere Längenerstreckung auf als im vorderen Überlappungsbereich.

Besonders vorteilhaft ist, wenn das Verstärkungsmittel sich in Längsrichtung zumindest bis zum jeweiligen Querrand des Absorptionskörpers und bis zum schrittzugewandten Querrand sowohl des Bauchabschnitts als auch des Rückenabschnitts erstreckt.

In einer weiteren bevorzugten Ausführungsform erstreckt sich das Verstärkungsmittel ausgehend vom vorderen und/oder vom hinteren Überlappungsbereich über den jeweiligen schrittzugewandten Querrand des Bauchabschnittes und/oder Rückenabschnittes in Richtung Quermittelachse.

In einer insbesondere weiteren bevorzugten Ausführungsform erstrecken sich das Verstärkungsmittel im vorderen Überlappungsbereich und das Verstärkungsmittel im hinteren Überlappungsbereich sich miteinander verbindend in Richtung Quermittelachse.

In vorteilhafter Weise weist das Verstärkungsmittel in dem vom Verstärkungsmittel überfangenen Teilbereich in Querrichtung des Überhangs eine Breite G' von größer 5 mm, vorzugsweise größer 10 mm, aber vorzugsweise kleiner 50 mm, vorzugsweise kleiner 40 mm, weiter vorzugsweise kleiner 30 mm auf.

In weiterer vorteilhafter Weise beträgt der Anteil G'/ H des durch das Verstärkungsmittel überfangenen Teilbereiches des jeweiligen Überhangs mit der Breite G' bezogen auf den jeweiligen Überhang mit der Breite H mindestens 0,10, insbesondere mindestens 0,15, weiter insbesondere mindestens 0,20, aber vorzugsweise höchstens 0,80, insbesondere höchstens 0,75, weiter insbesondere höchstens 0,70. Durch diesen Anteil des Verstärkungsmittels im jeweiligen Überhang kann eine ausreichende Verstärkung der chassisbildenden Hüllmaterialien des Schrittabschnitts erreicht werden, aber auch unter Vermeidung von übermäßigem Einsatz an zusätzlichen Materialien und damit von Kosten.

Weiter vorteilhaft überfängt das Verstärkungsmittel den Längsrandbereich des Absorptionskörpers in Querrichtung jeweils mit einer Breite G" von größer 5 mm, vorzugsweise größer 10 mm, aber vorzugsweise von kleiner 50 mm, vorzugsweise kleiner 40 mm, weiter vorzugsweise kleiner 30 mm.

Insbesondere vorteilhaft beträgt der Anteil G"/ H des jeweiligen durch das Verstärkungsmittel überfangenen Längsrandbereichs mit der Breite G" bezogen auf den jeweiligen Überhang mit der Breite H mindestens 0,10, insbesondere mindestens 0,15, weiter insbesondere mindestens 0,20 , aber vorzugsweise höchstens 0,80, insbesondere höchstens 0,75, weiter insbesondere höchstens 0,70.

Insbesondere vorteilhaft ist, wenn das Verstärkungsmittel den Längsrandbereich des Absorptionskörpers in Querrichtung in Summe, also beidseits, in einem Anteil von mindestens 5%, insbesondere mindestens 10% , weiter insbesondere mindestens 15% und höchstens 35%, insbesondere höchstens 30%, weiter insbesondere höchstens 25% bezogen auf die Breite K des Absorptionskörpers überfängt.

Insbesondere vorteilhaft beträgt der Anteil G"/K des jeweiligen durch das Verstärkungsmittel überfangenen Längsrandbereichs mit der Breite G" bezogen auf den Absorptionskörper mit der Breite K mindestens 0,02, insbesondere mindestens 0,04, weiter insbesondere mindestens 0,06 , aber vorzugsweise höchstens 0,30, insbesondere höchstens 0,25, weiter insbesondere höchstens 0,20.

Durch die im Wesentlichen nur längsrandbereichständige Überlappung des Verstärkungsmittels mit dem Absorptionskörper wird die durch den Absorptionskörper zur Aufnahme von Flüssigkeiten bereitgestellte Fläche nur in einem geringen Maße beeinträchtigt. Dennoch wird mit diesem geringen Anteil der Überlappung des Verstärkungsmittels über den Absorptionskörper ein genügend großer Bereich zum Erreichen einer gewissen Übertragung der Eigenstabilität des Absorptionskörpers auf die Kombination von Verstärkungsmittel mit den chassisbildenden Hüllmaterialien des Schrittabschnitts erhalten.

Insbesondere vorteilhaft weist das Verstärkungsmittel eine Gesamtbreite G von 10 - 60 mm, insbesondere von 15 - 55 mm, weiter insbesondere von 20 - 50 mm, weiter insbesondere von 20 - 40 mm auf.

In einer weiteren vorteilhaften Weise ist das Verstärkungsmittel parallel zur Längsrichtung und als Streifen mit einer gleich bleibenden Breite angeordnet. Derart kann das Verstärkungsmittel mit geringem technischen Aufwand und damit auch schneller und kostengünstiger in den Inkontinenzartikel eingebracht werden.

Das Verstärkungsmittel in Form einer Beschichtung wurde als vorteilhaft erkannt, da die Beschichtung materialspezifisch bedingt auch zumindest teilweise in die Poren des Deckblatt-Materials auf Vliesbasis eindringen bzw. Faseroberflächen umschließen kann und damit eine vorteilhafte Verbundkraft zu den chassisbildenden Hüllmaterialien des Schrittabschnitts herstellt.

Vorteilhafterweise sind die verstärkenden Beschichtungen entnommen aus der Gruppe der nicht-adhesiven oder adhesiven Beschichtungen. In einer besonders vorteilhaften Weiterbildung der Erfindung umfasst die verstärkende Beschichtung einen Kleber, insbesondere einen Hotmeltkleber.
Als Kleber können beispielsweise vorzugsweise eingesetzt werden: D 9105 ZP oder LC 3001 ZP (H.B. Fuller Deutschland GmbH, An der Roten Bleiche 2-3, 21335 Lüneburg, Deutschland); H20028 oder H 2481 (Bostik Nederland B.V., Zeggeveld 10, 4705 RP Roosendaal, Niederlanden); Technomelt Q2415 oder Technomelt Q5430 (Henkel KGaA, 40191 Düsseldorf, Deutschland).

Insbesondere werden Kleber eingesetzt, welche in einem kontaktlosen Auftragsverfahren aufgetragen werden können. Insbesondere vorteilhaft kann der Hotmeltkleber LC 3001 ZP von der Firma Fuller (H.B. Fuller Deutschland GmbH, An der Roten Bleiche 2-3, 21335 Lüneburg, Deutschland) eingesetzt werden.

Weiter insbesondere ist der Kleber bzw. Hotmeltkleber hydrophob. Dies ist vorteilhaft, da zusätzlich zur verstärkenden Funktion gleichzeitig eine Flüssigkeitsbarriere gebildet wird.

Der Einsatz von Kleber ist vorteilhafterweise auch geeignet, die haftvermittelnde Wirkung der Fügemittel, welche Backsheet-Material und/oder Deckblatt-Material und/oder Absorptionskörper miteinander verbinden, zu stärken. Vorzugsweise sind Backsheet-Material und Deckblatt-Material im Bereich des Überhangs direkt durch separate Fügemittel wie Kleber, Kalanderschweißen oder Ultraschallschweißen verbunden, insbesondere bis hin zum Längsrand des Schrittabschnitts. Auch zwischen Backsheet-Material und Absorptionskörper und/oder zwischen Deckblatt-Material und Absorptionskörper sind vorzugsweise derartige separate Fügemittel vorgesehen. Diese Fügemittel sind vorzugsweise nicht vollflächig, sondern in Form eines unterbrochenen Musters vorgesehen. Soweit als für derartige separate Fügemittel ein Kleber vorgesehen ist, kann dieser beispielsweise streifenförmig, rasterförmig oder als Spiralmuster angeordnet sein.

Weiter wurde als vorteilhaft erkannt, die verstärkende Beschichtung in einem Flächengewicht von insbesondere 2 - 20 g/m², weiter insbesondere 2 -10 g/m² einzusetzen.
Die verstärkende Beschichtung ist vorteilhafterweise vollflächig aufgetragen.

In einer weiteren vorteilhaften Ausbildung ist das Deckblatt-Material ein Verbund aus einem flüssigkeitsdurchlässigen Topsheet-Material mit Längsrändern und angrenzenden Längsrandbereichen und beidseits an den Längsrändern bzw. Längsrandbereichen des Topsheet-Materials in Fügestellen angefügten hydrophoben Barrieremitteln. Durch diesen Verbund werden in einem Inkontinenzartikel die bereichsweise unterschiedlichen Anforderungsprofile, nämlich im mittigen Bereich eine Flüssigkeitsaufnahme und an den Randbereichen eine Retardierung des seitlichen Flüssigkeitsaustritts, bereitgestellt.

In einer weiteren Ausführung erstreckt sich das hydrophobe Barrieremittel über die Längsränder des Topsheet-Materials hinaus, und zwar unter Ausbildung eines jeweils beidseits des Absorptionskörpers in Längsrichtung verlaufenden jeweils aufstehenden Barrieremittels, welches typischerweise als Cuffelement oder Bündchenelement bezeichnet wird. Die distalen Enden der Barrieremittel sind vorteilhafterweise mit Elastifizierungsmitteln versehen. Damit werden die Barrieremittel im Gebrauch des Inkontinenzartikels gegen die Hautoberfläche des Benutzers angehoben

Die Fixierung der Materialbahnen des Deckblatt-Material-Verbundes in den Fügestellen kann vorzugsweise mittels Kleber, insbesondere Hotmeltkleber, thermisches Kalandern (Thermobonding) oder Ultraschallschweißen erfolgen. Die Fixierung kann in Form von kontinuierlichen Fügestellen ausgeführt sein, um eine hohe Verbundkraft zwischen dem Topsheet-Material und dem Barrieremittel zu erzielen. Denkbar ist dabei eine durchgehende Linie. Denkbar und vorteilhaft ist jedoch auch eine Fixierung durch intermittierend angebrachte Fügestellen, also durch eine Abfolge von diskreten Bindepunkten oder Bindelinien oder jedem anderen Bindemuster.

In einer besonders bevorzugten Ausführungsform überfängt das Verstärkungsmittel die Fügestellen des Deckblatt-Materials. Dadurch werden vorteilhafterweise auch die in dem Deckblatt-Material durch die Fügestellen bedingten, möglicherweise problematischen, so nämlich der Gefahr des Einreißens, aber auch des Flüssigkeitsaustritts ausgesetzten Bereiche, auf synergistische Weise mit dem Verstärkungsmittel ebenso verstärkt und stabilisiert.

Des weiteren erweist es sich als vorteilhaft, wenn der Überhang des Backsheet-Materials und/oder des Deckblatt-Materials in Querrichtung in der Summe, also beidseits der Längsränder des Absorptionskörpers 25 - 50 %, insbesondere 30 - 45 % und weiter insbesondere 35 - 45 % bezogen auf die größte Breite E des Schrittabschnitts beträgt.

In Weiterbildung der Erfindung erweist es sich als vorteilhaft, wenn der Anteil der Fläche des Schrittabschnitts an der gesamten Fläche des Inkontinenzartikels 25 - 55 %, insbesondere 30 - 47 %, weiter insbesondere 35 - 47 % und weiter insbesondere 35 - 45 % beträgt.

Der Überlappungsbereich zwischen Schrittabschnitt und Bauchabschnitt ist in Weiterbildung der Erfindung so ausgebildet, dass der Schrittabschnitt 15 - 40 %, insbesondere 15 - 35 % und weiter insbesondere 15 - 25 % der Fläche des Bauchabschnitts überlappt. In vorteilhafter Weise überlappt der Schrittabschnitt den Bauchabschnitt mit einer Fläche von 25.000 - 45.000 mm².

Der Überlappungsbereich von Schrittabschnitt und Rückenabschnitt ist in Weiterbildung der Erfindung so ausgebildet, dass der Schrittabschnitt 20 - 40 %, insbesondere 20 - 35 % und weiter insbesondere 22 - 32 % der Fläche des Rückenabschnitts überlappt. In vorteilhafter Weise überlappt der Schrittabschnitt den Rückenabschnitt mit einer Fläche von 35.000 - 65.0000 mm², insbesondere von 40.000 - 55.000 mm².

Die Überlappung des Schrittabschnitts mit dem Rückenabschnitt ist in vorteilhafter Weise größer als die Überlappung des Schrittabschnitts mit dem Bauchabschnitt.

Es erweist sich des Weiteren als besonders vorteilhaft, dass der Schrittabschnitt mittels eines nicht vollflächigen Kleberauftrags mit dem Bauchabschnitt und/oder mit dem Rückenabschnitt verbunden werden kann. Es hat sich nämlich gezeigt, dass bei Verwendung eines nicht vollflächigen Kleberauftrags die Eigenschaften der Chassismaterialien in geringerem Maße beeinflusst werden als wenn ein vollflächiger Kleberauftrag zur Ausbildung der Verbindung von Schrittabschnitt und Bauchabschnitt bzw. Schrittabschnitt und Rückenabschnitt verwendet wird. Bei einem nicht vollflächigen Kleberauftrag kann es sich beispielsweise um ein streifenförmiges Muster, um eine stegförmige kontinuierliche oder nicht kontinuierliche Gitterstruktur oder um inselförmige Bereiche oder aber um eine streifenförmige oder spiralförmig angeordnete Kleberstruktur handeln.

Es ist bei der erfindungsgemäßen Ausbildung des Inkontinenzartikels möglich und vorteilhaft, wenn auch der Absorptionskörper 5 - 20%, insbesondere 5 - 15 % der Fläche des Bauchabschnitts und/oder 10 - 20 %, insbesondere 10 - 15 % der Fläche des Rückenabschnitts überlappt.

Die Erstreckung des Bauchabschnitts und des Rückenabschnitts im Seitennahtbereich in Längsrichtung beträgt vorteilhafterweise wenigstens 100 mm, insbesondere wenigstens 150 mm und insbesondere 150 mm bis 220 mm.

Der minimale Abstand des Bauchabschnitts und des Rückenabschnitts in Längsrichtung voneinander beträgt vorteilhafterweise 250 bis 400 mm.

Die maximale Erstreckung des Schrittabschnitts in Querrichtung, also die größte Breite, beträgt vorteilhafterweise 200 bis 350 mm, insbesondere 250 bis 320 mm.

Der verhältnismäßig große Überhang von Backsheet-Material und/oder Deckblatt-Material beidseits der Absorptionskörpers bedeutet also einen breiten Schrittabschnitt mit einem verhältnismäßig schmalen Absorptionskörper. Hierdurch ist es möglich, entlang der Beinöffnungen erstreckte Beinelastifizierungsmittel in dem Schrittabschnitt vorzusehen, die einen verhältnismäßig großen Abstand zum materialreichen und damit biegesteifen Absorptionskörper haben. Dies resultiert wiederum in einer guten Abdichtbarkeit und Anpassbarkeit der beidseitigen Beinöffnungsränder des Schrittabschnitts. Solchenfalls behindert nämlich der materialreiche und gegenüber dünnen Chassismaterialien verwindungssteife Absorptionskörper die Ausbildung eines flüssigkeitsdichten Beinabschlusses nur wenig; es muss daher zur Ausbildung eines flüssigkeitsdichten Beinabschlusses nicht mit extrem hohen Spannungen gearbeitet werden, was sich wiederum positiv auf den Tragekomfort des Inkontinenzartikels auswirkt.

In noch weitergehender Ausbildung der Erfindung erweist es sich als besonders vorteilhaft, wenn die Beinelastifizierungsmittel in Längsrichtung wenigstens 10 mm, insbesondere wenigstens 20 mm vor den zweiten Elastifizierungsmitteln enden. Insbesondere vorteilhaft ist, wenn die Beinelastifizierungsmittel in Längsrichtung vor dem Bauchabschnitt und/oder vor dem Rückenabschnitt enden. Die von ihnen ausgeübte Spannung und Rückstellkraft beeinflusst daher nicht Spannungsverhältnisse der zweiten Elastifizierungsmittel. Insbesondere werden nicht die Spannungsverhältnisse innerhalb des schrittseitigen und den Beinöffnungen zugewandten Bereichs des Bauchabschnitts und des Rückenabschnitts, in dem die zweiten Elastifizierungsmittel sich auffächernden vorgesehen sind, beeinflusst.

Weiter vorteilhaft ist, wenn die Beinelastifizierungsmittel der Kontur des Absorptionskörpers folgen.

Weiter ist es vorteilhaft, wenn die Beinelastifizierungsmittel mit veränderlichem Abstand zum Absorptionskörper verlaufen und an ihren Längsenden einen größeren Abstand zum Absorptionskörper aufweisen als mittig.

Als Beinelastifizierungsmittel werden vorzugsweise faden- oder bandförmige Elastifizierungsmittel, wie Gummi- oder Polyetherpolyurethan- oder Polyesterpolyurethanfäden, vorzugsweise Elastikfäden wie Lycra®-, Creora®- oder Spandex®-Fäden eingesetzt. Die Beinelastifizierungsmittel haben vorzugsweise eine Stärke von 300 - 1500 dtex, insbesondere von 500 - 1200 dtex, weiter insbesondere von 500 - 900 dtex.

Die Beinelastifizierungsmittel werden vorzugsweise unter einer Vorspannung von 1,5 - 6,0, insbesondere von 2,5 - 4,5 an den chassisbildenden Hüllmaterialien des Schrittabschnitts fixiert. Die Vorspannung ist definiert als Faktor des Dehnungsgrades gegenüber dem ungedehnten/relaxierten Zustand des Elastifizierungsmittels.

In Weiterbildung der Erfindung im Zuge der Entwicklung von Inkontinenzartikeln in Höschenform wurde erkannt, dass möglichst weitgehende Bereiche des Inkontinenzartikels elastisch nachgiebig, also entsprechend einer Körperform des Benutzers dehnbar, ausgebildet werden sollten. Dies führte zu der nicht immer zutreffenden Annahme, dass ein größtmöglicher Teil der hüllenbildenden Chassismaterialien des Inkontinenzartikels mit elastisch dehnbaren oder elastifizierten Materialien, insbesondere durch Einbringen vorgenannter Elastifizierungsmittel im Stretch-Bond-Verfahren, ausgebildet werden sollten. Dessen ungeachtet bzw. zusätzlich war man bestrebt, die Beinöffnungen des Inkontinenzartikels möglichst durchgehend zu elastifizieren, um einen sicheren Seitenauslaufschutz zu erreichen.

Es wurde aber nicht erkannt, dass die elastische Ausbildung von Chassismaterialien, insbesondere durch Einbringen von linear erstreckten Elastifizierungsmitteln auch mit nicht unerheblichen Nachteilen verbunden ist. Zwar kann mit einer flächenhaften, also im Wesentlichen über die Erstreckung durchgehenden Elastifizierung von Chassismaterialien erreicht werden, dass sich der Inkontinenzartikel in Höschenform überhaupt in gewisser Weise an unterschiedliche Körperformen anpassen lässt, der Artikel also überhaupt in bestimmungsgemäßer Form an einem Benutzer positionierbar ist. Dies ist jedoch häufig mit der Ausbildung sehr starker Zugspannungen in den Chassismaterialien verbunden, die sich in unangenehmer Weise auf den Benutzer auswirken. Hierbei schneiden die Elastifizierungsmittel oftmals in die Hautoberfläche des Benutzers ein, was als unangenehm empfunden wird und sogar Schmerzen, Hautreizungen und, insbesondere in Verbindung mit einem humiden Klima, sogar Hautverletzungen erzeugen kann. Unter hoher Dehnungsspannung stehende Chassismaterialien liegen häufig eng gegen die Hautoberfläche des Benutzers an, was auch bei Verwendung atmungsaktiver Materialien zu einem feuchten Mikroklima im-Bereich der Hautoberfläche führt und ebenfalls fatale Folgen im Hinblick auf die Hautoberfläche des Benutzers haben kann. Solchenfalls bildet sich unmittelbar angrenzend an die Hautoberfläche nämlich keine Gasphase sondern eine Flüssigkeitsphase aus. Ungeachtet dessen führt eine starke Spannung im Bereich der Elastifizierungsmittel zu starker Rüschenbildung, also zu einer großen Anzahl von Falten oder Fältelungen pro Zentimeter (betrachtet in Richtung der elastischen Hauptwirkung der Elastifizierungsmittel). Diese dreidimensional gewellte Struktur wird dann unter großer Spannung, welche durch die Elastifizierungsmittel ausgeübt wird, gegen die Hautoberfläche gedrückt und verursacht insbesondere bei hoher Mobilität des Benutzers Relativbewegungen, die wiederum die Hautoberfläche reizen und zu unangenehmen bis hin zu medizinisch problematischen Hautreizungen führen.

Wann immer Elastifizierungsmittel in Bezug auf eine Maschinenrichtung bogenförmig oder quer geführt werden, was zur Erreichung einer flächenhaften, im Wesentlichen die gesamten Chassismaterialien erfassenden Elastifizierung häufig angewandt wird, tritt das Problem auf, dass in Folge der Komponente quer zur Maschinenrichtung ein größerer Weg zurückgelegt werden muss und damit eine stärkere Vorspannung der Elastifizierungsmittel im Zuge des Stretch-Bond-Verfahrens einhergeht. Dies führt typischerweise zu einem stärkeren elastischen Dehnungswiderstand der entsprechenden Chassisbereiche verglichen mit Bereichen, in denen die Elastifizierungsmittel in Maschinenrichtung verlaufend eingebracht sind, was sich wiederum in höchstem Maße problematisch auswirken kann.

Nach einem weiteren Erfindungsgedanken sind zur flächenhaften Elastifizierung von Bauchabschnitt und Rückenabschnitt sind die jeweils in einem Abstand voneinander und parallel zueinander in Quer- oder Hüftumfangsrichtung erstreckten ersten Elastifizierungsmittel vorgesehen. Diese haben vorzugsweise gleiche Vorspannung und dienen im Wesentlichen einer flächenhaft durchgehenden gleichmäßigen Elastifizierung des Bauchabschnitts und des Rückenabschnitts in dem Bereich deutlich oberhalb der Beinöffnungen. Es ist indessen möglich, dass die ersten Elastifizierungsmittel in einem oberen Hüftrandbereich eine stärkere Vorspannung aufweisen oder mehrere dieser Elastifizierungsmittel mit geringerem Abstand voneinander vorgesehen sind, um eine etwas stärkere Elastifizierung am Hüftrand zu realisieren.

Es wurde unter anderem erkannt, dass die Spannungsverhältnisse in dem genannten schrittseitigen und den Beinöffnungen zugewandten Bereich des Bauchabschnitts und des Rückenabschnitts für die vorstehend geschilderten Probleme verantwortlich sind und vorzugsweise so gestaltet werden können, dass sich die genannten Probleme nicht oder in geringerem Maße stellen. Vorteilhaft ist der schrittseitige und den Beinöffnungen zugewandte Bereich, in welchem die zweiten Elastifizierungsmittel sich in Richtung auf die Längsmittelachse auffächern, so ausgebildet, dass die bei flächenhafter Dehnung dieses Bereichs auftretende Rückstellkraft geringer ist als bei flächenhafter Dehnung in einem hüftseitigen Bereich, in welchem nur die ersten Elastifizierungsmittel vorgesehen sind. Mit dem Begriff "hüftseitiger Bereich" ist gemeint, dass sich dieser Bereich in Längsrichtung außerhalb des schrittseitigen und den Beinöffnungen zugewandten Bereichs mit den sich auffächernden zweiten Elastifizierungsmitteln befindet. Es handelt sich bei der Rückstellkraft um diejenige Kraft, welche der Bauchabschnitt und der Rückenabschnitt einer flächenhaften Dehnung in Richtung des Verlaufs der Elastifizierungsmittel entgegensetzen. Es ist in Weiterbildung der Erfindung als wesentlich erkannt worden, dass der schrittseitige und den Beinöffnungen zugewandte Bereich bei flächenhafter Dehnung entlang der zweiten Elastifizierungsmittel vorteilhaft mit geringerer Rückstellkraft ausgebildet wird als daran in Längsrichtung angrenzende Bereiche, die weiter vom Schritt und den Beinöffnungen entfernt sind, beispielsweise insbesondere die Bereiche, in denen die ersten Elastifizierungsmittel angeordnet sind. Hierdurch wird ein besserer Tragekomfort des Inkontinenzartikels erreicht, und es kommt in geringerem Umfang zu den geschilderten Problemen, weil der Inkontinenzartikel im Bereich des Schritts bzw. im Bereich der Beinöffnungen der Körperform entsprechend auch in größerem Umfang gedehnt werden kann, ohne dass es gleich zu einer unangenehmen Erhöhung der Rückstellkräfte mit den eingangs geschilderten Konsequenzen kommt.

Zur Bestimmung der Rückstellkräfte können die zu vermessenden Bereiche des Chassis direkt, gleichsam zerstörungsfrei zwischen zwei Klemmbacken von definierter, gleicher Klemmbackenbreite fest eingespannt und die Rückstellkräfte bei einer definierten, den Gebrauchszustand simulierenden Dehnung der zu messenden Bereiche um insbesondere 30% oder 50% oder 80% der Ausgangslänge (des Klemmbackenabstandes bei Fixieren des zu messendes Bereiches in ungespanntem Zustand) ermittelt werden. Die Klemmbacken sollten möglichst viele, zumindest jedoch zwei nebeneinander angeordnete Elastifizierungsmittel des zu messenden Bereiches fixieren und im Wesentlichen senkrecht zum Verlauf der Elastifizierungsmittel orientiert sein, so dass die Dehnung zwischen den Klemmen im Wesentlichen in Richtung des Verlaufs der Elastifizierungsmittel erfolgt.

In noch weitergehender Ausbildung der vorliegenden Erfindung wurde auch erkannt, dass die Spannungsverhältnisse in dem genannten schrittseitigen und den Beinöffnungen zugewandten Bereich des Bauchabschnitts und des Rückenabschnitts im Hinblick auf den Tragekomfort wesentlich sind und so gestaltet werden können, dass der Tragekomfort verbessert wird.

Vorteilhafterweise erstrecken sich die zweiten Elastifizierungsmittel ausgehend von den beiden Seitennahtbereichen in Richtung auf eine Längsmittelachse des Inkontinenzartikels und verlaufen dabei bogenförmig sich auffächernd mit zunehmendem Abstand voneinander.

Hierfür ist der schrittseitige und den Beinöffnungen zugewandte Bereich, in welchem die zweiten Elastifizierungsmittel sich in Richtung auf die Längsmittelachse auffächern, so ausgebildet, dass bei flächenhafter Dehnung dieses Bereichs die dabei auftretende Rückstellkraft in Richtung auf den Schrittabschnitt abnimmt.

Betrachtet man also diesen schrittseitigen und den Beinöffnungen zugewandten Bereich des Bauchabschnitts und des Rückenabschnitts, und zwar in einer Richtung ausgehend von dem jeweiligen Seitennahtbereich in Richtung auf den Schrittabschnitt, also in Richtung auf eine Längsmittelachse des Inkontinenzartikels und gewissermaßen in Richtung der bogenförmigen Auffächerung der zweiten Elastifizierungsmittel, so wird die bei flächenhafter Dehnung auftretende Rückstellkraft in dieser Richtung reduziert. Es handelt sich also hierbei um diejenige Kraft, welche der Bauchabschnitt und der Rückenabschnitt einer flächenhaften Dehnung entgegensetzen. Eine Abnahme dieser Rückstellkraft, die sich dann naturgemäß auf den Benutzer überträgt, ist mit einer erheblichen Verbesserung des Tragekomforts des Inkontinenzartikels verbunden.

Es erweist sich weiter als besonders vorteilhaft, wenn die Abnahme der Rückstellkraft in dem genannten schrittseitigen und den Beinöffnungen zugewandten Bereich des Bauchabschnitts und des Rückenabschnitts so vorgesehen wird, dass in Richtung auf den Schrittabschnitt eine abnehmende Anzahl von Falten pro cm-Querrichtung des Inkontinenzartikels gebildet wird. Solchenfalls können sich der Bauchabschnitt und der Rückenabschnitt entsprechend der Körperform des Benutzers dehnen, ohne dass die hierdurch gebildeten elastischen Kräfte das Chassismaterial mit einer Vielzahl von Falten zu raffen versuchen. Es sei an dieser Stelle nochmals erläutert, dass die Abnahme der Rückstellkraft in Richtung auf den Schrittabschnitt bedeutet, dass diejenige Kraft, die infolge einer flächenhaften Dehnung erzeugt wird, mit zunehmender Annäherung an den Schrittabschnitt geringer wird. Die Rückstellkraft infolge flächenhafter Dehnung ist also in einem Gebiet näher der Seitennaht größer als in einem Gebiet näher dem Schrittabschnitt.

Die genannten Spannungsverhältnisse lassen sich in verschiedenster Weise erreichen, etwa durch Verwendung von in Querrichtung unterschiedlich elastischen Materialien in dem schrittseitigen und den Beinöffnungen zugewandten Bereich, in welchem auch die zweiten Elastifizierungsmittel vorgesehen sind. Es wäre auch denkbar, dass die Vorspannung der zweiten Elastifizierungsmittel mit zunehmender Annäherung an den Schrittabschnitt, also von außen nach innen in Richtung auf eine Längsmittelachse reduziert wird. Außerdem wäre es denkbar, dass die Abnahme der Rückstellkraft bei flächenhafter Dehnung dadurch erreicht wird, dass der Abstand der zweiten Elastifizierungsmittel voneinander zunimmt, wobei hier darauf zu achten ist, dass dies nicht mit einer starken Zunahme der Vorspannung infolge des fächerförmigen Verlaufs der Elastifizierungsmittel ausgeglichen oder gar in Richtung zunehmender Rückstellkraft übertroffen wird.

Es hat sich insbesondere als vorteilhaft erwiesen, wenn ein minimaler Abstand der zweiten Elastifizierungsmittel voneinander (Abstand von unmittelbar nebeneinander liegenden Elastifizierungsmitteln) in den Seitennahtbereichen 3 bis 8 mm, insbesondere 3 bis 7 mm und weiter insbesondere 3 bis 6 mm beträgt.

Des Weiteren hat es sich als vorteilhaft erwiesen, wenn ein maximaler Abstand der zweiten Elastifizierungsmittel voneinander (Abstand von unmittelbar nebeneinander liegenden Elastifizierungsmitteln) an einem Absorptionskörperrand oder an einem Längsrand des Schrittabschnitts 7 bis 35 mm, insbesondere 10 bis 32 mm und weiter insbesondere 12 bis 30 mm beträgt.

Des Weiteren hat es sich als vorteilhaft erwiesen, wenn die zweiten Elastifizierungsmittel einen Auffächerungsgrad F

$$F=(A-B)/B * 100\%$$

von 50 bis 900 %, insbesondere von 100 bis 700 % und weiter insbesondere von 150 bis 550 % haben.

Dabei ist der Auffächerungsgrad F als Verhältnis der Abstandszunahme (A-B) zum minimalen Abstand (B) in Prozent definiert. Dabei sind die Größen A und B definiert als der Abstand des in Längsrichtung äußersten zweiten Elastifizierungsmittels von dem in Längsrichtung innersten zweiten Elastifizierungsmittel (also nicht der Abstand von unmittelbar nebeneinander liegenden zweiten Elastifizierungsmitteln), und zwar A als der maximale Abstand, insbesondere am Längsrand des Schrittabschnitts oder am Absorptionskörperrand, und B als der minimale Abstand insbesondere im Seitennahtbereich. Es wurde auch erkannt, dass es sich als vorteilhaft erweist, wenn der Auffächerungsgrad F der zweiten Elastifizierungsmittel im Rückenabschnitt größer ist als im Bauchabschnitt.

Aufgrund der naturbedingten Körperformen im Rückenbereich bzw. Bauchbereich eines Benutzers erweisen sich die hier thematisierten Probleme typischerweise im Rücken- oder Gesäßbereich als gravierender. Insofern erweist es sich als vorteilhaft, wenn der maximale Abstand der zweiten Elastifizierungsmittel voneinander an einem Absorptionskörperrand im Rückenabschnitt größer ist als im Bauchabschnitt.

Es wäre durchaus denkbar, dass die zweiten Elastifizierungsmittel von dem einen Seitennahtbereich zu dem anderen Seitennahtbereich durchgehend verlaufen, was insbesondere die Einbringung in einem kontinuierlichen Herstellungsverfahren im Vergleich zu einem "cut-and-place"-Prozess vereinfacht. Infolge der Überdeckung des Schrittabschnitts mit dem Bauchabschnitt und mit dem Rückenabschnitt kann es je nach Gestaltung auch zu einer Überlappung oder Überdeckung des materialreichen Absorptionskörpers mit dem Bauchabschnitt und/oder dem Rückenabschnitt kommen und somit auch mit demjenigen schrittseitigen und den Beinöffnungen zugewandten Bereich des Bauchabschnitts und des Rückenabschnitts, in dem die zweiten Elastifizierungsmittel verlaufen. Der materialreiche Absorptionskörper behindert dabei üblicherweise eine elastische Dehnbarkeit der Chassismaterialien. Außerdem ist es nicht unbedingt vorteilhaft, wenn der materialreiche Absorptionskörper mit zusätzlichen Spannungskräften beaufschlagt wird. Deshalb kann es sich als vorteilhaft erweisen, wenn die zweiten Elastifizierungsmittel in einem Überlappungsbereich mit dem Absorptionskörper des Schrittabschnitts hinsichtlich ihrer elastischen Eigenschaften deaktiviert sind. Diese Deaktivierung kann beispielsweise durch eine Anzahl von Trennschnitten durch die zweiten Elastifizierungsmittel im Bereich der Überdeckung mit dem Absorptionskörper realisiert werden, wobei auch andere Trennverfahren, wie z. Bsp, mittels Ultraschallschweißen oder Laser denkbar sind.

Es sei erwähnt, dass auch die ersten Elastifizierungmittel im Bereich einer Überdeckung mit dem Absorptionskörper hinsichtlich ihrer elastischen Eigenschaften deaktiviert sein können.

Es wurde bereits darauf hingewiesen, dass die zweiten Elastifizierungsmittel ungeachtet der vorzugsweise herbeizuführenden Spannungsverhältnisse entsprechend dem Weg ihres aufgefächerten Verlaufs bei der Herstellung des Inkontinenzartikels einer stärkeren Dehnung und damit einer höheren Vorspannung ausgesetzt sein können als in einem nicht aufgefächerten Bereich, in welchem sie sich im Wesentlichen äquidistant zueinander und in der Maschinenrichtung erstrecken. Diese stärkere Vorspannung kann sich typischerweise infolge des Einbringens der zweiten Elastifizierungsmittel in einem üblichen und daher nicht im Einzelnen zu beschreibenden Stretch-Bond-Verfahren ergeben.

Hinsichtlich der Gesamtabmessungen des Inkontinenzartikels erweist es sich als vorteilhaft, wenn der Abstand (C) des schrittzugewandten innersten zweiten Elastifizierungsmittels des Bauchabschnitts von dem entsprechenden schrittzugewandten innersten zweiten Elastifizierungsmittel des Rückenabschnitts 250 bis 420 mm beträgt.

Der Abstand der innersten, schrittzugewandten zweiten Elastifizierungsmittel von der die Beinöffnungen begrenzenden Randkontur des schrittseitigen und den Beinöffnungen zugewandten Bereichs des Bauchabschnitts und des Rückenabschnitts beträgt vorzugsweise 2 - 40 mm, weiter vorzugsweise 3 - 30 mm, insbesondere vorzugsweise 4 - 15 mm.

Als erste und/oder zweite Elastifizierungsmittel werden vorzugsweise faden- oder bandförmige Elastifizierungsmittel, wie Gummi- oder Polyetherpolyurethan- oder Polyesterpolyurethanfäden, vorzugsweise Elastikfäden wie Lycra®-, Creora (R)- oder Spandex®-Fäden eingesetzt.
Die ersten und/oder zweiten Elastifizierungsmittel haben vorzugsweise eine Stärke von 300 - 1500 dtex, insbesondere von 500 - 900 dtex, weiter insbesondere von 500 - 600 dtex.

Die ersten und/oder zweiten Elastifizierungsmittel werden vorzugsweise unter einer Vorspannung von 1,5 - 6,0, insbesondere von 2,5 - 5,0 an den chassisbildenden Hüllmaterialien des Bauchabschnitts und Rückenabschnitts fixiert. Die Vorspannung ist dabei definiert als Faktor des Dehnungsgrades gegenüber dem ungedehnten/relaxierten Zustand des Elastifizierungsmittels.

Dessen ungeachtet erweist es sich als vorteilhaft, wenn der Bauchabschnitt und der Rückenabschnitt zumindest außerhalb des Absorptionskörpers über die Längsrichtung im Wesentlichen durchgehend flächenhaft querelastifiziert sind, wobei auch solchenfalls die vorteilhaften Spannungsverhältnisse einzuhalten bzw. zu realisieren sind.

Die chassisbildenden Materialien von Bauchabschnitt und/oder Rückenabschnitt umfassen vorzugsweise Vliesstoffmaterialien, wie Spinnvliese (S), Meltblownvliese (M), SM-Vliese, SMS-Vliese, SMMS-Vliese, Kardenvliese oder Through Air bonded Kardenvliese. Besonders bevorzugt umfasst das chassisbildende Material von Bauchabschnitt und/oder Rückenabschnitt Spinnvlies. Die für Bauchabschnitt und/oder Rückenabschnitt eingesetzten Vliesstoffmaterialien haben vorteilhafterweise ein Flächengewicht von 10 - 30 g/m$^2$ , weiter vorzugsweise von 15 - 25 g/m$^2$. Besonders bevorzugt umfassen der Bauchabschnitt und der Rückenabschnitt ein Spinnvlies aus Polypropylen, insbesondere mit einem Flächengewicht von 15 - 25 g/m$^2$.

Die chassisbildenden Hüllmaterialien des Schrittabschnitts sind weiter vorteilhaft ausgebildet:

Das Backsheet umfasst insbesondere eine Folie, insbesondere eines Flächengewichts von 10 - 40 g/m$^2$.
Insbesondere umfasst das Backsheet eine im Gebrauch flüssigkeitsdichte aber gleichwohl atmungsaktive, also wasserdampfdurchlässige, insbesondere mikroporöse Folie. Die Wasserdampfdurchlässigkeit des Backsheets beträgt insbesondere wenigstens 300 g/m$^2$/24h , weiter insbesondere wenigstens 1000 g/m$^2$/24h, weiter insbesondere wenigstens 2000 g/m$^2$/24h, weiter insbesondere wenigstens 3000 g/m$^2$/24h, weiter insbesondere wenigstens 4000 g/m$^2$/24h, weiter insbesondere höchstens 6000 g/m$^2$/24h gemessen nach DIN 53 122-1 (Ausgabe: 2001-08).
Die Folie kann vorteilhaft auch mit einer Vliesbeschichtung versehen sein, was eine textile Anmutung der körperabgewandten Außenseite des Inkontinenzartikels vermitteln kann. Die Vliesbeschichtung besteht vorzugsweise aus einem Vliesstoff, insbesondere einem Spinnvlies aus Polypropylen, insbesondere mit einem Flächengewicht von 7-25 g/m$^2$, 10 - 20 g/m$^2$, insbesondere von 12 - 17 g/m$^2$.

Das Deckblatt-Material umfasst vorzugsweise Vliesstoffmaterialien, wie Spinnvliese (S), Meltblownvliese (M), SM-Vliese, SMS-Vliese, SMMS-Vliese, Kardenvliese oder Through Air bonded Kardenvliese.

Das Deckblatt-Material kann dabei vorzugsweise nur aus Topsheet-Material gebildet sein. Weiter vorzugsweise kann das Deckblatt-Material ein Verbund aus Topsheet-Material und Barrieremittel sein.

Entsprechend der Funktionalität werden nachstehend genannte vorteilhafte Materialien eingesetzt.

Das Topsheet-Material umfasst vorzugsweise Vliesstoffmaterialien, wie Spinnvliese,

Kardenvliese oder Through Air bonded Kardenvliese. Besonders bevorzugt umfasst das Topsheet-Material Spinnvlies. Die für das Topsheet eingesetzten Vliesstoffmaterialien haben weiter vorteilhafterweise ein Flächengewicht von 5 - 20 g/m$^2$, 8 - 20 g/m$^2$, weiter vorzugsweise von 10 - 18 g/m$^2$, insbesondere vorzugsweise von 12 - 16 g/m$^2$. Besonders bevorzugt umfasst das Topsheet ein hydrophilisiertes Spinnvlies, insbesondere aus Polypropylen, insbesondere mit einem Flächengewicht von 12 - 16 g/m$^2$.

Das Barrieremittel-Material umfasst vorzugsweise Vliesstoffmaterialien, wie Spinnvliese, Meltblownvliese, Kardenvliese oder Through Air bonded Kardenvliese. Besonders bevorzugt umfasst das Barrieremittel-Material einlagige oder mehrlagige Vliese. Insbesondere bevorzugt umfasst das Barrieremittel-Material Laminate aus einer oder mehreren Spinnvlies(S) und/oder Meltblown(M)-Vlieslagen, insbesondere SMS-Laminate oder SMMS-Laminate, insbesondere auf Basis von Polyolefinen, wie beispielsweise Polyethylen oder Polypropylen. Derartige Materialien sind kostengünstig und aufgrund ihrer inhärent hydrophoben Eigenschaft geeignet, flüssigkeitsretardierend zu wirken. Die für das Barrieremittel eingesetzten Vliesstoffmaterialien haben weiter vorteilhafterweise ein Flächengewicht von 5 - 20 g/m$^2$, vorzugsweise von 8 - 20 g/m$^2$, weiter vorzugsweise von 10 - 18 g/m$^2$. Besonders bevorzugt umfasst das Barrieremittel ein Laminat aus Spinnvlies und Meltblown-Vlieslagen, insbesondere aus Polypropylen, insbesondere mit einem Flächengewicht von 10 - 18 g/m$^2$.

Der Absorptionskörper umfasst Körperflüssigkeiten absorbierende Materialien wie natürliche oder synthetische Fasern, insbesondere Zellulosefasern, vorzugsweise in Form von Zellstofffluff. Vorzugsweise umfasst der Absorptionskern außerdem superabsorbierende Materialien (SAP), insbesondere auf Basis oberflächenvernetzter, teilneutralisierter Polyacrylate.

Der Schrittabschnitt bzw. die Längsränder des Schrittabschnitts, welche die Beinöffnungen begrenzen, sind vorteilhafterweise bogenförmig konturiert ausgebildet.

Das erfindungsgemäße Verfahren:

Ebenso erfindungsgemäß ist das Verfahren gemäß den Merkmalen nach Anspruch 15, zur Herstellung eines Inkontinenzartikel in Höschenform für die Aufnahme von Körperausscheidungen, mit einem vorderen Bauchabschnitt und einem hinteren Rückenabschnitt, die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind, und mit einem einen Absorptionskörper aufweisenden Schrittabschnitt, der sich in einer Längsrichtung zwischen Bauchabschnitt mit einem schrittzugewandten Querrand und Rückenabschnitt mit einem schrittzugewandten Querrand erstreckt und wobei der Schrittabschnitt an den Bauchabschnitt und an den Rückenabschnitt unlösbar angefügt ist, wobei das erfindungsgemäße Verfahren die Bereitstellung des Schrittabschnitts umfasst und die Bereitstellung des Schrittabschnitts die folgenden Verfahrensschritte aufweist:

- Zuführen einer das spätere Deckblatt-Material bildenden endlosen Deckblatt-Materialbahn,
- bereichsweises Aufbringen eines Verstärkungsmittel als verstärkende Beschichtung auf die Deckblatt-Materialbahn, derart, dass das Verstärkungsmittel auf der späteren Innenseite des Deckblatt-Materials angeordnet ist und derart, dass das Verstärkungsmittel in jeweils einem den jeweiligen späteren Längsrand des Absorptionskörpers überbrückenden Bereich vorgesehen ist, also sowohl einen jeweiligen späteren Längsrandbereich des Absorptionskörpers überfängt als auch zumindest in einem jeweiligen angrenzenden Teilbereich des späteren Überhangs vorgesehen ist und derart, dass das Verstärkungsmittel in diesem Teilbereich in Querrichtung betrachtet schmäler ausgebildet ist als der Überhang.
- Zuführen einer das spätere Backsheet-Material bildenden endlosen Backsheet-Materialbahn,
- Zuführen und Anordnen von Absorptionskörpern voneinander beabstandet zwischen der Deckblatt-Materialbahn und der Backsheet-Materialbahn.

Dadurch, dass die Einbringung eines Verstärkungsmittels an den jeweiligen den späteren Längsrand des Absorptionskörpers überbrückenden Bereich realisiert wird, kann die Anbringung des Schrittabschnitts an den Bauchabschnitt und/oder Rückenabschnitt optimiert werden. Auf diese Weise wird die Eigenstabilität des Absorptionskörpers mittels des eingebrachten Verstärkungsmittels auf den angrenzenden Teilbereich des Überhangs aus Materialien mit geringen Flächengewichten übertragen. Das Verstärkungsmittel in dem Teilbereich des Überhangs ermöglicht eine technisch bessere Handhabbarkeit des ansonsten wenig stabilen Überhangs. Dadurch wird ein sauberes und planes Ablegen des Überhangs möglich.

Nach einer bevorzugten Ausführungsform des erfindungsgemäßen Verfahrens wird das Verstärkungsmittel derart aufgebracht wird, dass nur der Überhang im hinteren Überlappungsbereich ein Verstärkungsmittel aufweist.

Nach einer weiteren bevorzugten Ausführungsform des erfindungsgemäßen Verfahrens wird das Verstärkungsmittel derart aufgebracht, dass der Überhang sowohl im hinteren Überlappungsbereich als auch im vorderen Überlappungsbereich ein Verstärkungsmittel aufweist.

In einer weiteren insbesondere bevorzugten Ausführungsform wird das Verstärkungsmittel derart aufgebracht, dass sich das Verstärkungsmittel ausgehend vom vorderen und/oder vom hinteren Überlappungsbereich über den jeweiligen schrittzugewandten Querrand des Baucliabschnittes und/oder Rückenabschnittes in Richtung Quermittelachse erstreckt.

In einer weiteren besonders bevorzugten Ausführungsform wird das Verstärkungsmittel derart aufgebracht, dass das Verstärkungsmittel im vorderen Überlappungsbereich und das Verstärkungsmittel im hinteren Überlappungsbereich sich miteinander verbindend in Richtung Quermittelachse erstrecken.

Nach einer bevorzugten Ausführungsform des erfindungsgemäßen Verfahrens wird das Verstärkungsmittel derart aufgetragen, dass das Verstärkungsmittel den Teilbereich des späteren Überhangs mit der Breite G' bezogen auf den jeweiligen Überhang (66a, 66b) mit der Breite H in Querrichtung in einem Anteil G'/ H von mindestens 0,10, insbesondere mindestens 0,15, weiter insbesondere mindestens 0,20 und höchstens 0,80, insbesondere höchstens 0,75, weiter insbesondere höchstens 0,70 überfängt.

Nach einer weiteren bevorzugten Ausführungsform des erfindungsgemäßen Verfahrens wird das Verstärkungsmittel derart aufgetragen, dass das Verstärkungsmittel den jeweiligen Längsrandbereich mit der Breite G" bezogen auf die Breite H des Überhangs in Querrichtung in einem Anteil G"/ H von mindestens 0,10, insbesondere mindestens 0,15, weiter insbesondere mindestens 0,20 , aber vorzugsweise höchstens 0,80, insbesondere höchstens 0,75, weiter insbesondere höchstens 0,70 überfängt.

Nach einer weiter bevorzugten Ausführungsform des erfindungsgemäßen Verfahrens wird das Verstärkungsmittel derart aufgetragen, dass das Verstärkungsmittel den späteren Längsrandbereich des Absorptionskörpers in Querrichtung in Summe, also beidseits, in einem Anteil von mindestens 5%, insbesondere mindestens 10% , weiter insbesondere mindestens 15% und höchstens 35%, insbesondere höchstens 30%, weiter insbesondere höchstens 25% bezogen auf die Breite (K) des Absorptionskörpers überfängt.

Nach einer weiter bevorzugten Ausführungsform des erfindungsgemäßen Verfahrens wird das Verstärkungsmittel derart aufgetragen, dass das Verstärkungsmittel den späteren jeweiligen Längsrandbereich des Absorptionskörpers mit der Breite G" bezogen auf die Breite (K) des Absorptionskörpers in einem Anteil G"/K von mindestens 0,02, insbesondere mindestens 0,04, weiter insbesondere mindestens 0,06 , aber vorzugsweise höchstens 0,30, insbesondere höchstens 0,25, weiter insbesondere höchstens 0,20 überfängt.

Insbesondere vorteilhaft ist, wenn das Verstärkungsmittel mit einer Gesamtbreite G von 10 - 60 mm, insbesondere von 15 - 55 mm, weiter insbesondere von 20 - 50 mm, weiter insbesondere von 20 - 40 mm aufgetragen wird.

Insbesondere vorteilhaft ist, wenn das Verstärkungsmittel derart aufgetragen wird, so dass der vom Verstärkungsmittel überfangene Teilbereich in Querrichtung des jeweiligen Überhangs eine Breite G' von größer 5 mm, vorzugsweise größer 10 mm, aber vorzugsweise kleiner 50 mm, weiter vorzugsweise kleiner 40 mm, weiter vorzugsweise kleiner 30 mm aufweist.

Insbesondere vorteilhaft ist, wenn das Verstärkungsmittel derart aufgetragen wird, so dass der vom Verstärkungsmittel überfangene Längsrandbereich des Absorptionskörpers in Querrichtung eine Breite G" von größer 5 mm, vorzugsweise größer 10 mm, aber vorzugsweise kleiner 50 mm, weiter vorzugsweise kleiner 40 mm, weiter vorzugsweise kleiner 30 mm aufweist.

Nach einer weiteren vorteilhaften Ausformungsform wird das Verstärkungsmittel parallel zur späteren Längsrichtung und streifenförmig mit einer gleich bleibenden Breite aufgetragen.

Auf diese Weise kann das Verstärkungsmittel mit geringem technischem Aufwand direkt in Maschinenrichtung und damit auch schneller und kostengünstiger in den Inkontinenzartikel eingebracht werden.

Weiter vorteilhaft wird das Verstärkungsmittel als verstärkende Beschichtung in einem Flächengewicht von 1-30 g/m$^2$, insbesondere von 2 - 20g/m$^2$, weiter insbesondere 2 - 10 g/m$^2$ aufgetragen. Durch die Beschichtung mit diesen Flächengewichten werden die flexiblen Eigenschaften des Deckblatt-Materials auf Vliesbasis mit geringen Flächengewichten von 5 - 20 g/m$^2$ beibehalten. Verfahrenstechnisch würden zudem zu hohe Flächengewichte der Beschichtungen nachteiligerweise dazu führen, dass das Beschichtungsmittel durch das an sich dünne Deckblatt-Material auf die andere Oberseite des Deckblatt-Materials durchdringt, was zu nachteiligen Verschmutzungen der Maschinen durch unkontrollierte Beschichtungseinträge beispielsweise auf Walzen und Rollen führen kann, was sich besonders nachteilig bei Beschichtungsmittel in Form von Kleber auswirken kann.

In einer weiteren vorteilhaften Ausführungsform wird die verstärkende Beschichtung in Form eines Klebers, insbesondere eines Hotmeltklebers, weiter insbesondere eines hydrophoben Hotmeltklebers aufgetragen.

In einer besonders vorteilhaften Ausführungsform wird die verstärkende Beschichtung als vollflächiger Streifen, insbesondere in einem kontaktlosen Verfahren aufgebracht. Kontaktlose Beschichtungsverfahren sind bekannt. Die Technik ist den Dokumenten EP 0 901 781 B1 und EP 1 459 719 A2 beschrieben.

In einer weiteren bevorzugten Ausführungsform des erfindungsgemäßen Verfahren sind im Bereich des Überhangs zwischen Deckblatt-Material und Backsheet-Material und/oder zwischen Deckblatt-Material und Absorptionskörper und/oder zwischen Backsheet-Material und Absorptionskörper separate Fügemittel, vorzugsweise nicht vollflächig, vorgesehen. Weiter vorzugsweise ist das Verfahren derart, dass Backsheet-Material und Deckblatt-Material im Bereich des Überhangs direkt durch separate Fügemittel wie Kleber, Kalanderschweißen oder Ultraschallschweißen, insbesondere bis hin zum Längsrand des Schrittabschnitts, verbunden werden. Weiter vorzugsweise ist das Verfahren derart, dass auch zwischen Backsheet-Material und Absorptionskörper und/oder zwischen Deckblatt-Material und Absorptionskörper vorzugsweise derartige separate Fügemittel vorgesehen sind. Diese Fügemittel sind vorzugsweise nicht vollflächig, sondern in Form eines unterbrochenen Musters vorgesehen. Der Einsatz von Kleber ist vorteilhafterweise auch geeignet, die haftvermittelnde Wirkung der Fügemittel, welche Backsheet-Material und/oder Deckblatt-Material und/oder Absorptionskörper miteinander verbinden, zu stärken. Soweit als für derartige separate Fügemittel ein Kleber vorgesehen ist, kann dieser beispielsweise streifenförmig, rasterförmig oder als Spiralmuster angeordnet sein.

Weiter vorteilhafterweise wird die Deckblatt-Materialbahn als ein Verbund aus einer flüssigkeitsdurchlässigen, das spätere Topsheet-Material bildenden Topsheet-Materialbahn mit Längsrändern und angrenzenden Längsrandbereichen und aus beidseits an den Längsrändern bzw. Längsrandbereichen der Topsheet-Materialbahn in Fügestellen angefügten hydrophoben, die späteren Barrieremittel bildenden Barrieremittel-Materialbahnen in den Verfahrensschritt eingebracht.

Ganz besonders vorteilhaft hierbei ist, wenn im Fall dieses Verbundes das Verstärkungsmittel derart aufgebracht wird, dass das Verstärkungsmittel die Fügestellen der Deckblatt-Materialbahn überfängt.

Es wäre grundsätzlich denkbar, dass die Schrittabschnitte des Inkontinenzartikels, so wie voranstehend beschrieben, vorgefertigt werden und dann endlos, also wie von einer Rolle, dem weiteren Verfahren zugeführt werden. Es ist aber auch vorteilhaft, wenn die Schrittabschnitte innerhalb eines kontinuierlichen Verfahrens gebildet werden.

Das erfindungsgemäße Verfahren zur Herstellung des Inkontinenzartikels in Höschenform umfasst vorteilhafterweise die weiteren Verfahrensschritten:

- Zuführen zweier den späteren Bauchabschnitt bzw. den späteren Rückenabschnitt des Inkontinenzartikels bildenden Teilbahnen auf Vliesbasis,
- Zuführen und Aufbringen der zweiten Elastifizierungsmittel auf die beiden Teilbahnen und Fixieren daran,
- Zusammenführen der Schrittabschnitte mit den beiden Teilbahnen derart, dass die Schrittabschnitte in einer Längsrichtung quer zur Maschinenrichtung einenends mit der einen Teilbahn und anderenends mit der anderen Teilbahn im vorderen und/oder hinteren

Überlappungsbereich plan angeordnet sind und die Schrittabschnitte mit einem Abstand in der Maschinenrichtung voneinander angeordnet sind, und Fixieren der Schrittabschnitte und Teilbahnen in den Überlappungsbereichen und Weiterfördern in der Maschinenrichtung,

- Zuführen, Aufbringen und Fixieren der ersten Elastifizierungsmittel in der Maschinenrichtung an den Teilbahnen
- Falten um eine in Maschinenrichtung verlaufende Faltlinie, derart, dass die eine Teilbahn über die andere Teilbahn zu liegen kommt,
- Fügen der übereinander liegenden Teilbahnen quer zur Maschinenrichtung in Abständen voneinander zur Ausbildung von Seitennahtbereichen der herzustellenden Inkontinenzartikel, und Erhalt von einen Bauchabschnitt, einen Rückenabschnitt und einen dazwischen angeordneten Schrittabschnitt aufweisenden Produkten,
- Ausführen eines Trennschnitts quer zur Maschinenrichtung und Erhalt von vereinzelten fertigen Inkontinenzartikeln .

In einer weiteren bevorzugten Ausführungsform des erfindungsgemäßen Verfahrens werden die zweiten Elastifizierungsmittel derart zugeführt, dass sie sich ausgehend von den beiden Seitennahtbereichen (14) in Richtung (56) auf eine Längsmittelachse (44) des Inkontinenzartikels (2) erstrecken und dabei bogenförmig sich auffächernd mit zunehmendem Abstand voneinander verlaufen.

Weiter vorteilhafterweise werden zwischen die Hüllmaterialien des Schrittabschnitts, also zwischen Deckblatt-Materialbahn und Backsheet-Materialbahn, Beinelastifizierungsmittel zugeführt.

Weiter vorteilhaft werden die Beinelastifizierungsmittel derart zugeführt, dass sie der Kontur des Absorptionskörpers folgen.

Weiter werden in vorteilhafter Weise die Beinelastifizierungsmittel derart zugeführt, dass sie mit veränderlichem Abstand zum Absorptionskörper verlaufen und an ihren Längsenden einen größeren Abstand zum Absorptionskörper aufweisen als mittig.

Weiter vorteilhafterweise werden die Beinelastifizierungsmittel bogenförmig zugeführt.

In besonders vorteilhafter Weise werden die Beinelastifizierungsmittel derart zugeführt, dass die Beinelastifizierungsmittel im Wesentlichen den bogenförmigen Beinausschnitten des späteren Schrittabschnitts folgen.

In einer weiteren vorteilhaften Ausführungsform weisen die Schrittabschnitte zur Bildung von im Wesentlichen bogenförmigen Beinausschnitten durch einen ersten Konturschnitt gebildete Beinausschnitte auf.

In einer bevorzugten Ausführungsform des Verfahrens wird der erste Konturschnitt derart ausgeführt, dass im Wesentlichen bogenförmige Beinausschnitte der späteren Schrittabschnitte des Inkontinenzartikels gebildet werden.

In einer besonders bevorzugten Ausführungsform wird der erste Konturschnitt mittels eines Schneidwalzenpaars, also eines Rotationsmessers mit einer Ambosswalze, durchgeführt.

Das erfindungsgemäße Verfahren mit der Einbringung von Verstärkungsmitteln erweist sich gerade bei der Herstellung solcher Inkontinenzartikel als besonders vorteilhaft, bei denen der große Überhang an chassisbildenden Hüllmaterialen des Schrittabschnitts mit geringen Flächengewichten einem ersten Konturschnitt unterzogen wird.

Des Weiteren ist die Einbringung von Verstärkungsmitteln bei Schrittabschnitten vorteilhaft, welche den Beinöffnungen zugeordnete Beinelastifizierungsmittel aufweisen. Wenn nämlich solchenfalls die jeweiligen Schrittabschnitte mit Beinelastifizierungsmitteln versehen und dann vereinzelt werden, so üben die Beinelastifizierungsmittel hohe Zugkräfte auf die Materialien des jeweiligen Schrittabschnitts aus und versuchen, den Schrittabschnitt zusammenzuziehen. Dies ist herstellungstechnisch schwer beherrschbar, und es gestaltet sich umso schwieriger, bei vereinzelten Schrittabschnitten dann die Beinöffnungen zu konturieren. Es wird aber vorzugsweise derjenige Teil der Beinöffnungen, der von dem Schrittabschnitt begrenzt wird, schon im Zuge der Ausführung des ersten Konturschnitts gefertigt, und zwar zu einem Zeitpunkt, zu dem die Schrittabschnitte noch nicht vereinzelt, also noch endlos gefördert werden. Bei dieser endlosen Förderung können die vorstehend erläuterten Kräfte, die durch die Beinelastifizierungsmittel hervorgerufen werden, besser beherrscht werden, so dass es im Ergebnis möglich ist, mit einem geringeren technischen Aufwand ein besseres Ergebnis hinsichtlich der Genauigkeit bei der Konturierung der Beinöffnungen zu erzielen.

Es ist grundsätzlich denkbar, dass die Komponenten Deckblatt-Materialbahn, Backsheet-Materialbahn und Absorptionskörper in der späteren Längsrichtung des Schrittabschnitts bzw. des Hygieneartikels gefördert werden. Ebenso denkbar ist eine Förderung in Querrichtung. Im ersten Fall ist dann eine 90°-Umlenkung im Zuge des endlosen Fertigungsverfahrens erforderlich, da die weitere Fertigung vorzugsweise in der Querrichtung der Inkontinenzartikel erfolgt. In jedem Fall müssen die Schrittabschnitte quer zur Maschinenrichtung getrennt, also vereinzelt werden, und dann im Abstand voneinander weiter gefördert werden, um mit den endlosen, die den späteren Bauchabschnitt und den späteren Rückabschnitt bildenen Teilbahnen verbunden zu werden.

Die Backsheet-Materialbahn wird vorzugsweise in Form eines flüssigkeitsundurchlässigen Materials zugeführt, wie voranstehend bereits beschrieben ist.

Der Absorptionskörper umfasst vorzugsweise die Körperflüssigkeiten absorbierende Materialien, wie sie bereits voranstehend beschrieben sind.

Die Beinelastifizierungsmittel werden, wie voranstehend beschrieben, in den bevorzugten Materialien und mit den bevorzugten Vorspannungen eingesetzt.

Zum Zuführen der beiden den späteren Bauchabschnitt und späteren Rückenabschnitt des Inkontinenzartikels bildenden Teilbahnen ist es beispielsweise denkbar, dass jede Teilbahn von einer eigenen Rolle abgerollt und dem Herstellverfahren zugeführt wird. Gemäß einer bevorzugten Verfahrensvariante kann aber auch zunächst eine endlose Vliesbahn zugeführt werden, die dann zur Bildung der zwei Teilbahnen entlang der Maschinenrichtung aufgetrennt wird. Auf diese Weise braucht die Maschine nur mit einer Rolle bestückt zu werden.

Die Teilbahnen auf Vliesbasis des späteren Bauchabschnitts und/oder des späteren Rückenabschnitts sind vorzugsweise aus den Materialien und mit den Flächengewichten, so wie voranstehend beschrieben.

Vorzugweise werden die ersten und/oder zweiten Elastifizierungsmittel endlos in Maschinenrichtung zu den Teilbahnen zugeführt. Besonders bevorzugt werden die ersten und die zweiten Elastifierungsmittel endlos in Maschenrichtung an die Teilbahnen zugeführt.

Die erwähnten in Hüftumfangsrichtung verlaufenden ersten Elastifizierungsmittel werden vorzugsweise in einem Abstand von 4 bis 10 mm, insbesondere 4 bis 8 mm, insbesondere 4 bis 6 mm voneinander eingebracht.

Die zweiten Elastifizierungsmittel, die sich in Richtung auf die Längsmittelachse des Inkontinenzartikels bogenförmig auffächern, werden entsprechend in einem variierenden Abstand voneinander (Abstand von unmittelbar nebeneinanderliegenden Elastifizierungsmitteln) zwischen 3 mm und 35 mm eingebracht. Zum Einbringen der Elastifizierungsmittel werden vorzugsweise oszillierend antreibbare Führungsorgane für die Elastifizierungsmittel verwendet.

Vorzugsweise werden die zweiten Elastifizierungsmittel derart eingebracht, dass ein minimaler Abstand der zweiten Elastifizierungsmittel voneinander (also der Abstand von unmittelbar nebeneinander liegenden zweiten Elastifizierungsmitteln) in den späteren Seitennahtbereichen 3 bis 8 mm, insbesondere 3 bis 7 mm und weiter insbesondere 3 bis 6 mm beträgt.

Des Weiteren vorzugsweise werden die zweiten Elastifizierungsmittel derart eingebracht, dass ein maximaler Abstand der zweiten Elastifizierungsmittel voneinander (also der Abstand von unmittelbar nebeneinander liegenden Elastifizierungsmitteln) an einem späteren Absorptionskörperrand oder an einem späteren Längsrand des Schrittabschnitts 7 bis 35 mm, insbesondere 10 bis 32 mm und weiter insbesondere 12 bis 30 mm beträgt.

Vorzugsweise werden die zweiten Elastifizierungsmittel derart eingebracht, dass sich die Abstände der zweiten Elastifizierungsmittel im späteren Rückenabschnitt von den Abständen der zweiten Elastifizierungsmittel im späteren Bauchabschnitt unterscheiden. Vorzugsweise ist das Einbringen der zweiten Elastifizierungsmittel derart, dass der maximale Abstand der zweiten Elastifizierungsmittel voneinander im späteren Rückenabschnitt größer ist als der maximale Abstand der zweiten Elastifizierungsmittel im späteren Bauchabschnitt.

Des Weiteren vorteilhaft werden die zweiten Elastifizierungsmittel derart eingebracht, dass sie einen Auffächerungsgrad F

$$F=(A-B)/B * 100\%$$

wie vorstehend beschrieben, haben.

Der Verlauf der zweiten Elastifizierungsmittel und die Ausführung des zweiten Konturschnitts bei den Teilbahnen ist vorteilhafterweise derart, dass der zweite Konturschnitt entlang und im Abstand (D) zu dem in Längsrichtung jeweils innersten der schrittzugewandten zweiten Elastifizierungsmittel ausgeführt wird. Dieser Abstand (D) beträgt vorteilhafterweise 2 bis 40 mm, insbesondere 3 bis 30 mm und weiter insbesondere 4 bis 20 mm.

Vorteilhafterweise werden die ersten und/oder zweiten Elastifizierungsmittel einer Vorspannung, wie bereits voranstehend beschrieben, an den Teilbahnen fixiert. Die Vorspannung ist dabei definiert als Faktor des Dehnungsgrads gegenüber dem ungedehnten/relaxierten Zustand des Elastifizierungsmittels.

Als erste und/oder zweite Elastifizierungsmittel werden vorteilhafte Materialien und Stärken, wie voranstehend beschrieben, eingesetzt.

Das Fixieren der ersten und/oder zweiten Elastifizierungsmittel auf den Teilbahnen erfolgt vorzugsweise mittels Klebemittel. Die Klebemittel für das Fixieren der ersten und/oder zweiten Elastifizierungsmittel können dabei vorzugsweise direkt auf die Teilbahnen und/oder Vliesabdeckschichten aufgebracht oder direkt auf die Elastifizierungsmittel aufgebracht werden (Fadenbeleimung). Vorzugsweise werden die ersten Elastifizierungsmittel direkt mit Kleber versehen, insbesondere besprüht, und die zweiten Elastifizierungsmittel werden in zuvor flächenhaft auf die Teilbahnen und/oder Vliesabdeckschichten aufgebrachten Kleber gelegt.

In einer weiteren vorteilhaften Ausführungsform wird vor dem Falten um eine in Maschinenrichtung verlaufende Faltlinie, ein die Teilbahnen an ihren aneinander zugewandten Randabschnitten erfassender zweiter Konturschnitt zur Bildung von im Wesentlichen bogenförmigen Beinausschnitten ausgeführt.

Nach einer weiteren bevorzugten Ausführungsform des erfindungsgemäßen Verfahrens erfasst der zweite Konturschnitt nur die Teilbahnen, nicht aber den Schrittabschnitt. Es ist daher möglich, dass entlang der Beinöffnungen ein unstetiger Verlauf im Übergang vom Schrittabschnitt zum Bauchabschnitt bzw. Rückenabschnitt gebildet wird.

Nach einer bevorzugten Ausführungsform des erfindungsgemäßen Verfahrens ist die Ausführung des zweiten Konturschnitts bei den Teilbahnen derart, dass der bogenförmige Beinausschnitt der Teilbahn des späteren Bauchabschnittes einen anderen Verlauf, insbesondere einen anderen Winkel oder Radius, aufweist als der bogenförmige Beinausschnitt der Teilbahn des späteren Rückenabschnitts.

Nach einer bevorzugten Ausführungsform wird der zweite Konturschnitt zur Bildung der im Wesentlichen bogenförmigen Beinausschnitte derart ausgeführt, dass der zweite Konturschnitt bei der Teilbahn des späteren Bauchabschnitts und bei der Teilbahn des späteren Rückenabschnitts gleichzeitig ausgeführt wird.

In einer besonders bevorzugten Ausführungsform wird der zweite Konturschnitt mittels eines Schneidwalzenpaares, also eines Rotationsmessers mit einer Ambosswalze, durchgeführt.

Des Weiteren kann es sich als vorteilhaft erweisen, wenn das Fügen der übereinander liegenden Teilbahnen zur Ausbildung von Seitennahtbereichen der herzustellenden Inkontinenzartikel und das Ausführen des Trennschnitts in demselben Verfahrensschritt durchgeführt werden.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Inkontinenzartikels. In der Zeichnung zeigt:

Figur 1      eine Draufsicht auf einen erfindungsgemäßen Inkontinenzartikel, wobei ein Bauchabschnitt, ein Rückenabschnitt und ein die beiden verbindender Schrittabschnitt des Inkontinenzartikels noch nicht zur Bildung einer Höschenform verbunden, sondern in flachgelegtem und ausgedehntem Zustand dargestellt sind;

Figuren 2a, 2b, 2c      eine Draufsicht auf vorteilhafte Ausführungsformen des Schrittabschnitts des Inkontinenzartikels nach Figur 1 wiederum im flachgelegten und ausgedehnten Zustand;

Figur 3      eine Schnittansicht (schematisch) entlang einer Quermittelachse des Schrittabschnitts mit Schnittebene III-III in Figur 2b;

| Figur 4 | eine Schnittansicht (schematisch) des Schrittabschnitts mit Schnittebene IV-IV in Figur 2b; |
| Figur 5 | eine Schnittansicht (schematisch) des Schrittabschnitts mit Schnittebene V-V in Figur 2b; |
| Figur 6 | eine perspektivische Ansicht (schematisch) des an einen Benutzer angelegten Inkontinenzartikels nach Figur 1; |
| Figur 7 | eine ausschnittsweise Darstellung des Inkontinenzartikels nach Figur 1; |
| Figuren 8,9 | verdeutlichen beispielhaft die Bestimmung von Rückstellkräften im Bauchabschnitt bzw. Rückenabschnitt des Inkontinenzartikels; |
| Figur 10 | eine Figur 1 entsprechende Draufsicht des erfindungsgemäßen Inkontinenzartikels zur Verdeutlichung der Verbindung von Schrittabschnitt und Bauchabschnitt bzw. Rückenabschnitt. |
| Figur 11 | eine schematische Darstellung des erfindungsgemäßen Verfahrens zur Herstellung des Schrittabschnitts; |
| Figur 12 | eine schematische Darstellung der Zuführung und Anfügung von Teilabschnitten an den Schrittabschnitt und die Ausführung eines zweiten Konturschnitts; |
| Figur 13 | eine schematische Darstellung des Faltens auf Höschenform und des Ausbildens von Seitennahtbereichen und anschließender Vereinzelung der Hygieneartikel und |
| Figur 14 | eine schematische Darstellung einer bereichsweisen Beschichtung eines Backsheetmaterials bei der Herstellung des Schrittabschnitts des Hygieneartikels. |

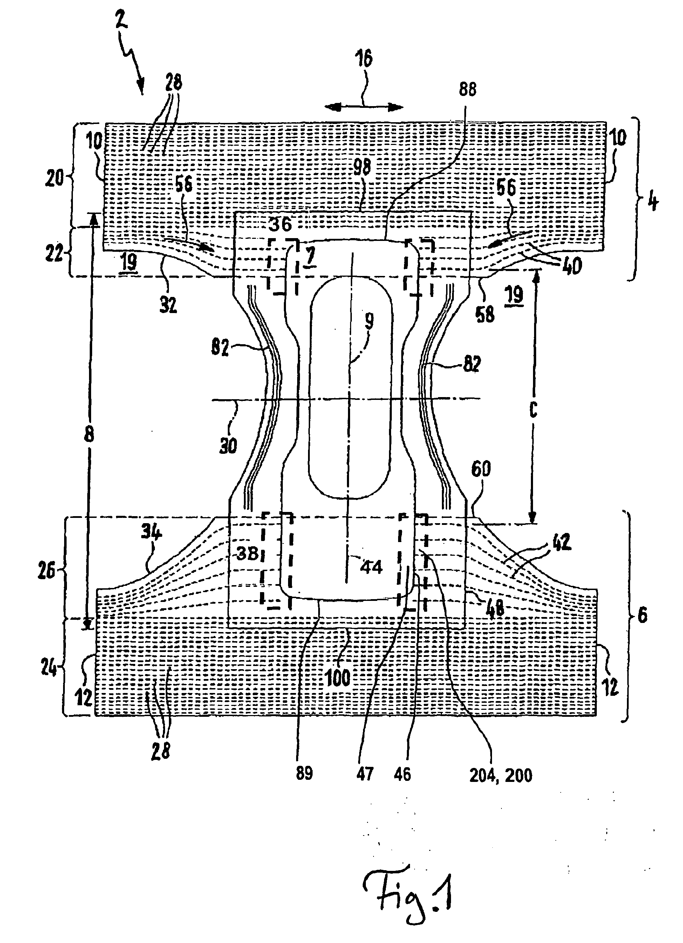

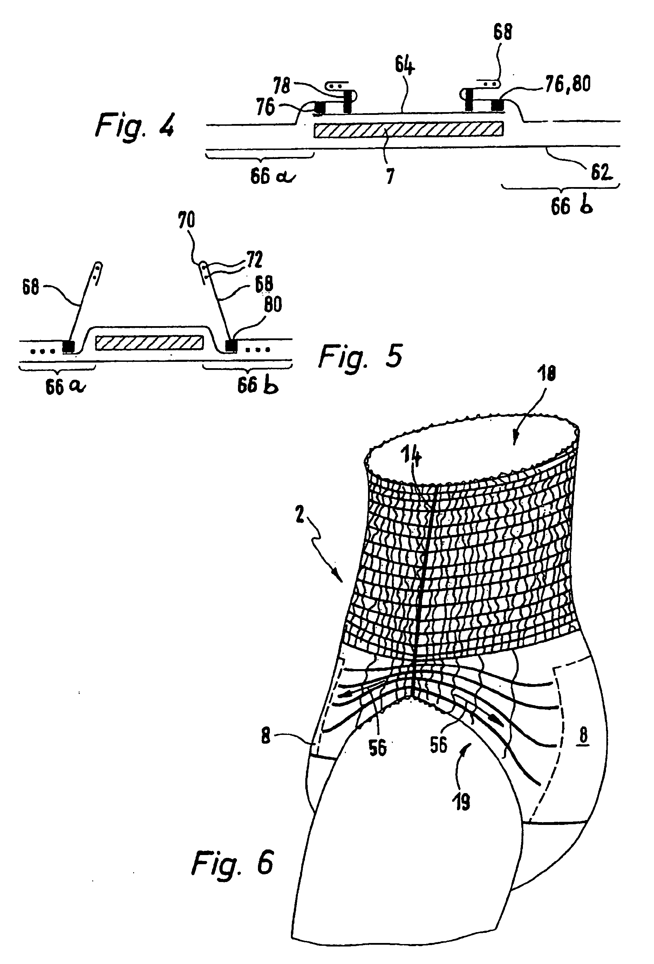

Die Figuren zeigen einen insgesamt mit dem Bezugszeichen 2 bezeichneten Inkontinenzartikel in Höschenform für die Aufnahme fester und flüssiger Körperausscheidungen. Der Inkontinenzartikel 2 ist aus drei weitestgehend unabhängig voneinander fertigbaren Komponenten, nämlich einem vorderen Bauchabschnitt 4, einem hinteren Rückenabschnitt 6 und einem zwischen diesen angeordneten und einen Absorptionskörper 7 aufweisenden Schrittabschnitt 8 gebildet, wobei der Schrittabschnitt 8 mit einem wesentlichen Flächenanteil mit dem Bauchabschnitt 4 einerseits und dem Rückenabschnitt 6 andererseits überlappt und im Überlappungsbereich herstellerseitig unlösbar verbunden ist. Wie aus Figur 1 ersichtlich, führt dies zu einer H-förmigen Grundstruktur des Inkontinenzartikels mit einer Längsrichtung 9. Die in Figur 1 dargestellten miteinander gefügten Bestandteile werden dann zur Bildung der in Figur 6 schematisch dargestellten Höschenform an jeweiligen seitlichen Längsrandabschnitten 10, 12 des Bauchabschnitts 4 und des Rückenabschnitts 6 ebenfalls herstellerseitig miteinander verbunden, wodurch beidseits Seitennahtbereiche 14 (Figur 6) ausgebildet werden. In diesem herstellerseitig gefertigten höschenförmigen Zustand des Inkontinenzartikels erstrecken sich der Bauchabschnitt 4 und der Rückenabschnitt 6 in Quer- oder Hüftumfangsrichtung 16 durchgehend bis zu den Seitennahtbereichen 14 und definieren so eine in Hüftumfangsrichtung geschlossene Hüftöffnung 18 und Beinöffnungen 19, durch welche hindurch der Benutzer den Inkontinenzartikel wie ein Höschen anlegt.

Der Bauchabschnitt 4 lässt sich in einen hüftseitigen Bereich 20 und in einen schrittseitigen und den Beinöffnungen zugewandten Bereich 22 unterteilen. Eine entsprechende Unterteilung ist im Rückenabschnitt 6 vorgesehen, und zwar ebenfalls in einen hüftseitigen Bereich 24 und einen schrittseitigen und den Beinöffnungen zugewandten Bereich 26.

In dem hüftseitigen Bereich 20 des Bauchabschnitts 4 und in dem hüftseitigen Bereich 24 des Rückenabschnitts 6 sind erste Elastifizierungsmittel 28 vorgesehen, bei denen es sich insbesondere um fadenförmige Elastifizierungsmittel, wie Lycra®-Fäden, handeln kann, die in vorgedehntem Zustand, im sogenannten Stretch-Bond-Verfahren, mit den Flachmaterialien (Chassismaterialien) des Bauchabschnitts 4 und des Rückenabschnitts 6 verbunden sind. Diese ersten Elastifizierungsmittel 28 erstrecken sich in Quer- oder Hüftumfangsrichtung 16 von einem Seitennahtbereich 14 zum anderen:

Der schrittseitige und den Beinöffnungen 19 zugewandte Abschnitt 22 des Bauchabschnitts 4 bzw. 26 des Rückenabschnitts 6 haben eine von der Quer- oder Hüftumfangsrichtung 16 abweichende in Richtung auf eine Quermittelachse 30 des Schrittabschnitts 8 zulaufende Randkontur 32 bzw. 34. Diese Randkontur 32, 34 ist auch in der Darstellung nach Figur 1 bogenförmig und daher zur Begrenzung der Beinöffnungen 19 geeignet. Durch diesen Verlauf des schrittseitigen und den Beinöffnungen zugewandten Bereichs 22 bzw. 26 wird auch ein verhältnismäßig großer Überlappungsbereich 36, 38 zwischen Schrittabschnitt 8 und Bauchabschnitt 4 bzw. Rückenabschnitt 6 realisiert, der im Hinblick auf eine reißfeste Verbindung von Schrittabschnitt 8 und Bauchabschnitt 4 bzw. Rückenabschnitt 6 wesentlich ist. Je größer der Überlappungsbereich 36, 38, umso geringere Klebermengen bezogen auf die Fläche des Überlappungsbereiches können verwendet werden, was sich im Hinblick auf die Steifigkeit der Chassismaterialien als vorteilhaft auswirkt. Insbesondere kann ein nicht vollflächiger Kleberauftrag zur Verbindung der Komponenten verwendet werden.

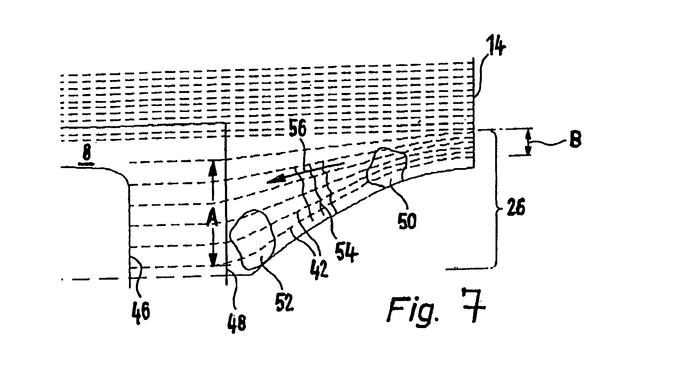

Der jeweilige schrittseitige und den Beinöffnungen 19 zugewandte Bereich 22, 26 des Bauchabschnitts 4 bzw. des Rückenabschnitts 6 ist ebenfalls elastifiziert ausgebildet. Dort sind zweite Elastifizierungsmittel 40 bzw. 42 vorgesehen. Die zweiten Elastifizierungsmittel 40, 42 erstrecken sich jeweils ausgehend von den Seitennahtbereichen 14 in Richtung auf eine Längsmittelachse 44 des Inkontinenzartikels. Wie aus den Figuren 1 und 7 zu erkennen ist, fächern die zweiten Elastifizierungsmittel 40, 42 in Richtung auf die Längsmittelachse 44 auf. Dies bedeutet, dass der Abstand zwischen ihnen in Richtung auf die Längsmittelachse 44 zunimmt. Diese Auffächerung der zweiten Elastifizierungsmittel 40 bzw. 42 lässt sich anhand Figur 7 auch quantitativ näher bezeichnen. Beispielsweise haben die in Figur 7 dargestellten zweiten Elastifizierungsmittel 42 des Rückenabschnitts 6 in den Seitennahtbereichen 14 einen minimalen Abstand von 3 bis 8 mm voneinander (Abstand von unmittelbar nebeneinander liegenden Elastifizierungsmitteln) und an einem Absorptionskörperrand 46 oder einem Längsrand 48 des Schrittabschnitts 8 einen maximalen Abstand (Abstand von unmittelbar nebeneinander liegenden Elastifizierungsmitteln) von 7 bis 35 mm voneinander. Es lässt sich anhand der Figur 7 auch ein Auffächerungsgrad F wie folgt definieren:

$$F = (A-B)/B * 100\%.$$

Dieser Auffächerungsgrad kann vorteilhafterweise zwischen 50 und 900 %, insbesondere zwischen 100 und 700 % und weiter insbesondere zwischen 150 bis 550 % liegen. Er ist im Rückenabschnitt 6 vorteilhafterweise größer als im Bauchabschnitt 4. Dabei sind die Größen A und B definiert als der Abstand des in Längsrichtung 9 äußersten zweiten Elastifizierungsmittels 40, 42 von dem in Längsrichtung 9 innersten zweiten Elastifizierungsmittel 40, 42 (also nicht der Abstand von unmittelbar nebeneinander liegenden Elastifizierungsmitteln), und zwar A als der maximale Abstand, insbesondere am Längsrand 48 des Schrittabschnitts 8, und B als der minimale Abstand, insbesondere im Seitennahtbereich 14 (vgl. Figur 7).

Sofern der Auffächerungsgrad bei den zweiten Elastifizierungsmitteln 40, 42 hinreichend groß gewählt wird, so lässt sich auf diese Weise eine abnehmende Rückstellkraft innerhalb des schrittseitigen und den Beinöffnungen 19 zugewandten Bereichs 22 beziehungsweise 26 in Richtung 56 auf den Schrittabschnitt 8 realisieren, sofern dafür Sorge getragen wird, dass in Folge des von der Hüft- oder Querrichtung 16 abgewandten bogenförmigen Verlaufs der zweiten Elastifizierungsmittel 40, 42 sich keine zu starke Erhöhung der Vorspannung in Folge des größeren Wegs dieser zweiten Elastifizierungsmittel 40, 42 ergibt. Betrachtet man dann ein näher am Seitennahtbereich 14 liegendes Gebiet 50 des betreffenden schrittseitigen Bereichs 22 beziehungsweise 26 mit einem näher am Schrittabschnitt 8 liegenden Gebiet 52, so ist die Rückstellkraft, die sich bei flächenhafter Dehnung des Gebiets 52 (Dehnung in Richtung der Elastifizierungsmittel 42) einstellt geringer als diejenige Rückstellkraft, die sich bei Dehnung des Gebiets 50 einstellt. Dies führt in vorteilhafter Weise weiter dazu, dass in Folge der geringeren elastischen Kräfte, welche im beispielhaft dargestellten Fall durch die zweiten Elastifizierungsmittel 40, 42 ausgeübt werden, die Chassismaterialien des Bauchabschnitts 4 und des Rückenabschnitts 6 weniger stark gerafft werden, so dass sich auch eine geringere Anzahl von Falten/Rüschen 54 ergibt, und zwar ausgehend vom jeweiligen Seitennahtbereich 14 in Richtung auf den Schrittabschnitt 8. Dadurch, dass die sich bei flächenhafter Dehnung des Bauchabschnitts in dem schrittseitigen und den Beinöffnungen zugewandten Bereich 22 des Bauchabschnitts 4 beziehungsweise 26 des Rückenabschnitts 6 sich einstellenden Rückstellkräfte in Richtung des Pfeils 56, also generell vom Seitennahtbereich 14 in Richtung auf den Schrittabschnitt 8 abnehmen, wird eine erhebliche Verbesserung des Tragekomforts erzielt, weil gerade in diesen Bereichen - wie festgestellt wurde - sich elastisch dehnbare Materialien als besonders problematisch erweisen, weil diese Materialien der Physiognomie der menschlichen Körperform entsprechend in diesen Bereichen besonders auf Zug und Dehnung beansprucht werden. Durch eine bewusst vorteilhaft vorgesehene Verringerung dieser Rückstellkraft, also abnehmende Rückstellkraft in Richtung des Pfeils 56, das heißt in Richtung zunehmender Annäherung an den Schrittabschnitt 8, wird hier ein zuvor nicht realisierter Freiheitsgrad geschaffen.

Bei der dargestellten bevorzugten Ausführungsform des Inkontinenzartikels 2 beträgt ein Abstand C des schrittzugewandten innersten zweiten Elastifizierungsmittels 40 des Bauchabschnitts 4 von dem entsprechenden schrittzugewandten innersten zweiten Elastifizierungsmittel 42 des Rückenabschnitts 6 zwischen 250 bis 420 mm je nach Konfektionsgröße des Inkontinenzartikels. Die zweiten Elastifizierungsmittel 40, 42 erstrecken sich im Wesentlichen bis zum schrittzugewandten Querrand 58, 60 des Bauchabschnitts 4 und des Rückenabschnitts 6. Der Abstand von Bauchabschnitt 4 und Rückenabschnitt 6 voneinander beträgt 250 - 400 mm.

Der Abstand der innersten, schrittzugewandten zweiten Elastifizierungsmittel 40, 42 von der die Beinöffnungen begrenzenden Randkontur 32, 34 des schrittseitigen und den Beinöffnungen zugewandten Bereichs 22, 26 des Bauchabschnitts 4 und des Rückenabschnitts 6 beträgt vorzugsweise 2 - 40 mm, weiter vorzugsweise 3 - 30 mm, insbesondere vorzugsweise 4 -15 mm.

Die Erstreckung des Bauchabschnitts 4 und des Rückenabschnitts 6 im Seitennahtbereich 14 in Längsrichtung 9 beträgt vorteilhafterweise zwischen 100 und 220 mm. Die Erstreckung des Schrittabschnitts 8 in Querrichtung 16 beträgt vorteilhafterweise 200 bis 350 mm.

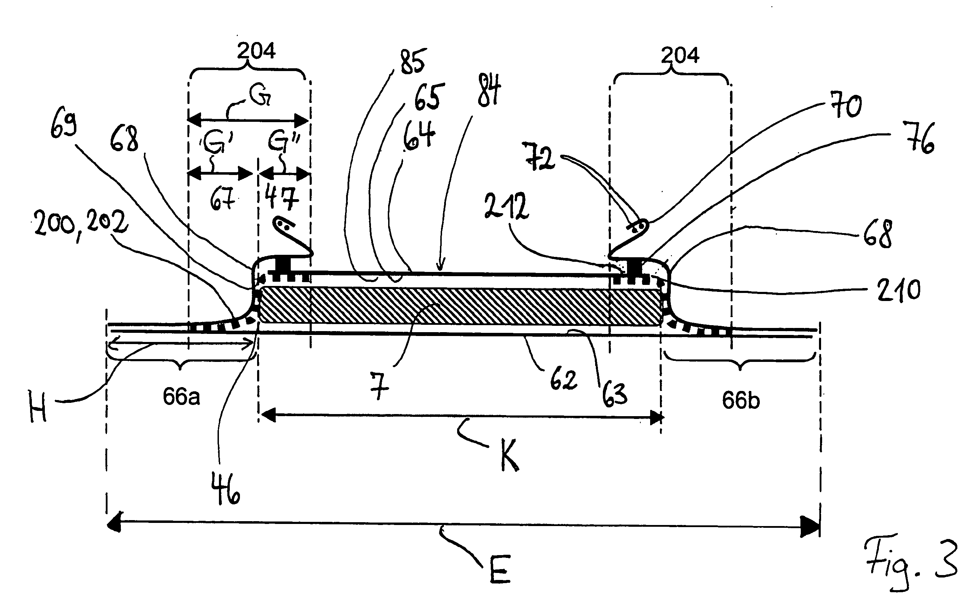

Der Schrittabschnitt 8 umfasst ein flüssigkeitsundurchlässiges Backsheet-Material 62, das insbesondere von einem atmungsaktiven, jedoch flüssigkeitsdichten Folienmaterial gebildet sein kann, und ein, vorzugsweise auf Vliesbasis hergestelltes Deckblatt-Material 84, welches ein Verbund aus einem auf Vliesbasis hergestelltem Topsheet-Material 64 und aus beidseits angeordneten Barrieremitteln 68 ist. Zwischen, dem Backsheet-Material und dem Topsheet-Material ist, wie aus den Figuren 3, 4, 5 ersichtlich, der Absorptionskörper 7 angeordnet. Der Absorptionskörper 7 weist Längsränder 46 und daran angrenzende Längsrandbereiche 47 auf. Im beispielhaft dargestellten Fall bildet das Backsheet-Material 62 in Querrichtung 16 beidseits der Längsränder 46 jeweils einen Überhang 66a, 66b. Das Topsheet 64 überragt den Absorptionskörper 7 in Querrichtung nur verhältnismäßig geringfügig; jedoch ist beidseits des Absorptionskörpers 7 in Längsrichtung 9 verlaufend jeweils ein aufstehendes Barrieremittel 68 vorgesehen, welches typischerweise als aufstehendes Cuffelement oder Bündchenelement bezeichnet wird und vorzugsweise aus einem hydrophoben, insbesondere flüssigkeitsundurchlässigen Vliesstoffmaterial gebildet ist, welches sich in Querrichtung 16 vorzugsweise bis zu den seitlichen Längsrändern 48 des Schrittabschnitts 8 erstreckt. Das Barrieremittel 68 ist dabei in Fügestellen 76 an den Längsrändern 210 bzw. den Längsrandbereichen 212 des Topsheet-Materials 64 angefügt. Die distalen Enden 70 der Barrieremittel 68 sind mit weiteren Elastifizierungsmitteln 72 versehen, welche die Barrieremittel 68 im Gebrauch des Inkontinenzartikels gegen die Hautoberfläche des Benutzers anheben, so wie dies in Figur 5 schematisch dargestellt ist. An ihren jeweiligen Längsendbereichen 74 sind die seitlichen Barrieremittel 68 zusätzlich zur Fixierung in den Fügestellen 76 über die schematisch angedeuteten Fixierungen 78 auf das Topsheet 64 beziehungsweise auf sich selbst in einer C-förmig gefalteten Konfiguration festgelegt. Vorteilhaft und erwähnenswert ist hier, dass die in der Figur 4 jeweils innenliegende Fixierung 78 das Barrieremittel 68 auf sich selbst festlegt, und zwar in Querrichtung 16 innerhalb der äußeren Fixierung 76, welche eine in Längsrichtung 9 durchgehend erstreckte Cuffsockellinie 80 bildet. Die innere Fixierung 78 ist hingegen nur in den Längsendbereichen 74 der Barrieremittel 68 vorgesehen.

Es erweist sich hier als besonders vorteilhaft, wenn der erwähnte Überhang 66a, 66b des Backsheet-Materials 62 und/oder des Deckblatt-Materials 84 beidseits der Längsränder 46 des Absorptionskörpers 7, also in der Summe, wenigstens 25 % bezogen auf die größte Breite E des Schrittabschnitts 8 beträgt. Auf diese Weise ist nämlich in Querrichtung 16 Raum für die Anordnung entlang der Beinöffnungen 19 erstreckter Beinelastifizierungsmittel 82. Es erweist sich nämlich als vorteilhaft, wenn die Beinelastifizierungsmittel 82 mit einem gewissen Abstand vom materialreichen und damit eher biegesteifen Absorptionskörper 7 verlaufen, um einerseits keine zusätzlichen Dehnungs- oder Verwindungskräfte auf den Absorptionskörper auszuüben, was dessen Absorptionsverhalten nachteilig beeinflussen könnte, und um andererseits einen vom Absorptionskörper weitgehend unbeeinflussten flüssigkeitsdichten Beinabschluss zu realisieren. Es erweist sich im dargestellten Fall als besonders vorteilhaft, dass diese Beinelastifizierungsmittel 82 in Längsrichtung 9 mit einem deutlichen Abstand von insbesondere wenigstens 10 mm, vorzugsweise wenigstens 20 mm vor den zweiten Elastifizierungsmitteln 40 und 42 des Bauchabschnitts 4 beziehungsweise des Rückenabschnitts 6 enden. Vorzugsweise enden diese Beinelastifizierungsmittel 82 in Längsrichtung 9 vor dem Bauchabschnitt 4 und dem Rückenabschnitt 6. Dies ist deshalb vorteilhaft und wesentlich, weil die Beinelastifizierungsmittel 82 solchenfalls das Spannungsverhalten des Bauchabschnitts 4 und des Rückenabschnitts 6 wenig oder gar nicht beeinflussen. Es wurde nämlich erkannt, dass es sich im Hinblick auf das vorteilhaft zu erreichende Ziel der Verbesserung des Tragekomforts gerade in dem schrittseitigen und den Beinöffnungen 19 zugewandten Bereich 22 und 26 des Bauchabschnitts 4 und des Rückenabschnitts 6 als negativ erweist, wenn dort die üblicherweise mit großer Vorspannung und entsprechend großer Rückstellkraft ausgebildeten Beinelastifizierungsmittel 82 zusätzlich verlaufen.

Wie aus Figur 1 ersichtlich ist, ist bei dem Schrittabschnitt 8 ein in Querrichtung 16 verhältnismäßig großer Überhang 66a, 66b beidseits der Längsränder 46 des Absorptionskörpers 7 realisiert, und zwar insbesondere auch an dem Bauchabschnitt 4 beziehungsweise dem Rückenabschnitt 6 zugewandten Bereichen des Schrittabschnitts 8. Hierdurch wird - worauf bereits hingewiesen wurde - ein verhältnismäßig großer Überlappungsbereich 36, 38 des Schrittabschnitts 8 mit dem Bauchabschnitt 4 und mit dem Rückenabschnitt 6 realisiert. Nach einer bevorzugten Ausführungsvariante umfasst der Überlappungsbereich 36 von Schrittabschnitt 8 mit dem Bauchabschnitt 4 wenigstens 12% der Fläche des Bauchabschnitts 4, und der Überlappungsbereich 38 von Schrittabschnitt 8 mit dem Rückenabschnitt 6 umfasst wenigstens 20% der Fläche des Rückenabschnitts 6. Dies erweist sich als vorteilhaft, da solchenfalls eine sichere Fixierung des Schrittabschnitts 8 an dem Bauchabschnitt 4 beziehungsweise an dem Rückenabschnitt 6 erreicht werden kann, und zwar auch, wenn kein vollflächiger Kleberauftrag verwendet wird. Es ist solchenfalls in vorteilhafter Weise hinreichend, wenn ein nur abschnittsweiser oder gerasterter Kleberauftrag zur Verbindung verwendet wird. Dies ist deshalb vorteilhaft, weil solchenfalls die miteinander gefügten Materialien nicht zu steif werden.

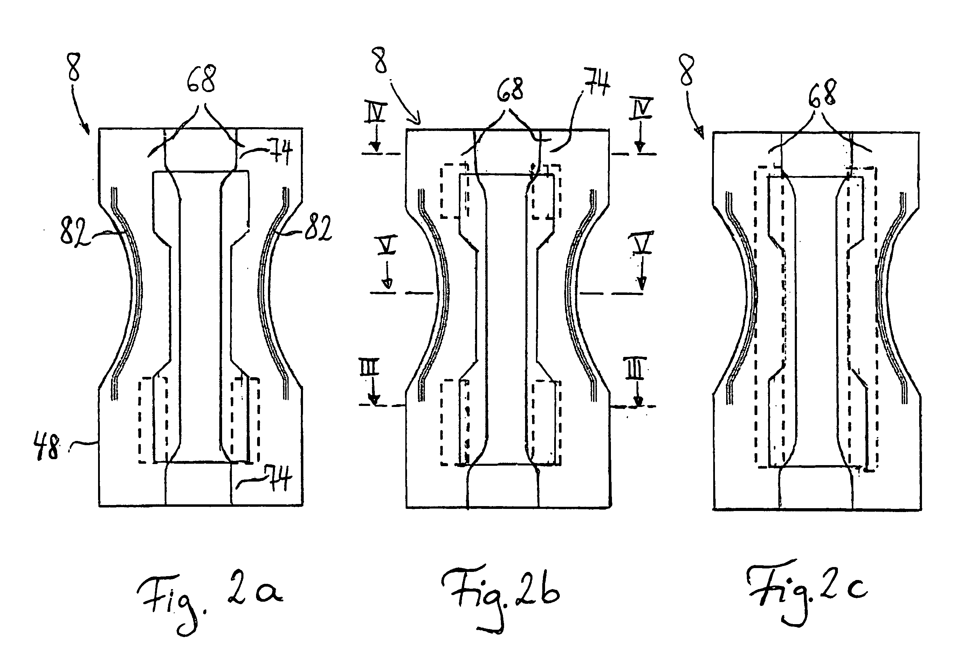

Wie in Figur 1 ersichtlich ist, weist der Inkontinenzartikel 2 Verstärkungsmittel 200 auf, welche im vorderen und im hinteren Überlappungsbereich 36, 38 angeordnet sind. Zusammen mit der Figur 3, welche eine schematische Schnittansicht entlang der Ebene III - III aus Figur 2b darstellt, wird ersichtlich, dass das Verstärkungsmittel 200 dabei in einem den jeweiligen Längsrand 46 des Absorptionskörpers 7 überbrückenden Bereich 204 angeordnet ist. Damit wird durch das Verstärkungsmittel 200 jeweils der Längsrandbereich 47 des Absorptionskörpers 7 überfangen als auch ein an den Längsrand 46 angrenzender Teilbereich 67 des Überhangs 66a, 66b.

Das Backsheet-Material 62 und das Deckblatt-Material 84 sind im Bereich des Überhangs 66a, 66b durch ein separates Fügemittel verbunden (in der Figur 3 nicht dargestellt). Auch zwischen Backsheet-Material und Absorptionskörper und zwischen Deckblatt-Material und Absorptionskörper ist ein separates Fügemittel vorgesehen. Diese separaten Fügemittel sind in Form eines Klebers nicht vollflächig, sondern in Form eines unterbrochenen Musters aufgetragen. Diese in Form eines Klebers vorgesehenen separaten Fügemittel sind also beispielsweise rasterförmig, streifenförmig oder als Spiralmuster aufgetragen.

Das Verstärkungsmittel 200 ist auf der Innenseite 85 des Deckblatt-Materials 84 angebracht. Das Deckblatt-Material ist, wie im vorliegenden Beispiel, ein Verbund aus einem Topsheet 64 und beidseits an den Längsrändern 210 bzw. den Längsrandbereichen 212 des Topsheets 64 angefügten Barrieremitteln 68. Das Verstärkungsmittel 200 ist dabei derart aufgebracht, dass die diese beiden Materialien verbindende Fügestelle 76 überfangen ist. Das Verstärkungsmittel 200 ist dabei als verstärkende Beschichtung 202, in Form eines Klebers, insbesondere eines Hotmeltklebers aufgebracht. Besonders bevorzugt ist hierbei ein hydrophober Kleber, insbesondere der Kleber LC 3001ZP von der Firma Fuller (H.B. Fuller Deutschland GmbH, An der Roten Bleiche 2-3, 21335 Lüneburg, Deutschland) aufgebracht. Die verstärkende Beschichtung 202 ist dabei in einem Flächengewicht von 2 - 10 g/m$^2$ aufgebracht.

Das Verstärkungsmittel 200 in dem überbrückenden Bereich 204 weist eine Gesamtbreite G von 20 - 50 mm, insbesondere vorzugsweise von 20 - 40 mm auf. In dem vom Verstärkungsmittel 200 überfangenden Teilbereich 67 des Überhangs 66a, 66b weist das Verstärkungsmittel eine Breite G' von vorzugsweise größer 5 mm und kleiner 30 mm auf. Das Verstärkungsmittel 200 überfängt den jeweiligen Teilbereich 67 des Überhangs (66a, 66b) in Querrichtung derart, dass der Anteil G'/ H des durch das Verstärkungsmittel (200) überfangenen Teilbereiches (67) des jeweiligen Überhangs (66a, 66b) mit der Breite (G') bezogen auf den jeweiligen Überhang (66a, 66b) mit der Breite (H) mindestens 0,10, aber vorzugsweise höchstens 0,80 beträgt.

Zudem überfängt das Verstärkungsmittel 200 den Längsrandbereich 47 des Absorptionskörpers 7 in Querrichtung in einer Breite G" von vorzugsweise größer 5 mm und kleiner 30 mm. Das Verstärkungsmittel 200 überfängt den Längsrandbereich 47 des Absorptionskörpers 7 derart, dass der Anteil G"/ H des jeweiligen durch das Verstärkungsmittel 200 überfangenen Längsrandbereichs 47 mit der Breite G" bezogen auf den jeweiligen Überhang 66a, 66b mit der Breite (H) mindestens 0,10 aber vorzugsweise höchstens 0,80 beträgt. Das Verstärkungsmitteel 200 überfängt den Längsrandbereich 47 des Absorptionskörpers 7 in Summe, also beidseits, in einem Anteil von mindestens 10% und höchstens 35 % bezogen auf die Breite K des Absorptionskörpers 7.

In der bevorzugten Ausführung, so wie in Figur 1 angedeutet, weisen sowohl der vordere Überlappungsbereich 36 als auch der hintere Überlappungsbereich 38 Verstärkungsmittel 200 auf.

Die Verstärkungsmittel 200 im vorderen und hinteren Überlappungsbereich 36, 38 unterscheiden sich in ihrer Erstreckung in Längsrichtung 9. Die Verstärkungsmittel 200 im vorderen und im hinteren Überlappungsbereich 36, 38 erstrecken sich dabei zumindest jeweils bis zum Querrand 88, 89 des Absorptionskörpers 7 und bis zum schrittzugewandten Querrand 58 des Bauchabschnitts 4 und bis zum schrittzugewandten Querrand 60 des Rückenabschnitts 6.

Es sind aber weitere Ausführungsformen der Verstärkungsmittel denkbar. Die Figuren 2a, 2b, 2c zeigen dabei Draufsichten auf einen Schrittabschnitt 8 des Inkontinenzartikels nach Figur 1 im flachgelegten und ausgedehnten Zustand. Der in Figur 1 dargestellte Schrittabschnitt 8 mit den darin im vorderen und hinteren Überlappungsbereich 36, 38 integrierten Verstärkungsmittel 200, so wie auch schematisch in Figur 2b dargestellt, kann durch eine alternative Ausführungsform des Schrittabschnitts 8, so wie in den Figuren 2a und 2c schematisch dargestellt, ersetzt sein.

So zeigt Figur 2a einen Schrittabschnitt 8 des Inkontinenzartikels nach Figur 1 im flachgelegten und ausgedehnten Zustand mit Verstärkungsmitteln 200, welche dann in Zusammenschau im gesamten Inkontinenzartikel 2, nur im hinteren Überlappungsbereich 38 vorhanden sind.

Figur 2c zeigt einen Schrittabschnitt 8 des Inkontinenzartikels 2 nach Figur 1 im flachgelegten und ausgedehnten Zustand mit Verstärkungsmittel 200, welche dann in Zusammenschau im gesamten Inkontinenzartikel 2 im vorderen und im hinteren Überlappungsbereich 36, 38 angeordnet sind. Ausgehend vom vorderen und vom hinteren Überlappungsbereich 36, 38 erstrecken sich die Verstärkungsmittel 200 über den jeweiligen schrittzugewandten Querrand 58, 60 des Bauchabschnittes 4 und des Rückenabschnittes 6 in Richtung Quermittelachse 30, und zwar derart, dass sich die Verstärkungsmittel 200 in Richtung Quermittelachse 30 miteinander verbindend erstrecken.

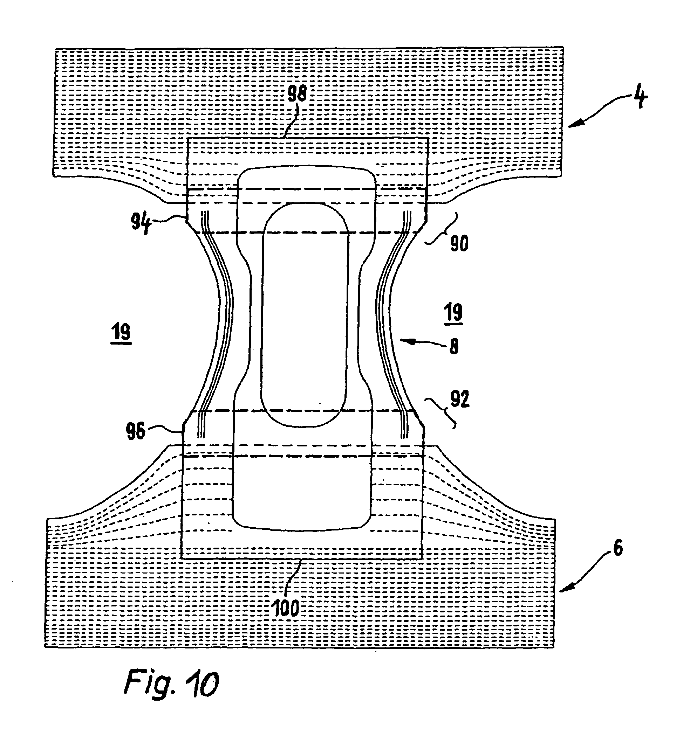

Anhand der Figur 10, die der Figur 1 entspricht, wird ein weiteres vorteilhaftes Detail einer bevorzugten Ausführungsform des Inkontinenzartikels erläutert. Durch die Verfolgung des dreikomponentigen Konzepts zur Herstellung des erfindungsgemäßen Inkontinenzartikels ergibt sich ein Übergang 90 zwischen Schrittabschnitt 8 und Bauchabschnitt 4 sowie ein Übergang 92 zwischen Schrittabschnitt 8 und Rückenabschnitt 6, bei denen sich üblicherweise ein unstetiger, also mit Ecken oder Winkeln oder Knicken versehener Verlauf der die Beinöffnungen 19 begrenzenden Ränder der Chassismaterialien ergibt. Dies birgt die Gefahr, dass im Bereich der Übergänge 90, 92 Kraftspitzen gebildet werden, die zu einem Einreißen der Chassismaterialien führen können, was die Anfügung des Schrittabschnitts 8 an den Bauchabschnitt 4 beziehungsweise an den Rückenabschnitt 6 beeinträchtigen kann. Um dem entgegen zu wirken ist im jeweiligen Übergang 90 und 92 eine verstärkende Beschichtung 94, 96 des flüssigkeitsundurchlässigen Backsheet-Materials 62 des Schrittabschnitts 8 vorgesehen. Es erweist sich als hinreichend, wenn diese verstärkende Beschichtung 94, 96 nur in dem durch die unterbrochene Linie jeweils bezeichneten Bereich der Figur 10 vorgesehen wird. Die verstärkende Beschichtung 94, 96 überlappt im beispielhaft und vorzugsweise dargestellten Fall den Bauchabschnitt 4 und den Rückenabschnitt 6 in Längsrichtung 9 lediglich um etwa 10 bis 20 mm, insbesondere um ca. 15 mm. Die verstärkende Beschichtung endet in Längsrichtung 9 jeweils vor den Längsenden 98, 100 des Schrittabschnitts, und zwar wenigstens 30 mm vor dem bauchseitigen Längsende 98 und wenigstens 90 mm vor dem rückenseitigen Längsende 100. Dies erweist sich als vorteilhaft, da solchenfalls die verstärkende Beschichtung 94, 96 nicht unnötig zur Versteifung der Chassismaterialien in Bereichen beiträgt, wo dies nicht hilfreich, sondern eher unerwünscht und nachteilig ist. Außerdem können auf diese Weise Materialkosten gespart werden. Jedoch bleibt die Möglichkeit unberührt, die verstärkende Beschichtung 94, 96 nicht nur im Übergang 90 beziehungsweise 92 vorzusehen.

Die verstärkende Beschichtung 94, 96 besteht vorzugsweise aus einem Vliesstoff, insbesondere aus einem Spinnvlies aus Polypropylen, insbesondere mit einem Flächengewicht von 10 - 20 g/m$^2$, insbesondere von 12 - 17 g/m$^2$.

Erfindungsgemäßes Verfahren:

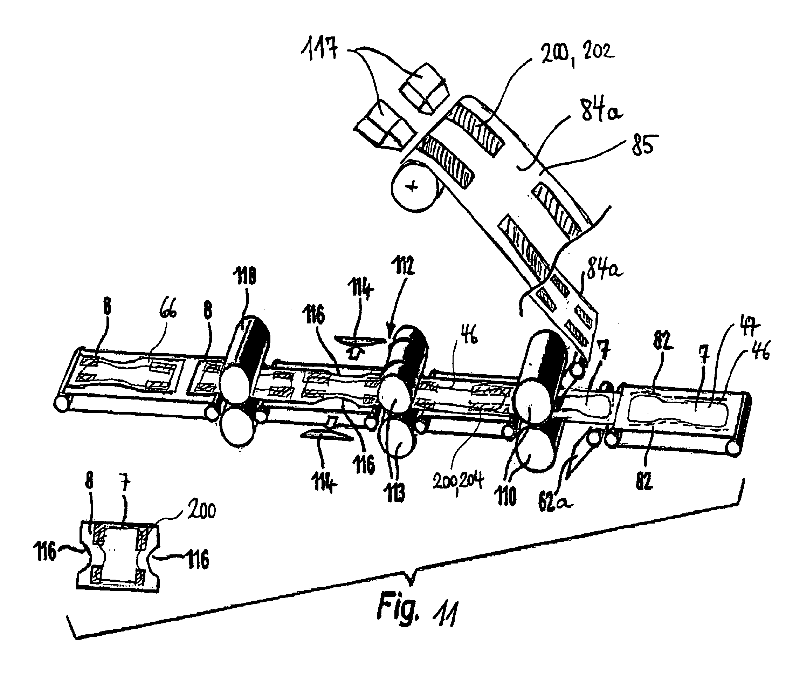

Die Figuren 11 bis 13 zeigen das erfindungsgemäße Herstellungsverfahren.
Hierfür werden einer schnell laufenden Herstellungsmaschine eine endlose Backsheet-Materialbahn 62a und eine endlose Deckblatt-Materialbahn 84a und aufeinander folgend Absorptionskörper 7 sowie (nur angedeutet) den späteren Beinöffnungen zugeordnete Beinelastifizierungsmittel 82 zugeführt.

Auf die spätere Innenseite 85 der endlosen Deckblatt-Materialbahn 84a wird zuvor ein Verstärkungsmittel 200 in Form einer verstärkenden Beschichtung 202 aufgetragen, wobei das Beschichtungsmaterial aus schematisch dargestellten Düsen 117 bereitgestellt wird. Vorzugsweise wird die verstärkende Beschichtung 202 mittels eines kontaktlosen Beschichtungsverfahrens auf die Deckblatt-Materialbahn 84a aufgebracht. Vorzugsweise wird die verstärkende Beschichtung vollflächig aufgetragen.

Das Verstärkungsmittel 200 wird dabei derart aufgetragen, dass dann nach Zusammenfügen von Deckblatt-Materialbahn 84a und Absorptionskörper 7, das Verstärkungsmittel 200 in jeweils einem den Längsrand 46 des Absorptionskörpers 7 überbrückenden Bereich 204 angeordnet ist. Das Verstärkungsmittel 200 überfängt dabei sowohl den an den Längsrand 46 angrenzenden Längsrandbereich 47 des Absorptionskörpers 7 als auch einen angrenzenden Teilbereich 67 des Überhangs 66.

Die Absorptionskörper 7 werden voneinander beabstandet zwischen die Deckblatt-Materialbahn 84a und die Backsheet-Materialbahn 62a angeordnet, und der so gebildete Verbund wird durch geeignete separate Fügemittel, insbesondere Kleber, vorzugsweise nicht vollflächig aufgetragen, z.B. rasterförmig, linienförmig oder als Spiralmuster, fixiert. Auch die Beinelastifizierungsmittel 82 werden zwischen die Deckblatt-Materialbahn 84a und die Backsheet-Materialbahn 62a zugeführt und fixiert. Hierfür wird ein erstes Walzenpaar 110 und in nicht dargestellter Weise ein Kleber verwendet. Der Vollständigkeit halber sei erwähnt, dass der Schrittabschnitt 8 zusätzlich seitliche aufstehende und vorzugsweise elastifizierte Cuffs als Seitenauslaufschutz umfasst. Diese sind vorliegend auf der körperzugewandten Seite der Deckblatt-Materialbahn 84a bereits vorgesehen jedoch nicht dargestellt; sie könnten aber auch an beliebiger Stelle des in Figur 11 dargestellten Fertigungsablaufs oder auch an späterer Stelle eingebracht werden.

In Förderrichtung an das vorstehend Beschriebene anschließend wird in dem Verbund ein erster Konturschnitt 112 unter Verwendung eines Schneidwalzenpaars 113 ausgeführt. Im Zuge des Konturschnitts 112 werden von dem den Schrittabschnitt 8 bildenden Verbund aus Backsheet-Materialbahn 62a und Deckblatt-Materialbahn 84a bogenförmige Segmente 114 ausgeschnitten, um so Beinausschnitte 116 bei dem Schrittabschnitt 8 zu bilden. Der Verbund wird dann zu einer nachfolgenden Schneidstation 118 gefördert, und dort wird quer zur Förderrichtung ein Trennschnitt zur Vereinzelung der Schrittabschnitte 8 des herzustellenden Inkontinenzartikels ausgeführt. Ebenfalls in Figur 11 angedeutet ist eine perspektivische Ansicht des Schrittabschnitts 8 mit Absorptionskörper 7, Verstärkungsmittel 200 und Beinausschnitten 116.

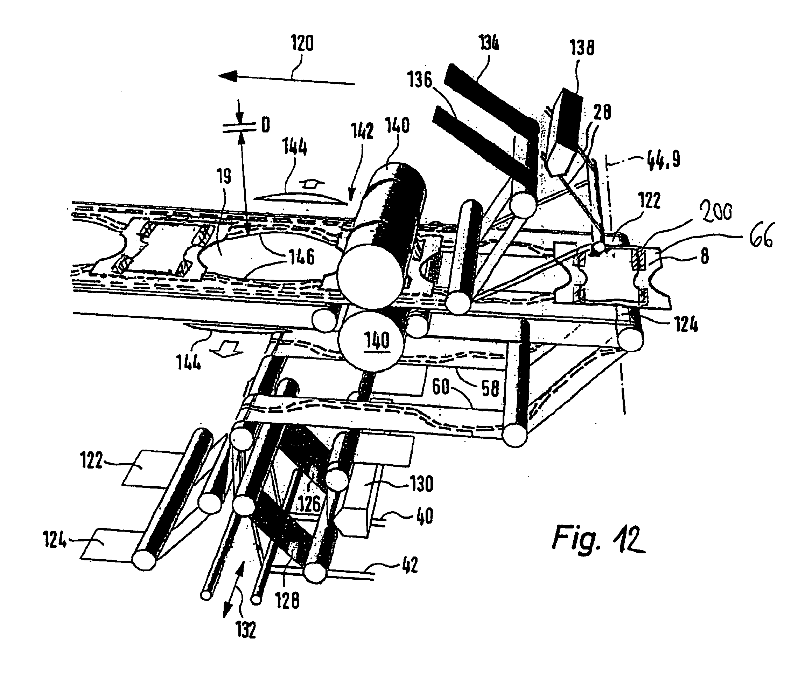

Nach dem Vereinzeln der Schrittabschnitte 8 werden diese im Zuge der weiteren Förderung um 90° gedreht und dann quer zur späteren Längsmittelachse 44 des Inkontinenzartikels 2 in der Maschinenrichtung 120 weitergefördert (Figur 12). Wie ebenfalls aus Figur 12 ersichtlich ist, werden zur Herstellung des späteren Bauchabschnitts 4 und Rückenabschnitts 6 des Inkontinenzartikels Teilbahnen 122, 124 auf Vliesbasis zugeführt. Diese Teilbahnen 122, 124 können ausgehend von einer einzigen Bahn durch Trennen in Längsrichtung gebildet sein. Auf diese Teilbahnen 122, 124 werden die eingangs erwähnten zweiten Elastifizierungsmittel 40 und 42 aufgebracht, die hierfür ebenfalls endlos und in Förderrichtung der Teilbahnen 122, 124 zugeführt werden. Zur Fixierung der zweiten Elastifizierungsmittel 40, 42 auf den Teilbahnen 122, 124 wird jeweils eine Vliesdeckschicht 126, 128, die zuvor in einer Beleimungsstation 130 mit Kleber beaufschlagt wurde, aufgebracht, so dass die zweiten Elastifizierungsmittel 40, 42 zwischen den Teilbahnen 122, 124 und den Vliesdeckschichten 126, 128 einlaminiert werden. Obschon aus Figur 12 infolge der schematischen Darstellung nicht ersichtlich werden die zweiten Elastifizierungsmittel 40, 42 in einem variierenden Abstand voneinander zugeführt, was durch eine oszillierende Führungseinrichtung, die durch den Doppelpfeil 132 angedeutet ist, realisiert wird. So wird durch entsprechende Ansteuerung der Führungseinrichtung für jedes einzelne der Elastifizierungsmittel 40, 42 der bogenförmige sich auffächernde Verlauf der zweiten Elastifizierungsmittel 40, 42 in Richtung auf den Schrittabschnitt 8 gebildet.

Die Teilbahnen 122, 124 werden dann in dem beschriebenen Verbund weitergefördert und mit den Schrittabschnitten 8 zusammengeführt, und zwar derart, dass die Schrittabschnitte in einer Längsrichtung 9 quer zur Maschinenrichtung 120 einenends mit der einen Teilbahn 122 und anderenends mit der anderen Teilbahn 124 überlappen.

Durch das Verstärkungsmittel 200 erhält der Überhang 66 des Schrittabschnitts 8 eine Stabilisierung. Dieser mit dem Verstärkungsmittel überfangende Teilbereich des Überhangs kann damit vorteilhaft faltenfrei auf die Teilbahnen 122, 124, also den späteren Bauchabschnitt und Rückenabschnitt angeordnet werden kann. Die Anordnung der Schrittabschnitte 8 zu den Teilbahnen 122 und 124 ist dabei derart, dass das Verstärkungsmittel 200 zumindest in dem mit den Teilbahnen 122, 124 überlappenden Bereich vorgesehen ist.

Die Schrittabschnitte 8 werden so zugeführt, dass sie nach dem Zusammenführen mit einem Abstand in der Maschinenrichtung 120 voneinander angeordnet sind. Die Schrittabschnitte 8 und die Teilbahnen 122, 124 werden in der aus Figur 12 erhaltenen Konfiguration miteinander fixiert und in der Maschinenrichtung 120 weitergefördert.

Es werden dann die in Quer- oder Hüftumfangsrichtung 16 erstreckten ersten Elastifizierungsmittel 28 endlos in der Maschinenrichtung 120 zugeführt und auf den Teilbahnen 122, 124 fixiert. Es werden wiederum Vliesbahnen 134, 136 zugeführt. Jedoch werden die Vliesbahnen 134, 136 nicht direkt mit Kleber versehen, sondern der Kleber wird auf die ersten Elastifizierungsmittel 28 aufgebracht, und die ersten Elastifizierungsmittel 28 werden dann auf die Teilbahnen 122, 124 aufgebracht und von den Vliesbahnen 134, 136 überdeckt, so dass sie einlaminiert werden.

Es wäre aber möglicherweise auch denkbar, dass die Elastifizierungsmittel 28 und 40, 42 alle einzeln mit Kleber versehen werden, also fadenbeleimt werden.

Es wäre möglicherweise auch denkbar, dass unabhängig von der Art des Klebemittelauftrags auf die Elastifizierungsmittel 28 und 40, 42 auf die Vliesdeckschichten 126, 128, 134 und/oder 136 verzichtet werden kann. Allerdings haben die Vliesdeckschichten den Vorteil, dass sie zugleich eine weich anfühlende Innenseite des Inkontinenzartikels bilden.

Dem nachfolgend ist in Figur 12 ein weiteres Schneidwalzenpaar 140, also ein Rotationsmesser mit einer Ambosswalze, vorgesehen, zwischen denen der bislang gebildete Verbund in der Maschinenrichtung 120 in der beschriebenen Orientierung hindurchgeführt wird. Dabei wird ein zweiter Konturschnitt 142 ausgeführt, im Zuge dessen, vorzugsweise nur von den Teilbahnen 122, 124, je ein bogenförmiges Segment 144 abgetrennt wird, und zwar von den einander zugewandten Querrändern oder Randabschnitten 58 und 60 der Teilbahnen 122, 124, so dass Beinausschnitte 146 auch bei den Teilbahnen 122, 124 gebildet werden. Dadurch, dass der zweite Konturschnitt 142 nicht den Schrittabschnitt 8 sondern nur die Teilbahnen 122, 124 erfasst, verläuft der zweite Konturschnitt 142 im Wesentlichen entlang der Maschinenrichtung 120 und jedenfalls nicht unter großem Winkel quer hierzu. Auf diese Weise kann der Schnitt optimal konfiguriert werden, ebenso wie der erste Konturschnitt 112 im Zuge der Herstellung des Schrittabschnitts 8. Insgesamt können so die späteren Beinöffnungen 19 des Inkontinenzartikels 2 mit hoher Genauigkeit den als optimal erachteten Anforderungen entsprechend ausgebildet werden. Dabei erweist es sich als vorteilhaft, dass der zweite Konturschnitt 142 an der Teilbahn 122 mit einem anderen Verlauf ausgebildet sein kann als an der Teilbahn 124. So kann die Gestalt der Beinausschnitte 146 bzw. der späteren Beinöffnungen 19 des Inkontinenzartikels 2 im Bauchabschnitt 4 und im Rückenabschnitt 6 verschieden konfiguriert werden.

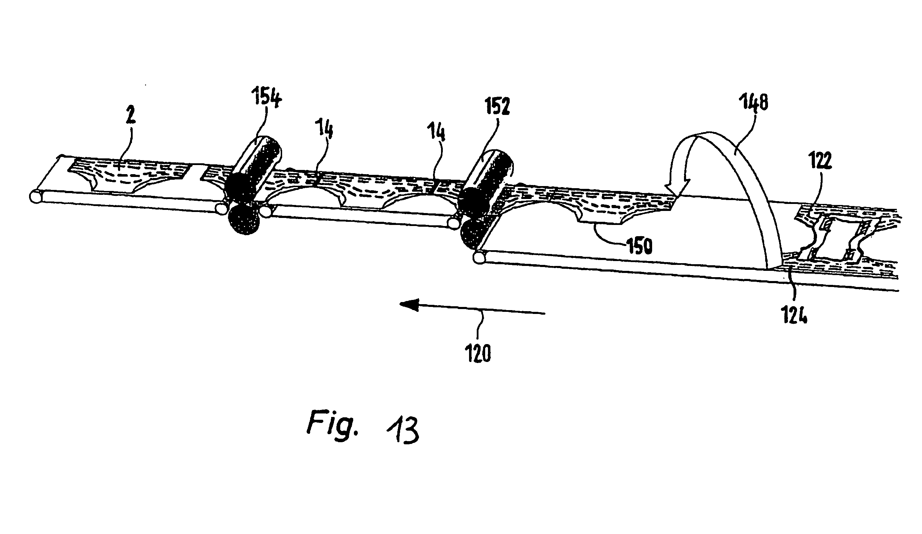

Der so gebildete Verbund wird weitergefördert, und in einer in Figur 13 nur angedeuteten Faltstation 148 wird der Verbund um eine in Maschinenrichtung 120 verlaufende Faltlinie 150 auf sich selbst gefaltet, derart, dass die eine Teilbahn 124 über die andere Teilbahn 122 zu liegen kommt. Daran anschließend wird in einer Fügestation 152 ein jeweiliger Seitennahtbereich 14 zwischen den Teilbahnen 122, 124 ausgebildet, es wird also die eigentliche Höschenform gebildet. An diesen Verfahrensschritt anschließend wird in einer Trennstation 154 ein Trennschnitt quer zur Maschinenrichtung 120 ausgeführt, der zur Vereinzelung der fertigen Inkontinenzartikel 2 führt. Es wäre auch denkbar, dass die Fügestation 152 zugleich als Trennstation ausgebildet ist, beispielsweise in Form einer Trennschweißeinrichtung, so dass zusammen mit der Ausbildung des Seitennahtbereichs 14 die Inkontinenzartikel 2 vereinzelt werden.

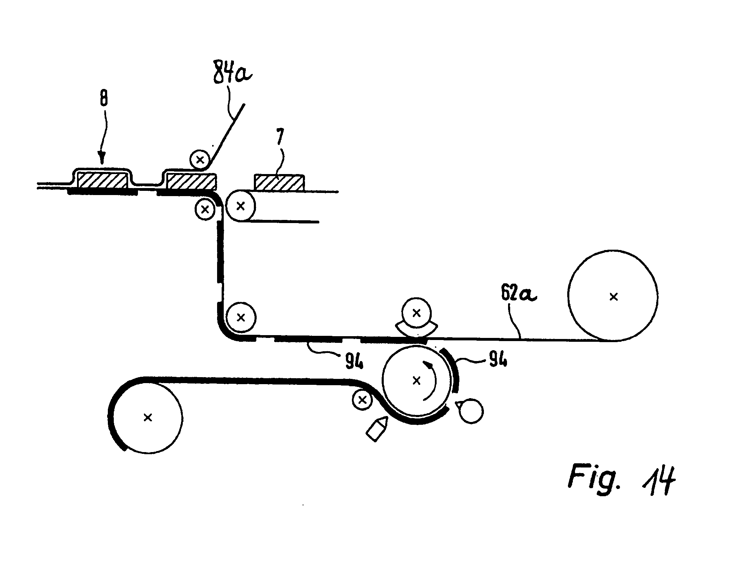

Schließlich zeigt Figur 14 schematisch eine Verfahrensführung, bei der die Backsheet-Materialbahn 62a abschnittsweise mit einer im Zusammenhang mit Figur 10 erläuterten verstärkenden Beschichtung 94 versehen wird, und zwar im Zuge der Zuführung der Backsheet-Materialbahn 62a zur Herstellung der Schrittabschnitte, was bereits im Zusammenhang mit Figur 11 erläutert wurde. Im Unterschied zu Figur 10 überfängt die verstärkende Beschichtung 94, bei der es sich insbesondere um einen Vliesabschnitt handeln kann, im Wesentlichen einen Großteil des Schrittabschnitts 8; an den Längsenden des zu bildenden Schrittabschnitts 8 bleibt die Backsheet-Materialbahn 62a aber unbeschichtet.

**Patentansprüche**

1. Inkontinenzartikel (2) in Höschenform für die Aufnahme von Körperausscheidungen mit einem vorderen Bauchabschnitt (4) und einem hinteren Rückenabschnitt (6), die zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, und mit einem einen Absorptionskörper (7) aufweisenden Schrittabschnitt (8), der sich in einer Längsrichtung (9) zwischen dem Bauchabschnitt (4) mit einem schrittzugewandten Querrand (58) und dem Rückenabschnitt (6) mit einem schrittzugewandten Querrand (60) erstreckt, wobei der Schrittabschnitt (8) an den Bauchabschnitt (4) und an den Rückenabschnitt (6) unlösbar angefügt ist, wobei sowohl der Schrittabschnitt (8) als auch der Bauchabschnitt (4) und der Rückenabschnitt (6) die Beinöffnungen (19) des Inkontinenzartikels begrenzen, wobei in dem Bauchabschnitt (4) und dem Rückenabschnitt (6) erste Elastifizierungsmittel (28) vorgesehen sind, die sich in einem Abstand voneinander und parallel zueinander in Quer- oder Hüftumfangsrichtung (16) erstrecken und so den Bauchabschnitt (4) und den Rückenabschnitt (6) flächenhaft elastifizieren, wobei in einem schrittseitigen und den Beinöffnungen (19) zugewandten Bereich (22, 26) des Bauchabschnitts (4) und des Rückenabschnitts (6) zweite Elastifizierungsmittel (40, 42) vorgesehen sind, wobei der Schrittabschnitt (8) ein flüssigkeitsundurchlässiges Backsheet-Material (62) mit einer Innenseite (63) und mit einem Flächengewicht von 10 - 40 g/m$^2$ und ein Deckblatt-Material (84) auf Vliesbasis mit einer Innenseite (85) umfasst, und wobei der Absorptionskörper (7) zwischen Backsheet-Material (62) und Deckblatt-Material (84) angeordnet ist und der Absorptionskörper (7) eine Breite (K), Längsränder (46), daran angrenzende Längsrandbereiche (47) und Querränder (88, 89) aufweist, wobei das Deckblatt-Material (84) oder das Deckblatt-Material (84) und das Backsheet-Material (62) in Querrichtung (16) einen sich jeweils außerhalb der Längsränder (46) des Absorptionskörpers (7) erstreckenden Überhang (66a, 66b) bilden, **dadurch gekennzeichnet, dass** der Schrittabschnitt (8) in Querrichtung (16) eine Breite (E) von wenigstens 200 mm aufweist und der Überhang (66a, 66b) in der Summe, also beidseits der Längsränder (46) des Absorptionskörpers (7) wenigstens 25 % bezogen auf die größte Breite des Schrittabschnitts (8) beträgt, wobei der Schrittabschnitt (8) in einem vorderen Überlappungsbereich (36) wenigstens 12 % der Fläche des Bauchabschnitts (4) und in einem hinteren Überlappungsbereich (38) wenigstens 20 % der Fläche des Rückenabschnitts (6) überlappt, und wobei der Überhang (66a, 66b) im vorderen und/oder im hinteren Überlappungsbereich (36, 38) ein Verstärkungsmittel (200) aufweist, wobei das Verstärkungsmittel (200) in jeweils einem den jeweiligen Längsrand (46) des Absorptionskörpers (7) überbrückenden Bereich (204) vorgesehen ist, also sowohl jeweils einen Längsrandbereich (47) des Absorptionskörpers (7) überfängt als auch zumindest jeweils in einem daran angrenzenden Teilbereich (67) des Überhangs (66a, 66b) vorgesehen ist und wobei das Verstärkungsmittel (200) in diesem Teilbereich (67) in Querrichtung (16) betrachtet schmäler als der Überhang (66a, 66b) ausgebildet ist, wobei das Verstärkungsmittel (200) eine verstärkende Beschichtung (202) mit einem Flächengewicht von 1 - 30 g/m$^2$ umfasst oder daraus besteht, und wobei das Verstärkungsmittel (200) auf der Innenseite (85) des Deckblatt-Materials (84) angeordnet ist, und wobei das Deckblatt-Material (84) ein Flächengewicht von 5 - 20 g/m$^2$ aufweist.

2. Inkontinenzartikel nach Anspruch 1, **dadurch gekennzeichnet, dass** das Verstärkungsmittel (200) sowohl im vorderen als auch im hinteren Überlappungsbereich (36, 38) in jeweils einem den jeweiligen Längsrand (46) des Absorptionskörpers (7) überbrückenden Bereich (204) vorgesehen ist.

3. Inkontinenzartikel nach einem der vorstehenden Ansprüche 1 oder 2, **dadurch gekennzeichnet, dass** das Verstärkungsmittel (200) sich in Längsrichtung (9) zumindest bis zum Querrand (88, 89) des Absorptionskörpers (7) und bis zum schrittzugewandten Querrand (58, 60) des Bauchabschnitts (4) und/oder des Rückenabschnitts (6) erstreckt.

4. Inkontinenzartikel nach einem oder mehreren der vorstehenden Ansprüche, **dadurch gekennzeichnet, dass** das Verstärkungsmittel (200) ausgehend vom vorderen und/oder vom hinteren Überlappungsbereich (36, 38) sich über den jeweiligen schrittzugewandten Querrand (58, 60) des Bauchabschnittes (4) und/oder Rückenabschnittes (6) hinaus in Richtung Quermittelachse (30) erstreckt.

5. Inkontinenzartikel nach einem oder mehreren der vorstehenden Ansprüche, **dadurch gekennzeichnet, dass** das Verstärkungsmittel (200) im vorderen Überlappungsbereich (36) und das Verstärkungsmittel (200) im hinteren Überlappungsbereich (38) sich miteinander verbindend in Richtung Quermittelachse (30) erstrecken.

6. Inkontinenzartikel nach einem oder mehreren der vorstehenden Ansprüche, **dadurch gekennzeichnet, dass** der Anteil G'/ H des durch das Verstärkungsmittel (200) überfangenen Teilbereiches (67) des jeweiligen Überhangs (66a, 66b) mit der Breite (G') bezogen auf den jeweiligen Überhang (66a, 66b) mit der Breite (H) mindestens 0,10, insbesondere mindestens 0,15, weiter insbesondere mindestens 0,20, aber vorzugsweise höchstens 0,80, insbesondere höchstens 0,75, weiter insbesondere höchstens 0,70 beträgt.

7. Inkontinenzartikel nach einem oder mehreren der vorstehenden Ansprüche, **dadurch gekennzeichnet, dass** der Anteil G"/ H des jeweiligen durch das Verstärkungsmittel (200) überfangenen Längsrandbereichs (47) mit der Breite (G") bezogen auf den jeweiligen Überhang (66a, 66b) mit der Breite (H) mindestens 0,10, insbesondere mindestens 0,15, weiter insbesondere mindestens 0,20 , aber vorzugsweise höchstens 0,80, insbesondere höchstens 0,75, weiter insbesondere höchstens 0,70 beträgt.

8. Inkontinenzartikel nach einem oder mehreren der vorstehenden Ansprüche, **dadurch gekennzeichnet, dass** das Verstärkungsmittel (200) den Längsrandbereich (47) des Absorptionskörpers (7) in Querrichtung (16) in Summe, also beidseits, in einem Anteil von mindestens 5%, insbesondere mindestens 10%, weiter insbesondere mindestens 15%, aber vorzugsweise höchstens 35%, insbesondere höchstens 30%, weiter insbesondere höchstens 25% bezogen auf die Breite (K) des Absorptionskörpers (7) überfängt.

**9.** Inkontinenzartikel nach einem oder mehreren der vorstehenden Ansprüche, **dadurch gekennzeichnet, dass** das Verstärkungsmittel (200) parallel zur Längsrichtung (9) und als Streifen mit einer gleich bleibenden Breite (G) angeordnet ist.

**10.** Inkontinenzartikel nach einem oder mehreren der vorstehenden Ansprüche, **dadurch gekennzeichnet, dass** die verstärkende Beschichtung (202) ein Flächengewicht von 2 - 20 g/m$^2$, weiter insbesondere von 2 -10 g/m$^2$ aufweist.

**11.** Inkontinenzartikel nach einem oder mehreren der vorstehenden Ansprüche, **dadurch gekennzeichnet, dass** die verstärkende Beschichtung (202) einen Kleber, insbesondere einen Hotmeltkleber, weiter insbesondere einen hydrophoben Hotmeltkleber umfasst.

**12.** Inkontinenzartikel nach einem oder mehreren der vorstehenden Ansprüche, **dadurch gekennzeichnet, dass** das Deckblatt-Material (84) ein Verbund aus einem flüssigkeitsdurchlässigen Topsheet-Material (64) mit Längsrändern (210) und angrenzenden Längsrandbereichen (212) und beidseits an den Längsrandbereichen (212) des Topsheet-Materials (64) in Fügestellen (76) angefügten hydrophoben Barrieremitteln (68) ist.

**13.** Inkontinenzartikel nach Anspruch 12, **dadurch gekennzeichnet, dass** das Verstärkungsmittel (200) die Fügestellen (76) des Deckblatt-Materials (84) überfängt.

**14.** Inkontinenzartikel nach einem oder mehreren der vorstehenden Ansprüche, **dadurch gekennzeichnet, dass** der Überhang (66a, 66b) des Backsheet-Materials (62) und/oder des Deckblatt-Materials (84) in Querrichtung (16) in der Summe, also beidseits der Längsränder (46) des Absorptionskörpers (7) 25 - 50 %, insbesondere 30 - 45 % und weiter insbesondere 35 - 45 % bezogen auf die größte Breite (E) des Schrittabschnitts (8) beträgt.

**15.** Verfahren zum Herstellen von Inkontinenzartikeln (2) in Höschenform für die Aufnahme von Körperausscheidungen, mit einem vorderen Bauchabschnitt (4) und einem hinteren Rückenabschnitt (6), die zur Bildung eines in Quer- oder Hüftumfangsrichtung (16) durchgehenden Bauch- und Rückenbands mit einer in Hüftumfangsrichtung (16) geschlossenen Hüftöffnung (18) an beidseitigen Seitennahtbereichen (14) herstellerseitig miteinander verbunden sind, und mit einem einen Absorptionskörper (7) aufweisenden Schrittabschnitt (8), der sich in einer Längsrichtung (9) zwischen Bauchabschnitt (4) mit einem schrittzugewandten Querrand (58) und Rückenabschnitt (6) mit einem schrittzugewandten Querrand (60) erstreckt, wobei der Schrittabschnitt (8) an den Bauchabschnitt (4) und an den Rückenabschnitt (6) unlösbar angefügt ist, wobei sowohl der Schrittabschnitt (8) als auch der Bauchabschnitt (4) und der Rückenabschnitt (6) die Beinöffnungen (19) des Inkontinenzartikels begrenzen, wobei in dem Bauchabschnitt (4) und dem Rückenabschnitt (6) erste Elastifizierungsmittel (28) vorgesehen sind, die sich in einem Abstand voneinander und parallel zueinander in Quer- oder Hüftumfangsrichtung (16) erstrecken und so den Bauchbereich (4) und den Rückenbereich (6) flächenhaft elastifizieren, wobei in einem schrittseitigen und den Beinöffnungen (19) zugewandten Bereich (22, 26) des Bauchabschnitts (4) und des Rückenabschnitts (6) zweite Elastifizierungsmittel (40, 42) vorgesehen sind, wobei der Schrittabschnitt (8) ein flüssigkeitsundurchlässiges Backsheet-Material (62) mit einer Innenseite (63) und mit einem Flächengewicht von 10 - 40 g/m$^2$ und ein Deckblatt-Material (84) auf Vliesbasis mit einer Innenseite (85) und mit einem Flächengewicht von 5 - 20 g/m$^2$ umfasst, und wobei der Absorptionskörper (7) zwischen Backsheet-Material (62) und Deckblatt-Material (84) angeordnet ist und der Absorptionskörper (7) eine Breite (K), Längsränder (48), daran angrenzende Längsrandbereiche (47) und Querränder (88,89) aufweist, wobei das Deckblatt-Material (84) oder das Deckblatt-Material (84) und das Backsheet-Material (62) in Querrichtung (16) einen sich jeweils außerhalb der Längsränder des Absorptionskörpers (7) erstreckenden Überhang (66a, 66b) bilden, wobei der Schrittabschnitt (8) in Querrichtung (16) eine Breite (E) von wenigstens 200 mm aufweist und der Überhang (66a, 66b) in Querrichtung (16) in der Summe, also beidseits der Längsränder (46) des Absorptionskörpers (7) wenigstens 25 % bezogen auf die größte Breite (E) des Schrittabschnitts (8) beträgt, wobei der Schrittabschnitt (8) in einem vorderen Überlappungsbereich (36) wenigstens 12 % der Fläche des Bauchabschnitts (4) und in einem hinteren Überlappungsbereich (38) wenigstens 20 % der Fläche des Rückenabschnitts (6) überlappt, wobei der Überhang (66a,66b) im vorderen und/oder im hinteren Überlappungsbereich (36,38) ein Verstärkungsmittel (200) aufweist, und wobei das Verfahren die Bereitstellung des Schrittabschnitts (8) umfasst und die Bereitstellung des Schrittabschnitts (8) die folgenden Verfahrensschritte aufweist:

- Zuführen einer das spätere Deckblatt-Material (84) bildenden endlosen Deckblatt-Materialbahn (84a),
- bereichsweises Aufbringen eines Verstärkungsmittel (200) als verstärkende Beschichtung(202) auf der Deckblatt-Materialbahn (84a), derart dass das Verstärkungsmittel (200) auf der späteren Innenseite (85) des Deckblatt-Materials (84) angeordnet ist, und derart, dass das Verstärkungsmittel (200) in jeweils einem den jeweiligen späteren Längsrand (46) des Absorptionskörpers (7) überbrückenden Bereich (204) vorgesehen ist, also sowohl einen jeweiligen späteren Längsrandbereich (47) des Absorptionskörpers (7) überfängt als auch zumindest in einem jeweiligen angrenzenden Teilbereich (67) des späteren Überhangs (66a, 66b) vorgesehen ist und derart, dass das Verstärkungsmittel (200) in diesem Teilbereich (67) in Querrichtung (16) betrachtet schmäler ausgebildet ist als der Überhang (66a, 66b),
- Zuführen einer das spätere Backsheet-Material (62) bildenden endlosen Backsheet-Materialbahn (62a),
- Zuführen und Anordnen von Absorptionskörpern (7) voneinander beabstandet zwischen der Deckblatt- Materialbahn (84a) und der Backsheet-Materialbahn (62a).

16. Verfahren nach Anspruch 15, **dadurch gekennzeichnet, dass** das Verstärkungsmittel derart aufgebracht wird, dass der Überhang (66a, 66b) sowohl im hinteren Überlappungsbereich (38)als auch im vorderen Überlappungsbereich (36) ein Verstärkungsmittel (200) aufweist.

17. Verfahren nach einem oder mehreren der vorstehenden Ansprüche 15 - 16, **dadurch gekennzeichnet, dass** das Verstärkungsmittel (200) derart aufgetragen wird, dass der Anteil G'/H des durch das Verstärkungsmittel (200) überfangenen Teilbereiches (67) des jeweiligen Überhangs (66a, 66b) mit der Breite (G') bezogen auf den jeweiligen Überhang (66a, 66b) mit der Breite (H) mindestens 0,10, insbesondere mindestens 0,15, weiter insbesondere mindestens 0,20 , aber vorzugsweise höchstens 0,80, insbesondere höchstens 0,75, weiter insbesondere höchstens 0,70 beträgt.

18. Verfahren nach einem oder mehreren der vorstehenden Ansprüche 15 - 17, **dadurch gekennzeichnet, dass** das Verstärkungsmittel derart aufgetragen wird, dass der Anteil G"/H des jeweiligen durch das Verstärkungsmittel (200) überfangenen Längsrandbereichs (47) mit der Breite (G") bezogen auf den jeweiligen Überhang (66a, 66b) mit der Breite (H) mindestens 0,10, insbesondere mindestens 0,15, weiter insbesondere mindestens 0,20 , aber vorzugsweise höchstens 0,80, insbesondere höchstens 0,75, weiter insbesondere höchstens 0,70 beträgt.

19. Verfahren nach einem oder mehreren der vorstehenden Ansprüche 15- 18, **dadurch gekennzeichnet, dass** das Verstärkungsmittel (200) derart aufgetragen wird, dass das Verstärkungsmittel (200) den späteren Längsrandbereich (47) des Absorptionskörpers (7) in Querrichtung (16) in Summe, also beidseits, in einem Anteil von mindestens 5%, insbesondere mindestens 10%, weiter insbesondere mindestens 15% und höchstens 35%, insbesondere höchstens 30%, weiter insbesondere höchstens 25% bezogen auf die Breite (K) des Absorptionskörpers (7) überfängt.

20. Verfahren nach einem oder mehreren der vorstehenden Ansprüche 15 - 19, **dadurch gekennzeichnet, dass** das Verstärkungsmittel (200) parallel zur späteren Längsrichtung (9) und streifenförmig mit einer gleich bleibenden Breite (G) aufgetragen wird.

21. Verfahren nach einem oder mehreren der vorstehenden Ansprüche 15 - 20, **dadurch gekennzeichnet, dass** die verstärkende Beschichtung (202) mit einem Flächengewicht von 1 - 30 g/m$^2$, insbesondere von 2 - 20g/m$^2$, weiter insbesondere 2 - 10 g/m$^2$ aufgetragen wird.

22. Verfahren nach einem oder mehreren der vorstehenden Ansprüche 15-21, **dadurch gekennzeichnet, dass** im Bereich des Überhangs (66a, 66b) zwischen Deckblatt-Material (84) und Backsheet-Material (62) und/oder zwischen Deckblatt-Material (84) und Absorptionskörper (7) und/oder zwischen Backsheet-Material (62) und Absorptionskörper (7) separate Fügemittel, vorzugsweise nicht vollflächig, vorgesehen sind.

23. Verfahren nach einem oder mehreren der vorstehenden Ansprüche 15 - 22 mit den weiteren Verfahrensschritten

- Zuführen zweier den späteren Bauchabschnitt (4) bzw. den späteren Rückenabschnitt (6) des Inkontinenzartikels (2) bildenden Teilbahnen (122, 124) auf Vliesbasis,
- Zuführen und Aufbringen der zweiten Elastifizierungsmittel (40, 42) auf die beiden Teilbahnen (122, 124) und Fixieren daran,
- Zusammenführen der Schrittabschnitte (8) mit den beiden Teilbahnen (122, 124), derart, dass die Schrittabschnitte (8) in einer Längsrichtung (9) quer zur Maschinenrichtung (120) einenends mit der einen Teilbahn (122) und anderenends mit der anderen Teilbahn (124) im vorderen und/oder hinteren Überlappungsbereich (36, 38) plan angeordnet sind und die Schrittabschnitte (8) mit einem Abstand in der Maschinenrichtung (120) voneinander angeordnet sind, und Fixieren der Schrittabschnitte (8) und Teilbahnen (122, 124) in den Überlappungsbereichen (36, 38), und Weiterfördern in der Maschinenrichtung (120),

- Zuführen, Aufbringen und Fixieren der ersten Elastifizierungsmittel (28) in der Maschinenrichtung (120) an den Teilbahnen (122, 124),

- Falten um eine in Maschinenrichtung (120) verlaufende Faltlinie (150), derart, dass die eine Teilbahn (122) über die andere Teilbahn (124) zu liegen kommt,

- Fügen der übereinander liegenden Teilbahnen (122, 124) quer zur Maschinenrichtung (120) in Abständen voneinander zur Ausbildung von Seitennahtbereichen (14) der herzustellenden Inkontinenzartikel (2), und Erhalt von einen Bauchabschnitt (4), einen Rückenabschnitt (6) und einen dazwischen angeordneten Schrittabschnitt (8) aufweisenden Produkten,

- Ausführen eines Trennschnitts quer zur Maschinenrichtung (120) und Erhalt von vereinzelten fertigen Inkontinenzartikeln (2).

**Claims**

1. Incontinence article (2) in the form of briefs for receiving body excretions, with a front stomach portion (4) and a rear back portion (6), which are connected to each other at side seam regions (14) on both sides at the manufacturer's in order to form a stomach and back band which is continuous in the transverse or waist-encircling direction (16) and has a waist opening (18) that is closed in the waist-encircling direction, and with a crotch portion (8), which has an absorbent body (7) and extends in a longitudinal direction (9) between the stomach portion (4) with a crotch-facing transverse edge (58) and the back portion (6) with a crotch-facing transverse edge (60), wherein the crotch portion (8) is joined non-detachably to the stomach portion (4) and to the back portion (6), wherein the crotch portion (8) and also the stomach portion (4) and the back portion (6) delimit the leg openings (19) of the incontinence article, wherein first elasticating means (28) are provided in the stomach portion (4) and the back portion (6), are spaced apart from one another and extend parallel to one another in the transverse or waist-encircling direction (16) and thus elasticate the stomach portion (4) and the back portion (6) across the surface area thereof, wherein second elasticating means (40, 42) are provided in a crotch-side region (22, 26) of the stomach portion (4) and of the back portion (6) facing the leg openings (19), wherein the crotch portion (8) comprises a liquid-impermeable backsheet material (62), with an inner side (63) and with a weight per unit area of 10-40 g/m$^2$, and a nonwoven-based cover sheet material (84) with an inner side (85), and wherein the absorbent body (7) is arranged between backsheet material (62) and cover sheet material (84) and the absorbent body (7) has a width (K), longitudinal edges (46), longitudinal edge regions (47) adjoining the latter, and transverse edges (88, 89), wherein the cover sheet material (84) or the cover sheet material (84) and the backsheet material (62) form(s), in the transverse direction (16), an overhang (66a, 66b) extending in each case outside the longitudinal edges (46) of the absorbent body (7), **characterized in that** the crotch portion (8) has a width (E) of at least 200 mm in the transverse direction (16), and the overhang (66a, 66b) measures in total, that is to say on both sides of the longitudinal edges (46) of the absorbent body (7), at least 25% relative to the greatest width of the crotch portion (8), wherein the crotch portion (8) overlaps at least 12% of the surface of the stomach portion (4) in a front overlapping region (36) and at least 20% of the surface of the back portion (6) in a rear overlapping region (38), and wherein the overhang (66a, 66b) has a reinforcing means (200) in the front and/or in the rear overlapping region (36, 38), wherein the reinforcing means (200) is provided in a region (204) in each case bridging the respective longitudinal edge (46) of the absorbent body (7), that is to say extends in each case over a longitudinal edge region (47) of the absorbent body (7) and is also provided at least in each case in an adjoining subregion (67) of the overhang (66a, 66b), and wherein the reinforcing means (200) in this subregion (67), viewed in the transverse direction (16), is narrower than the overhang (66a, 66b), wherein the reinforcing means (200) comprises or consists of a reinforcing coating (202) with a weight per unit area of 1 to 30 g/m$^2$, and wherein the reinforcing means (200) is arranged on the inner side (85) of the cover sheet material (84), and wherein the cover sheet material (84) has a weight per unit area of 5 to 20 g/m$^2$.

2. Incontinence article according to Claim 1, **characterized in that** the reinforcing means (200), both in the front and in the rear overlapping region (36, 38), is in each case provided in a region (204) bridging the respective longitudinal edge (46) of the absorbent body (7).

3. Incontinence article according to either of Claims 1 and 2, **characterized in that** the reinforcing means (200) extends in the longitudinal direction (9) at least as far as the transverse edge (88, 89) of the absorbent body (7) and as far as the crotch-facing transverse edge (58, 60) of the stomach portion (4) and/or of the back portion (6).

**4.** Incontinence article according to one or more of the preceding claims, **characterized in that**, starting from the front and/or from the rear overlapping region (36, 38), the reinforcing means (200) extends over the respective crotch-facing transverse edge (58, 60) of the stomach portion (4) and/or back portion (6) in the direction of a transverse centre axis (30).

**5.** Incontinence article according to one or more of the preceding claims, **characterized in that** the reinforcing means (200) in the front overlapping region (36) and the reinforcing means (200) in the rear overlapping region (38) extend in the direction of the transverse centre axis (30) to join each other.

**6.** Incontinence article according to one or more of the preceding claims, **characterized in that** the proportion G'/H of the subregion (67) of the respective overhang (66a, 66b) that is extended over by the reinforcing means (200), with the width (G'), relative to the respective overhang (66a, 66b) with the width (H) is at least 0.10, particularly at least 0.15, more particularly at least 0.20, but preferably at most 0.80, particularly at most 0.75, more particularly at most 0.70.

**7.** Incontinence article according to one or more of the preceding claims, **characterized in that** the proportion G"/H of the respective longitudinal edge region (47) extended over by the reinforcing means (200), with the width (G"), relative to the respective overhang (66a, 66b) with the width (H) is at least 0.10, particularly at least 0.15, more particularly at least 0.20, but preferably at most 0.80, particularly at most 0.75, more particularly at most 0.70.

**8.** Incontinence article according to one or more of the preceding claims, **characterized in that** the reinforcing means (200) extends in the transverse direction (16) over the longitudinal edge region (47) of the absorbent body (7) in total, that is to say on both sides, in a proportion of at least 5%, particularly at least 10%, more particularly at least 15%, but preferably at most 35%, particularly at most 30%, more particularly at most 25%, relative to the width (K) of the absorbent body (7).

**9.** Incontinence article according to one or more of the preceding claims, **characterized in that** the reinforcing means (200) is arranged parallel to the longitudinal direction (9) and in the form of a strip of constant width (G).

**10.** Incontinence article according to one or more of the preceding claims, **characterized in that** the reinforcing coating (202) has a weight per unit area of 2 to 20 $g/m^2$, more particularly of 2 to 10 $g/m^2$.

**11.** Incontinence article according to one or more of the preceding claims, **characterized in that** the reinforcing coating (202) comprises an adhesive, particularly a hotmelt adhesive, more particularly a hydrophobic hotmelt adhesive.

**12.** Incontinence article according to one or more of the preceding claims, **characterized in that** the cover sheet material (84) is a composite of a liquid-permeable topsheet material (64) with longitudinal edges (210) and adjoining longitudinal edge regions (212) and of hydrophobic barrier means (68) joined at seams (76) on both sides to the longitudinal edge regions (212) of the topsheet material (64).

**13.** Incontinence article according to Claim 12, **characterized in that** the reinforcing means (200) extends over the seams (76) of the cover sheet material (84).

**14.** Incontinence article according to one or more of the preceding claims, **characterized in that** the overhang (66a, 66b) of the backsheet material (62) and/or of the cover sheet material (84) in the transverse direction (16) measures in total, that is to say on both sides of the longitudinal edges (46) of the absorbent body (7), 25 to 50%, in particular 30 to 45% and more particularly 35 to 45% relative to the greatest width (E) of the crotch portion (8).

**15.** Method for producing incontinence articles (2) in the form of briefs for receiving body excretions, with a front stomach portion (4) and a rear back portion (6), which are connected to each other at side seam regions (14) on both sides at the manufacturer's in order to form a stomach and back band which is continuous in the transverse or waist-encircling direction (16) and has a waist opening (18) that is closed in the waist-encircling direction (16), and with a crotch portion (8), which has an absorbent body (7) and extends in a longitudinal direction (9) between stomach portion (4) with a crotch-facing transverse edge (58) and back portion (6) with a crotch-facing transverse edge (60), wherein the crotch portion (8) is joined non-detachably to the stomach portion (4) and to the back portion (6), wherein the crotch portion (8) and also the stomach portion (4) and the back portion (6) delimit the leg openings (19) of the incontinence article, wherein first elasticating means (28) are provided in the stomach portion (4) and the back portion (6), are spaced apart from one another and extend parallel to one another in the transverse or waist-encircling direction (16) and thus elasticate the stomach portion (4) and the back portion (6) across the surface area thereof, wherein second elasticating means (40, 42) are provided in a crotch-side region (22, 26) of the stomach portion (4) and of the back portion (6) facing the leg openings (19), wherein the crotch portion (8) comprises a liquid-impermeable backsheet material (62), with an inner side (63) and with a weight per unit area of 10 to 40 $g/m^2$, and a nonwoven-based cover sheet material (84), with an inner side (85) and with a weight per unit area of 5 to 20 $g/m^2$, and wherein the absorbent body (7) is arranged between backsheet material (62) and cover sheet material (84) and the absorbent body (7) has a width (K), longitudinal edges (48), longitudinal edge regions (47) adjoining the latter, and transverse edges (88, 89), wherein the cover sheet material (84) or the cover sheet material (84) and the backsheet material (62) form(s), in the transverse direction (16), an overhang (66a, 66b) extending in each case outside the longitudinal edges of the absorbent body (7), wherein the crotch portion (8) has a width (E) of at least 200 mm in the transverse direction (16), and the overhang (66a, 66b) in the transverse direction (16) measures in total, that is to say on both sides of the longitudinal edges (46) of the absorbent body (7), at least 25% relative to the greatest width (E) of the crotch portion (8), wherein the crotch portion (8) overlaps at least 12% of the surface of the stomach portion (4) in a front overlapping region (36) and at least 20% of the surface of the back portion (6) in a rear overlapping region (38), wherein the overhang (66a, 66b) has a reinforcing means (200) in the front and/or in the rear overlapping region (36, 38), and wherein the method comprises the provision of the crotch portion (8), and the provision of the crotch portion (8) comprises the following method steps:

- supplying an endless cover sheet material web (84a), which forms the subsequent cover sheet material (84),
- applying a reinforcing means (200) to some areas of the cover sheet material web (84a) as a reinforcing coating (202), in such a way that the reinforcing means (200) is arranged on the subsequent inner side (85) of the cover sheet material (84), and in such a way that the reinforcing means (200) is provided in each case in a region (204) bridging the respective subsequent longitudinal edge (46) of the absorbent body (7), that is to say extends in each case over a respective subsequent longitudinal edge region (47) of the absorbent body (7) and is also provided at least in a respective adjacent subregion (67) of the subsequent overhang (66a, 66b), and in such a way that the reinforcing means (200) in this subregion (67), viewed in the transverse direction (16), is narrower than the overhang (66a, 66b),
- supplying an endless backsheet material web (62a), which forms the subsequent backsheet material (62),
- supplying and arranging absorbent bodies (7) spaced apart from one another between the cover sheet material web (84a) and the backsheet material web (62a).

16. Method according to Claim 15, **characterized in that** the reinforcing means is applied in such a way that the overhang (66a, 66b) has a reinforcing means (200) both in the rear overlapping region (38) and also in the front overlapping region (36).

17. Method according to one or both of Claims 15 and 16, **characterized in that** the reinforcing means (200) is applied in such a way that the proportion G'/H of the subregion (67) of the respective overhang (66a, 66b) that is extended over by the reinforcing means (200), with the width (G'), relative to the respective overhang (66a, 66b) with the width (H) is at least 0.10, particularly at least 0.15, more particularly at least 0.20, but preferably at most 0.80, particularly at most 0.75, more particularly at most 0.70.

18. Method according to one or more of Claims 15 to 17, **characterized in that** the reinforcing means is applied in such a way that the proportion G"/H of the respective longitudinal edge region (47) extended over by the reinforcing means (200), with the width (G"), relative to the respective overhang (66a, 66b) with the width (H) is at least 0.10, particularly at least 0.15, more particularly at least 0.20, but preferably at most 0.80, particularly at most 0.75, more particularly at most 0.70.

19. Method according to one of more of Claims 15 to 18, **characterized in that** the reinforcing means (200) is applied in such a way that the reinforcing means (200) extends in the transverse direction (16) over the subsequent longitudinal edge region (47) of the absorbent body (7) in total, that is to say on both sides, in a proportion of at least 5%, particularly at least 10%, more particularly at least 15% and at most 35%, particularly at most 30%, more particularly at most 25%, relative to the width (K) of the absorbent body (7).

20. Method according to one or more of Claims 15 to 19, **characterized in that** the reinforcing means (200) is applied parallel to the subsequent longitudinal direction (9) and in strip form with a constant width (G).

21. Method according to one or more of Claims 15 to 20, **characterized in that** the reinforcing coating (202) is applied with a weight per unit area of 1 to 30 $g/m^2$, particularly of 2 to 20 $g/m^2$, more particularly of 2 to 10 $g/m^2$.

**22.** Method according to one or more of Claims 15 to 21, **characterized in that** separate joining means are provided in the region of the overhang (66a, 66b) between cover sheet material (84) and backsheet material (62) and/or between cover sheet material (84) and absorbent body (7) and/or between backsheet material (62) and absorbent body (7), preferably not over the entire surface.

**23.** Method according to one or more of Claims 15 to 22, with the following further method steps:

- supplying two nonwoven-based partial webs (122, 124), which form the subsequent stomach portion (4) and the subsequent back portion (6), respectively, of the incontinence article (2),
- supplying and applying the second elasticating means (40, 42) to the two partial webs (122, 124) and fixing them thereto,
- bringing the crotch portions (8) and the two partial webs (122, 124) together in such a way that, in a longitudinal direction (9) transverse with respect to the machine direction (120), one end of the crotch portions (8) is arranged flat with one partial web (122) and another end thereof is arranged flat with the other partial web (124) in the front and/or rear overlapping region (36, 38), and the crotch portions (8) are arranged at a distance from one another in the machine direction (120), and fixing the crotch portions (8) and partial webs (122, 124) in the overlapping regions (36, 38) and forwarding them in the machine direction (120),
- supplying, applying and fixing the first elasticating means (28) to the partial webs (122, 124) in the machine direction (120),
- folding about a fold line (150) extending in the machine direction (120), in such a way that one partial web (122) comes to lie over the other partial web (124),
- joining the superposed partial webs (122, 124) transversely with respect to the machine direction (120) and at a distance from one another in order to form side seam regions (14) of the incontinence articles (2) to be produced, thereby obtaining products that have a stomach portion (4), a back portion (6) and, arranged therebetween, a crotch portion (8),
- making a separating cut transversely with respect to the machine direction (120) and thereby obtaining separate, finished incontinence articles (2).

**Revendications**

**1.** Article d'incontinence (2) en forme de slip, destiné à reprendre les déjections corporelles et présentant une partie ventrale avant (4) et une partie dorsale arrière (6) qui sont reliées l'une à l'autre lors de la fabrication pour former une bande ventrale et dorsale continue dans la direction transversale ou de tour de hanche (16), avec une ouverture de hanche (18), fermée dans la direction du tour de hanche, sur des parties (14) de couture latérale situées des deux côtés,
une partie d'entrejambe (8) qui présente un corps d'absorption (7) et qui s'étend dans le sens de la longueur (9) entre la partie ventrale (4) avec un bord transversal (58) tourné vers l'entrejambe et la partie dorsale (6) avec un bord transversal (60) tourné vers l'entrejambe,
la partie d'entrejambe (8) étant reliée de manière non libérale à la partie ventrale (4) et à la partie dorsale (6),
la partie d'entrejambe (8) la partie ventrale (4) et la partie dorsale (6) délimitant dans l'article d'incontinence des ouvertures (19) prévues pour les jambes,
des premiers moyens d'élastification (28) qui s'étendent à distance l'un de l'autre et parallèlement l'un à l'autre dans la direction transversale ou de tour de hanche (16) étant prévus dans la partie ventrale (4) et la partie dorsale (6) et élastifiant ainsi la surface de la partie ventrale (4) et de la partie dorsale (6),
des deuxièmes moyens d'élastification (40, 42) étant prévus dans des portions (22, 26) de la partie ventrale (4) et de la partie dorsale (6) situées du côté de l'entrejambe et tournées vers les ouvertures (19) prévues pour les jambes,
la partie d'entrejambe (8) comprenant un matériau (62) de feuille de dos imperméable aux liquides qui présente un côté intérieur (63) et dont le poids par unité de surface est de 10 à 40 $g/m^2$ ainsi qu'un matériau (84) de feuille de recouvrement à base d'un feutre et doté d'un côté intérieur (85),
le corps d'absorption (7) étant disposé entre le matériau (62) de feuille de dos et le matériau (84) de feuille de recouvrement et le corps d'absorption (7) présentant une largeur (K), des bords longitudinaux (46) auxquels sont adjacents des parties (47) de bord longitudinal et des bords longitudinaux (88, 89),
le matériau (84) de feuille de recouvrement ou le matériau (84) de feuille de recouvrement et le matériau (62) de feuille de dos formant dans la direction transversale (16) des débords (66a, 66b) qui s'étendent à l'extérieur des bords longitudinaux (46) du corps d'absorption (7), **caractérisé en ce que**
la partie d'entrejambe (8) présente dans la direction transversale (16) une largeur (E) d'au moins 200 mm et les débords (66a, 66b) représentent au total et donc des deux côtés des bords longitudinaux (46) du corps d'absorption (7), au moins 25 % de la plus grande largeur de la partie d'entrejambe (8),

**en ce que** la partie d'entrejambe (8) recouvre au moins 12 % de la surface de la partie ventrale (4) dans une partie de superposition avant (36) et au moins 20 % de la surface de la partie dorsale (6) dans une partie de superposition arrière (38),

**en ce que** les débords (66a, 66b) présentent un moyen de renfort (200) dans la partie de superposition avant et/ou dans la partie de superposition arrière (36, 38),

**en ce que** le moyen de renfort (200) est prévu dans une portion (204) qui surmonte le bord longitudinal respectif (46) du corps d'absorption (7), chevauche ainsi une partie (47) de bord longitudinal du corps d'absorption (7) et est également prévu au moins dans la partie (67) des débords (66a, 66b) qui lui est adjacente,

**en ce que** dans cette partie (67), le moyen de renfort (200) est plus étroit que les débords (66a, 66b) dans la direction transversale (16),

**en ce que** le moyen de renfort (200) comporte d'un revêtement de renfort (202) dont le poids par unité de surface est de 1 à 30 g/m$^2$ ou en est constitué,

**en ce que** le moyen de renfort (200) est disposé sur le côté intérieur (85) du matériau (84) de feuille de recouvrement et

**en ce que** le matériau (84) de feuille de recouvrement présente un poids par unité de surface de 5 à 20 g/m$^2$.

2. Article d'incontinence selon la revendication 1, **caractérisé en ce que** le moyen de renfort (200) est prévu à la fois dans la partie de superposition avant et dans la partie de superposition arrière (36, 38), chaque fois dans une partie (204) qui chevauche le bord longitudinal (46) concerné du corps d'absorption (7).

3. Article d'incontinence selon l'une des revendications 1 ou 2 qui précèdent, **caractérisé en ce que** le moyen de renfort (200) s'étend dans le sens de la longueur (9) au moins jusqu'au bord transversal (88, 89) du corps d'absorption (7) et jusqu'aux bord transversaux (58, 60), tournés vers l'entrejambe, de la partie ventrale (4) et/ou de la partie dorsale (6).

4. Article d'incontinence selon l'une ou plusieurs des revendications précédentes, **caractérisé en ce que** le moyen de renfort (200) s'étend depuis la partie de superposition avant et/ou depuis la partie de superposition arrière (36, 38) sur les bords transversaux (58, 60), tournés vers l'entrejambe, de la partie ventrale (4) et/ou de la partie dorsale (6) en direction de l'axe transversale central (30).

5. Article d'incontinence selon l'une ou plusieurs des revendications précédentes, **caractérisé en ce que** le moyen de renfort (200) de la partie de superposition avant (36) et le moyen de renfort (200) de la partie de superposition arrière (38) s'étendent dans la direction de l'axe transversal central (30) et sont reliés l'un à l'autre.

6. Article d'incontinence selon l'une ou plusieurs des revendications précédentes, **caractérisé en ce que** le rapport G'/H entre la partie (67) de chaque débord (66a, 66b) qui chevauche le moyen de renfort (200), de largeur (G'), et chaque débord (66a, 66b), de largeur (H), vaut au moins 0,10, en particulier au moins 0,15, de manière plus particulière au moins 0,20, mais de préférence au plus 0,80, en particulier au plus 0,75 et de manière plus particulière au plus 0,70.

7. Article d'incontinence selon l'une ou plusieurs des revendications précédentes, **caractérisé en ce que** le rapport G"/H entre chaque partie (47) de bord longitudinal recouverte par le moyen de renfort (200), de largeur (G"), et chaque débord (66a, 66b), de largeur (H), vaut au moins 0,10, en particulier au moins 0,15, de manière plus particulière au moins 0,20, mais de préférence au plus 0,80, en particulier au plus 0,75 et de manière plus particulière au plus 0,70.

8. Article d'incontinence selon l'une ou plusieurs des revendications précédentes, **caractérisé en ce que** le moyen de renfort (200) recouvre la partie (47) de bord longitudinal du corps d'absorption (7) dans la direction transversale (16), au total et donc des deux côtés, dans une proportion d'au moins 5 %, en particulier d'au moins 10 %, de manière plus particulière d'au moins 15 % mais de préférence d'au plus 35 %, en particulier d'au plus 30 %, et de manière plus particulière d'au plus 25 % de la largeur (K) du corps d'absorption (7).

9. Article d'incontinence selon l'une ou plusieurs des revendications précédentes, **caractérisé en ce que** le moyen de renfort (200) est disposé parallèlement au sens de la longueur (9) et sous la forme d'un ruban de largeur (G) constante.

10. Article d'incontinence selon l'une ou plusieurs des revendications précédentes, **caractérisé en ce que** le revêtement de renfort (202) présente un poids par unité de surface de 2 à 20 g/m$^2$ et de manière plus particulière de 2 à 10 g/m$^2$.

**11.** Article d'incontinence selon l'une ou plusieurs des revendications précédentes, **caractérisé en ce que** le revêtement de renfort (202) comporte un adhésif, en particulier un adhésif fusible à chaud et de manière plus particulière un adhésif fusible à chaud et hydrophobe.

**12.** Article d'incontinence selon l'une ou plusieurs des revendications précédentes, **caractérisé en ce que** le matériau (84) de feuille de recouvrement est un composite constitué d'un matériau (64) de feuille supérieure perméable aux liquides et doté de bords longitudinaux (210) ainsi que de parties adjacentes de bord longitudinal (212), et de moyens hydrophobes de barrière (68) reliés des deux côtés aux parties de bord longitudinal (212) du matériau (64) de feuille supérieure dans des points de jonction (76).

**13.** Article d'incontinence selon la revendication 12, **caractérisé en ce que** le moyen de renfort (200) chevauche les emplacements (76) de jonction du matériau (84) de feuille de recouvrement.

**14.** Article d'incontinence selon l'une ou plusieurs des revendications précédentes, **caractérisé en ce que** les débords (66a, 66b) du matériau (62) de feuille de dos et/ou du matériau (84) de feuille de recouvrement représentent dans la direction transversale (16) au total et donc des deux côtés des bords longitudinaux (46) du corps d'absorption (7) de 25 à 50 %, en particulier de 30 à 45 % et de manière plus particulière de 35 à 45 % de la plus grande largeur (E) de la partie d'entrejambe (8).

**15.** Procédé de fabrication d'articles (2) d'incontinence en forme de slip, destiné à reprendre les déjections corporelles et présentant
une partie ventrale avant (4) et une partie dorsale arrière (6) qui sont reliées l'une à l'autre lors de la fabrication pour former une bande ventrale et dorsale continue dans la direction transversale ou de tour de hanche (16), avec une ouverture de hanche (18), fermée dans la direction du tour de hanche, sur des parties (14) de couture latérale situées des deux côtés,
une partie d'entrejambe (8) qui présente un corps d'absorption (7) et qui s'étend dans le sens de la longueur (9) entre la partie ventrale (4) avec un bord transversal (58) tourné vers l'entrejambe et la partie dorsale (6) avec un bord transversal (60) tourné vers l'entrejambe,
la partie d'entrejambe (8) étant reliée de manière non libérale à la partie ventrale (4) et à la partie dorsale (6),
la partie d'entrejambe (8) la partie ventrale (4) et la partie dorsale (6) délimitant dans l'article d'incontinence des ouvertures (19) prévues pour les jambes,
des premiers moyens d'élastification (28) qui s'étendent à distance l'un de l'autre et parallèlement l'un à l'autre dans la direction transversale ou de tour de hanche (16) étant prévus dans la partie ventrale (4) et la partie dorsale (6) et élastifiant ainsi la surface de la partie ventrale (4) et de la partie dorsale (6),
des deuxièmes moyens d'élastification (40, 42) étant prévus dans des portions (22, 26) de la partie ventrale (4) et de la partie dorsale (6) situées du côté de l'entrejambe et tournées vers les ouvertures (19) prévues pour les jambes,
la partie d'entrejambe (8) comprenant un matériau (62) de feuille de dos imperméable aux liquides qui présente un côté intérieur (63) et dont le poids par unité de surface est de 10 à 40 g/m$^2$ ainsi qu'un matériau (84) de feuille de recouvrement à base d'un feutre et doté d'un côté intérieur (85) et dont le poids par unité de surface est de 5 à 20 g/m$^2$,
le corps d'absorption (7) étant disposé entre le matériau (62) de feuille de dos et le matériau (84) de feuille de recouvrement et le corps d'absorption (7) présentant une largeur (K), des bords longitudinaux (48) auxquels sont adjacents des parties (47) de bord longitudinal et des bords longitudinaux (88, 89),
le matériau (84) de feuille de recouvrement ou le matériau (84) de feuille de recouvrement et le matériau (62) de feuille de dos formant dans la direction transversale (16) des débords (66a, 66b) qui s'étendent à l'extérieur des bords longitudinaux du corps d'absorption (7),
la partie d'entrejambe (8) présentant dans la direction transversale (16) une largeur (E) d'au moins 200 mm et les débords (66a, 66b) représentant au total et donc des deux côtés des bords longitudinaux (46) du corps d'absorption (7) au moins 25 % de la plus grande largeur de la partie d'entrejambe (8),
la partie d'entrejambe (8) recouvrant au moins 12 % de la surface de la partie ventrale (4) dans une partie de superposition avant (36) et au moins 20 % de la surface de la partie dorsale (6) dans une partie de superposition arrière (38),
les débords (66a, 66b) présentant un moyen de renfort (200) dans la partie de superposition avant et/ou dans la partie de superposition arrière (36, 38),
le procédé comportant la réalisation de la partie d'entrejambe (8), la réalisation de la partie d'entrejambe (8) présentant les étapes de procédé suivantes :

- amenée d'une bande sans fin (84a) de matériau de feuille de recouvrement qui formera ultérieurement le matériau (84) de feuille de recouvrement,

- placement dans certaines portions de la bande (84a) de matériau de feuille de recouvrement d'un moyen de renfort (200) qui présente la forme d'un revêtement de renfort (202), de telle sorte que le moyen de renfort (200) soit disposé sur le futur côté intérieur (85) du matériau (84) de feuille de recouvrement et de telle sorte que le moyen de renfort (200) soit prévu dans une partie (204) qui chevauche l'un des deux bords longitudinaux futurs (46) du corps d'absorption (7), qui recouvre ainsi à la fois une partie (47) de bord longitudinal ultérieure du corps d'absorption (7) et qui est prévue également au moins dans une partie adjacente (67) des futurs débords (66a, 66b), de telle sorte que le moyen de renfort (200) soit plus étroit dans cette partie (67) dans la direction transversale (16) que les débords (66a, 66b),
- amenée d'une bande sans fin (62a) de matériau de feuille de dos qui forme le matériau (62) de la future feuille de dos,
- amenée et placement de corps d'absorption (7) à distance les uns des autres entre la bande (84a) de matériau de feuille de recouvrement et la bande (62a) de matériau de feuille de dos.

16. Procédé selon la revendication 15, **caractérisé en ce que** le moyen de renfort est placé de telle sorte que les débords (66a, 66b) présentent un moyen de renfort (200) à la fois dans la partie de superposition arrière (38) et dans la partie de superposition avant (36).

17. Procédé selon l'une ou plusieurs des revendications 15 à 16 qui précèdent, **caractérisé en ce que** le moyen de renfort (200) est placé de telle sorte que le rapport G'/H entre la partie (67) de chaque débord (66a, 66b) qui chevauche le moyen de renfort (200), de largeur (G'), et chaque débord (66a, 66b), de largeur (H), vaut au moins 0,10, en particulier au moins 0,15, de manière plus particulière au moins 0,20, mais de préférence au plus 0,80, en particulier au plus 0,75 et de manière plus particulière au plus 0,70.

18. Procédé selon l'une ou plusieurs des revendications 15 à 17 qui précèdent, **caractérisé en ce que** le moyen de renfort est placé de telle sorte que le rapport G"/H entre chaque partie (47) de bord longitudinal recouverte par le moyen de renfort (200), de largeur (G"), et chaque débord (66a, 66b), de largeur (H), vaut au moins 0,10, en particulier au moins 0,15, de manière plus particulière au moins 0,20, mais de préférence au plus 0,80, en particulier au plus 0,75 et de manière plus particulière au plus 0,70.

19. Procédé selon l'une ou plusieurs des revendications 15 à 18 qui précèdent, **caractérisé en ce que** le moyen de renfort (200) est appliqué de telle sorte que le moyen de renfort (200) recouvre la future partie (47) de bord longitudinal du corps d'absorption (7) dans la direction transversale (16), au total et donc des deux côtés, dans une proportion d'au moins 5 %, en particulier d'au moins 10 %, de manière plus particulière d'au moins 15 % mais de préférence d'au plus 35 %, en particulier d'au plus 30 %, et de manière plus particulière d'au plus 25 % de la largeur (K) du corps d'absorption (7).

20. Procédé selon l'une ou plusieurs des revendications 15 à 19 qui précèdent, **caractérisé en ce que** le moyen de renfort (200) est disposé parallèlement au futur sens de la longueur (9) et sous la forme d'un ruban de largeur (G) constante.

21. Procédé selon l'une ou plusieurs des revendications 15 à 20 qui précèdent, **caractérisé en ce que** le revêtement de renfort (202) est appliqué à un poids par unité de surface de 1 à 30 g/m$^2$, en particulier de 2 à 20 g/m$^2$ et de manière plus particulière de 2 à 10 g/m$^2$.

22. Procédé selon l'une ou plusieurs des revendications 15 à 21 qui précèdent, **caractérisé en ce que** des moyens séparés de jonction sont prévus, de préférence non sur toute la surface, dans la partie des débords (66a, 66b) située entre le matériau (84) de feuille de recouvrement et le matériau (62) de feuille de dos, entre le matériau (84) de feuille de recouvrement et le corps d'absorption (7) et/ou entre le matériau (62) de feuille de dos et le corps d'absorption (7).

23. Procédé selon l'une ou plusieurs des revendications 15 à 22 qui précèdent, qui présente les étapes supplémentaires de procédé suivantes :

- amenée de deux bandes partielles (122, 124) à base de feutre qui forment la future partie ventrale (4) et la future partie dorsale (6) de l'article d'incontinence (2),
- amenée, placement et fixation des deuxièmes moyens d'élastification (40, 42) sur les deux bandes partielles (122, 124),
- assemblage des parties d'entrejambe (8) et des deux bandes partielles (122, 124) de telle sorte que les parties d'entrejambe (8) soient disposées à plat dans le sens de la longueur (9), transversalement à la direction machine (120), avec une extrémité avec une bande partielle (122) et à l'autre extrémité avec l'autre bande partielle (124) dans la partie de superposition avant et/ou la partie de superposition arrière (36, 38), les parties d'entrejambe (8) étant placées à distance mutuelle dans la direction machine (120), fixation des parties d'entrejambe (8) et des bandes partielles (122, 124) dans les parties de superposition (36, 38), et poursuite du transport dans la direction machine (120),

- amenée, placement et fixation des premiers moyens d'élastification (28) sur les bandes partielles (122, 124) dans la direction machine (120),

- pliage autour d'une ligne de pliage (150) qui s'étend dans la direction machine (120), de telle sorte qu'une bande partielle (122) vienne se placer au-dessus de l'autre bande partielle (124),

- jonction des bandes partielles (122, 124) posées l'une au-dessus de l'autre, transversalement par rapport à la direction machine (120) et à distance l'une de l'autre pour former des parties (14) de couture latérale de l'article d'incontinence (2) à fabriquer, et obtention de produits qui présentent une partie ventrale (4), une partie dorsale (6) et entre elles une partie d'entrejambe (8),

- exécution d'une découpe de séparation transversale par rapport à la direction machine (120) et obtention d'articles d'incontinence (2) terminés et séparés les uns des autres.

Fig.1

Fig. 2c

Fig. 2b

Fig. 2a

Fig. 3

Fig. 4

Fig. 5

Fig. 6

Fig. 7

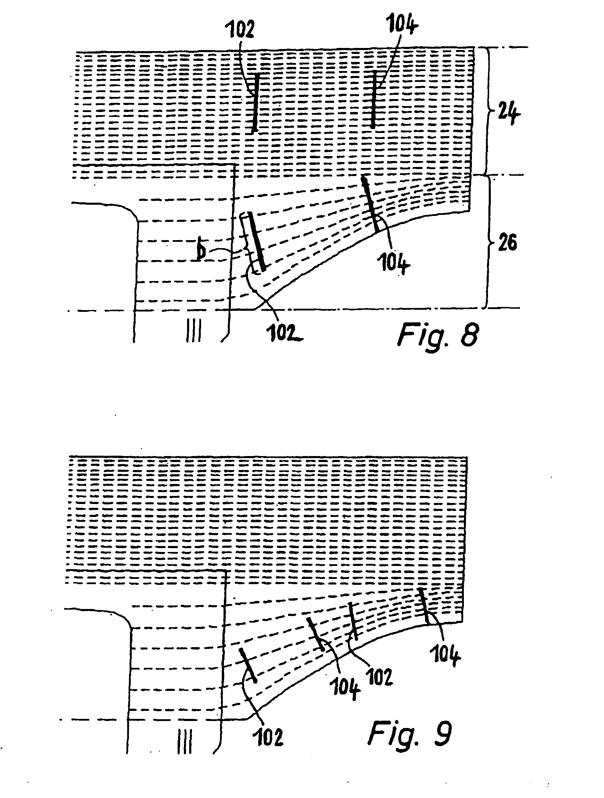

Fig. 8

Fig. 9

Fig. 10

Fig. 11

EP 2 323 604 B1

*Fig. 12*

42

Fig. 13

EP 2 323 604 B1

Fig. 14

**IN DER BESCHREIBUNG AUFGEFÜHRTE DOKUMENTE**

**In der Beschreibung aufgeführte Patentdokumente**

- WO 2004052260 A1
- EP 0901781 B1
- EP 1459719 A2